(19) Europäisches Patentamt
European Patent Office
Office européen des brevets

(11) Veröffentlichungsnummer : **0 344 543 B1**

# (12) EUROPÄISCHE PATENTSCHRIFT

(45) Veröffentlichungstag der Patentschrift :
**22.07.92 Patentblatt 92/30**

(21) Anmeldenummer : **89109040.9**

(22) Anmeldetag : **19.05.89**

(51) Int. Cl.⁵ : **C07D 471/04,** C07D 243/38, C07D 495/04, C07D 487/04, C07D 495/14, C07D 471/14, C07D 487/14, A61K 31/55, // (C07D471/04, 243:00, 221:00), (C07D495/04, 333:00, 243:00)

## (54) Neue kondensierte Diazepinone, Verfahren zu ihrer Herstellung und diese Verbindungen enthaltende Arzneimittel.

Die Akte enthält technische Angaben, die nach dem Eingang der Anmeldung eingereicht wurden und die nicht in dieserPatentschrift enthalten sind.

(30) Priorität : **30.05.88 DE 3818299**

(43) Veröffentlichungstag der Anmeldung :
**06.12.89 Patentblatt 89/49**

(45) Bekanntmachung des Hinweises auf die Patenterteilung :
**22.07.92 Patentblatt 92/30**

(84) Benannte Vertragsstaaten :
**AT BE CH DE ES FR GB GR IT LI LU NL SE**

(56) Entgegenhaltungen :
**EP-A- 0 213 293**
**FR-M- 911**
**US-A- 3 150 125**

(73) Patentinhaber : **Dr. Karl Thomae GmbH**
**Postfach 1755**
**W-7950 Biberach 1 (DE)**

(72) Erfinder : **Engel, Wolfhard, Dr. Dipl.-Chem.**
**Mozartstrasse 13**
**W-7950 Biberach 1 (DE)**
Erfinder : **Eberlein, Wolfgang, Dr. Dipl.-Chem.**
**Obere Au 6**
**W-7950 Biberach 1 (DE)**
Erfinder : **Mihm, Gerhard, Dr. Dipl.-Chem.**
**Nickeleshalde 5/1**
**W-7950 Biberach 1 (DE)**
Erfinder : **Trummlitz, Günter, Dr. Dipl.-Chem.**
**Buchenweg 27**
**W-7951 Warthausen (DE)**
Erfinder : **Mayer, Norbert, Dr.**
**Friedrich-Ebert-Strasse 66**
**W-7950 Biberach 1 (DE)**
Erfinder : **de Jonge, Adriaan, Dr.**
**De Boomgaard 19**
**NL-3971 LD Driebergen (DE)**

EP 0 344 543 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 ist beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen kondensierten Diazepinone bei vergleichbarer oder verbesserter Selektivität und Resorption nach oraler Gabe durch eine wesentlich verstärkte Wirkung und ausgeprägte Hydrolysestabilität aus.

Die neuen kondensierten Diazepinone besitzen die allgemeine Formel I,

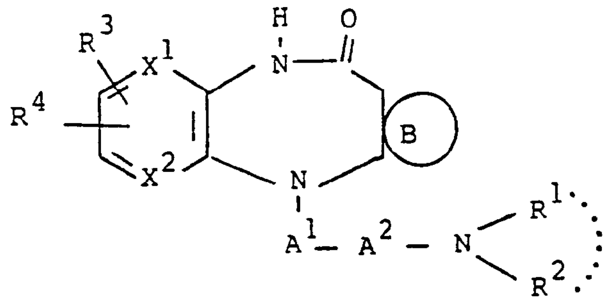

(I)

in der 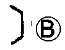 einen der zweiwertigen Reste

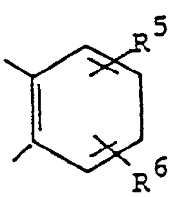 , 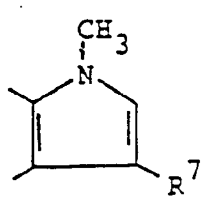 , 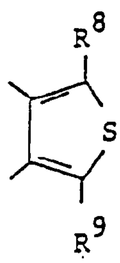 , 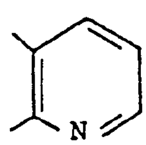 oder 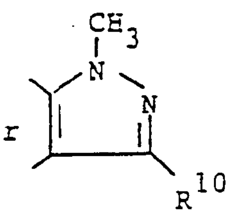

(S) (T) (U) (V) (W)

darstellt und
$X^1$, $X^2$, $A^1$, $A^2$ und $R^1$ bis $R^{10}$ die folgenden Bedeutungen besitzen:

$X^1$ und $X^2$ stellen eine =CH-Gruppe dar oder, sofern 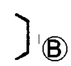 die Bedeutungen der oben genannten, zweiwertigen Reste (S), (U) oder (W) annimmt, können beide oder nur $X^1$ oder nur $X^2$ auch ein Stickstoffatom darstellen;
$A^1$ ist eine gegebenenfalls durch ein oder zwei Methylgruppen substituierte Methylengruppe;
$A^2$ eine geradkettige, gegebenenfalls eine Doppel- oder Dreifachbindung enthaltende und drei bis sieben Kohlenstoffatome umfassende Alkylenkette, die zusätzlich methylsubstituiert sein kann;
$R^1$ ist ein verzweigter oder unverzweigter Alkylrest mit 1 bis 4 Kohlenstoffatomen;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder eine Stickstoffunktion unterbrochen und/oder durch einen gegebenenfalls durch eine Phenyl-, Di-($C_1$-$C_3$)alkylamino- oder (Cycloalkyl)alkylaminogruppe substituierten Alkylrest mit 1 bis 6 Kohlenstoffatomen oder durch

einen gegebenenfalls ein oder zwei Ether-Sauerstoffatome enthaltenden (Cycloalkyl)alkyl-Rest substituiert sein kann, wobei die zuvor genannten Cycloalkylgruppen 4 bis 7 Kohlenstoffatome und die an diese gebundenen Alkylengruppen 1 bis 3 Kohlenstoffatome enthalten können;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe.

Für den Fall, daß 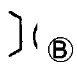 den zweiwertigen Rest T darstellt und $R^7$ ein Wasserstoffatom ist, kann $R^3$ kein Chloratom bedeuten und $A^2$ keine Doppel- oder Dreifachbindungen enthalten.

Bevorzugte Verbindungen der obigen allgemeinen Formel I sind solche, in denen
$X^1$ eine =CH-Gruppe,

$X^2$ entweder ein Stickstoffatom und 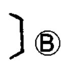 den zweiwertigen Rest (S) darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist,

oder eine =CH-Gruppe darstellt, wenn 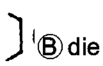 die Bedeutung der zweiwertigen Reste U oder V annimmt, wobei $R^8$ ein Wasserstoffatom und $R^9$ eine Methylgruppe ist;
$A^1$ die Methylengruppe;
$A^2$ eine geradkettige, gegebenenfalls eine Doppel- oder Dreifachbindung enthaltende und drei bis sechs Kohlenstoffatome umfassende Alkylenkette;
$R^1$ einen verzweigten oder unverzweigten Alkylrest mit 1 bis 4 Kohlenstoffatomen;
$R^2$ einen verzweigten oder unverzweigten Alkylrest mit 1 bis 7 Kohlenstoffatomen, einen Cycloalkyl- oder (Cycloalkyl)methyl-Rest mit 3 bis 7 Kohlenstoffatomen im Ring, wobei der Cycloalkylring gegebenenfalls noch durch eine Hydroxygruppe substituiert sein kann, bedeuten
oder
$R^1$ und $R^2$ zusammen mit dem dazwischenliegenden Stick stoffatom einen 5- bis 7-gliedrigen gesättigten, monocyclischen, heterocyclischen Ring bilden, der gegebenenfalls durch ein Sauerstoffatom oder ein Stickstoffatom unterbrochen und/oder durch einen gegebenenfalls durch eine Phenyl-, Di-$(C_1$-$C_3)$alkylamino- oder (Cycloalkyl)alkylaminogruppe substituierten Alkylrest mit 1 bis 3 Kohlenstoffatomen oder durch einen gegebenenfalls ein oder zwei Ether-Sauerstoffatome enthaltenden (Cycloalkyl)alkyl-Rest substituiert sein kann, wobei die zuvor genannten Cycloalkylgruppen 4 bis 7 Kohlenstoffatome und die an diese gebundenen Alkylengruppen 1 bis 3 Kohlenstoffatome enthalten können.

Ganz besonders bevorzugte Verbindungen der allgemeinen Formen I sind solche, in denen
$X^1$ eine =CH-Gruppe,

$X^2$ ein Stickstoffatom und 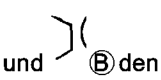 den zweiwertigen Rest (S) darstellt, mit der Maßgabe, daß $R^3$, $R^4$ und $R^5$ Wasserstoffatome sind und $R^6$ ein Wasserstoff-, Chlor- oder Bromatom oder eine Methyl- oder Ethylgruppe in 8- oder 9-Stellung des Heterocyclus ist,
$A^1$ die Methylengruppe,
$A^2$ eine geradkettige, gegebenenfalls eine Doppel- oder Dreifachbindung enthaltende und drei bis sechs Kohlenstoffatome umfassende Alkylenkette und
$R^1$ und $R^2$ zusammen mit dem dazwischenliegenden Stickstoffatom den Dimethylamino-, Diethylamino-, Dipropylamino-, [Bis(methylethyl)]amino-; 1-Pyrrolidinyl-, 1-Piperidinyl-, Hexahydro-1H-1-azepinyl-, 4-Morpholinyl-, 2-[(Diethylamino)methyl]-1-piperidinyl-, trans-(4-Hydroxycyclohexyl)methylamino-, (Cyclohexyl)methylamino-, 2-[[(Cyclohexyl)methylamino]methyl]-1-piperidinyl-, 4-Methyl-1-piperazinyl-, 4-[2-(1,3-Dioxolan-2-yl)ethyl]-1-piperazinyl-, 4-(Phenylmethyl)-1-piperazinyl- oder 4-(2-Phenylethyl)-1-piperazinyl- Rest bedeuten.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure,Amidosulfonsäure oder Cyclohexansulfaminsäure als geeignet erwiesen.

Zur Erläuterung des Erfindungsgegenstandes seien als Beispiele folgende Verbindungen genannt:

5,11-Dihydro-11-[4-(1-pyrrolidinyl)-2-butinyl]-6H-pyrido[2,3-b][1,4]benzodiazepin-6-on;
5,11-Dihydro-11-[4-(dimethylamino)-2-butinyl]-6H-pyrido[2,3-b][1,4]benzodiazepin-6-on;
11-[4-(Diethylamino)-2-butinyl]-5,11-dihydro-6H-pyrido[2,3-b][1,4]benzodiazepin-6-on;
11-[4[Bis(methylethyl)amino]-2-butinyl]-5,11-dihydro-6H-pyrido[2,3-b][1,4]benzodiazepin-6-on;
11-[4-(Cyclohexyl)methylamino]-2-butinyl]-5,11-dihydro-6H-pyrido[2,3-b][1,4]benzodiazepin-6-on;
11-[4-[2-[(Diethylamino)methyl] -1-piperidinyl]-2-butinyl]-5,11-dihydro-6H-pyrido[2,3-b][1,4] benzodiazepin-6-on;
5,11-Dihydro-11-[4-[2-[(dipropylamino)methyl]-1-piperidinyl]-2-butinyl]-6H-pyrido[2,3-b][1,4]benzodiazepin-6-on;
trans-5,11-Dihydro-11-[4-[(4-hydroxycyclohexyl)methylamino]-2-butinyl]-6H-pyrido[2,3-b][1,4]benzodiazepin-6-on;
5,11-Dihydro-11-[4-(1-piperidinyl)-2-butinyl]-6H-pyrido[2,3-b][1,4]benzodiazepin-6-on-hemifumarat;
5,11-Dihydro-11-[4-(hexahydro-1H-1-azepinyl)-2-butinyl]-6H-pyrido[2,3-b][1,4]benzodiazepin-6-on-hydrochlorid;
5,11-Dihydro-11-[4-(4-morpholinyl)-2-butinyl]-6H-pyrido[2,3-b][1,4]benzodiazepin-6-on;
11-[4-[2-[[(Cyclohexyl)methylamino]methyl]-1-piperidinyl]-2-butinyl]-5,11-dihydro-6H-pyrido[2,3-b][1,4]benzodiazepin-6-on;
11-[4-[2-[[(Cyclohexyl)methylamino]methyl]-1-piperidinyl]-butyl]-5,11-dihydro- 6H-pyrido[2,3-b][1,4]benzodiazepin-6-on;
(E)-11-[4-[2-[[(Cyclohexyl)methylamino]methyl]-1-piperidinyl]-2-butenyl]-5,11-dihydro-6H-pyrido[2,3-b][1,4]-benzodiazepin-6-on;
(Z)-11-[4-[2-[[(Cyclohexyl)methylamino]methyl]-1-piperidinyl]-2-butenyl]-5,11-dihydro-6H-pyrido[2,3-b][1,4]-benzodiazepin-6-on;
5,11-Dihydro-11-[4-(4-methyl-1-piperazinyl)-2-butinyl]-6H-pyrido[2,3-b][1,4]benzodiazepin-6-on;
5,11-Dihydro-11-[4-(1-piperidinyl)-butyl]-6H-pyrido[2,3-b][1,4]benzodiazepin-6- on;
5,11-Dihydro-11-[5-(1-piperidinyl)-2-pentinyl]-6H-pyrido[2,3-b][1,4]benzodiazepin-6-on;
5,11-Dihydro-11-[5-(1-piperidinyl)pentyl]-6H-pyrido[2,3-b][1,4]benzodiazepin-6-on;
(E)-5,11-Dihydro-11-[5-(1-piperidinyl)-2-pentenyl]-6H-pyrido[2,3-b][1,4]benzodiazepin-6-on;
(Z)-5,11-Dihydro-11-[5-(1-piperidinyl)-2-pentenyl]-6H-pyrido[2,3-b][1,4]benzodiazepin-6-on;
5,11-Dihydro-11-[5-(hexahydro-1H-1-azepinyl)-2-pentinyl]-6H-pyrido[2,3-b][1,4]benzodiazepin-6-on;
5,11-Dihydro-11-[5-(hexahydro-1H-1-azepinyl)pentyl]-6H-pyrido[2,3-b][1,4]benzodiazepin-6-on;
5,11-Dihydro-11-[1-methyl-5-(1-piperidinyl)-2-pentinyl]-6H-pyrido[2,3-b][1,4]benzodiazepin-6-on;
5,11-Dihydro-11-[1,1-dimethyl-5-(1-piperidinyl)-2-pentinyl]-6H-pyrido[2,3-b][1,4]benzodiazepin-6-on;
5,11-Dihydro-11[5-(1-piperidinyl)-2-hexinyl]-6H-pyrido[2,3-b] [1,4]benzodiazepin-6-on;
5,11-Dihydro-11-[5-(1-piperidinyl)-3-pentinyl]-6H-pyrido[2,3-b][1,4]benzodiazepin-6-on;
5,11-Dihydro-11-[6-(1-piperidinyl)-2-hexinyl]-6H-pyrido[2,3-b][1,4]benzodiazepin-6-on;
5,11-Dihydro-11-[6-(1-piperidinyl)hexyl]-6H-pyrido[2,3-b][1,4]benzodiazepin-6-on;
(E)-5,11-Dihydro-11-[6-(1-piperidinyl)-2-hexenyl]-6H-pyrido[2,3-b][1,4]benzodiazepin-6-on;
(Z)-5,11-Dihydro-11-[6-(1-piperidinyl)-2-hexenyl]-6H-pyrido[2,3-b][1,4]benzodiazepin-6-on;
5,11-Dihydro-11-[6-(hexahydro-1H-1-azepinyl)-2-hexinyl]-6H-pyrido[2,3-b][1,4]benzodiazepin-6-on;
5,11-Dihydro-11-[1-methyl-6-(1-piperidinyl)-2-hexinyl]-6H-pyrido[2,3-b][1,4]benzodiazepin-6-on;
5,11-Dihydro-11-[1,1-dimethyl-6-(1-piperidinyl)-2-hexinyl]-6H-pyrido[2,3-b][1,4]benzodiazepin-6-on;
5,11-Dihydro-11-[6-(1-piperidinyl)-2-heptinyl]-6H-pyrido[2,3-b][1,4]benzodiazepin-6-on;
5,11-Dihydro-11-[6-(1-piperidinyl)-3-hexinyl]-6H-pyrido[2,3-b][1,4]benzodiazepin-6-on;
5,11-Dihydro-11-[6-(1-piperidinyl)-4-hexinyl]-6H-pyrido[2,3-b][1,4]benzodiazepin-6-on;
5,11-Dihydro-11-[6-(4-methyl-1-piperazinyl)hexyl]-6H-pyrido[2,3-b][1,4]benzodiazepin-6-on;
5,11-Dihydro-11-[6-(hexahydro-1H-1-azepinyl)hexyl]-6H-pyrido[2,3-b][1,4]benzodiazepin-6-on;
5,11-Dihydro-11-[6-(1-pyrrolidinyl)hexyl]-6H-pyrido[2,3-b][1,4]benzodiazepin-6-on;
(E) -5,11-Dihydro-11-[6-(1-piperidinyl)-3-hexenyl]-6H-pyrido[2,3-b][1,4]benzodiazepin-6-on;
(Z)-5,11-Dihydro--11-[6-(1-piperidinyl)-3-hexenyl]-6H-pyrido[2,3-b][1,4]benzodiazepin-6-on,
(E)-5,11-Dihydro-11-[6-(1-piperidinyl)-4-hexenyl]-6H-pyrido[2,3-b][1,4]benzodiazepin-6-on;
(Z)-5,11-Dihydro-11-[6-(1-piperidinyl)-4-hexenyl]-6H-pyrido[2,3-b][1,4] benzodiazepin-6-on;
D,L-11-[6-[2-[(Diethylamino)methyl]-1-piperidinyl]hexyl]-5,11-dihydro-6H-pyrido[2,3-b][1,4]benzodiazepin-6-on;
(R)-11-[6-[2-[(Diethylamino)methyl]-1-piperidinyl]hexyl]-5,11-dihydro-6H-pyrido[2,3-b][1,4]benzodiazepin-6-on;
(S)-11-[6-[2-[(Diethylamino)methyl]-1-piperidinyl]hexyl]-5,11-dihydro-6H-pyrido[2,3-b][1,4]benzodiazepin-6-on;
5,11-Dihydro-11-[7-(1-piperidinyl)heptyl]-6H-pyrido[2,3-b][1,4]benzodiazepin-6-on;
5,11-Dihydro-11-[7-(1-piperidinyl)octyl]-6H-pyrido[2,3-b][1,4]benzodiazepin-6-on;
5,11-Dihydro-11-[7-(1-piperidinyl)-2-heptinyl]-6H-pyrido[2,3-b][1,4]benzodiazepin-6-on;

5,11-Dihydro-11-[7-(1-piperidinyl)-3-heptinyl]-6H-pyrido[2,3-b][1,4]benzodiazepin-6-on;
5,11-Dihydro-11-[7-(1-piperidinyl)-4-heptinyl]-6H-pyrido[2,3-b][1,4]benzodiazepin-6-on;
5,11-Dihydro-11-[7-(1-piperidinyl)-5-heptinyl]-6H-pyrido[2,3-b][1,4]benzodiazepin-6-on;
5,11-Dihydro-11-[1-methyl-7-(1-piperidinyl)heptyl]-6H-pyrido[2,3-b][1,4]benzodiazepin-6-on;
5,11-Dihydro-11-[1,1-dimethyl-7-(1-piperidinyl)-2-heptinyl]-6H-pyrido[2,3-b][1,4]benzodiazepin-6-on;
5,11-Dihydro-11-[7-(hexahydro-1H-1-azepinyl)-2-heptinyl]-6H-pyrido[2,3-b][1,4] benzodiazepin-6-on;
11-[7-[2-[(Diethylamino)methyl]-1-piperidinyl]heptyl]-5,11-dihydro-6H-pyrido[2,3-b][1,4]benzodiazepin-6-on;
5,11-Dihydro-11-[4-methyl-7-(1-piperidinyl)heptyl]-6H-pyrido-2,3-b][1,4]benzodiazepin-6-on;
6,11-Dihydro-11-[6-[1-piperidinyl]-2-hexinyl]-5H-pyrido[2,3-b][1,5]benzodiazepin-5-on;
6,11-Dihydro-11-[6-(1-piperidinyl)hexyl]-5H-pyrido[2,3-b][1,5]benzodiazepin-5-on;
(E)-6,11-Dihydro-11-[6-(1-piperidinyl)-2-hexenyl]-5H-pyrido[2,3-b][1,5]benzodiazepin-5-on;
(Z)-6,11-Dihydro-11-[6-(1-piperidinyl)-2-hexenyl]-5H-pyrido[2,3-b][1,5]benzodiazepin-5-on;
6,11-Dihydro-11-[6-(hexahydro-1H-1-azepinyl)-2-hexinyl]-5H-pyrido[2,3-b][1,5]benzodiazepin-5-on;
6,11-Dihydro-11-[1,1-dimethyl-6-(1-piperidinyl)-2-hexinyl]-5H-pyrido[2,3-b][1,5]benzodiazepin-5-on;
6,11-Dihydro-11-[6-(4-methyl-1-piperazinyl)hexyl]-5H-pyrido[2,3-b][1,5]benzodiazepin-5-on;
6,11-Dihydro-11-[6-(1-pyrrolidinyl)hexyl]-5H-pyrido[2,3-b][1,5]benzodiazepin-5-on;
5,11-Dihydro-2-methyl-11-[6-(1-piperidinyl)hexyl]-6H-pyrido[2,3-b][1,4]benzodiazepin-6-on;
5,11-Dihydro-8-methyl-11-[6-(1-piperidinyl(hexyl]-6H-pyrido[2,3-b][1,4]benzodiazepin-6-on;
5,11-Dihydro-9-methyl-11-[6-(1-piperidinyl)hexyl]-6H-pyrido[2,3-b][1,4]benzodiazepin-6-on;
5,11-Dihydro-8-ethyl-11-[6-(1-piperidinyl)hexyl)-6H-pyrido[2,3-b][1,4]benzodiazepin-6-on;
8-Chlor-5,11-dihydro-11-[6-(1-piperidinyl)hexyl]-6H-pyrido[2,3-b][1,4]benzodiazepin-6-on;
9-Chlor-5,11-dihydro-11-[6-(1-piperidinyl)hexyl]-6H-pyrido[2,3-b][1,4]benzodiazepin-6-on;
9-Brom-5,11-dihydro-11-[6-(1-piperidinyl)hexyl]-6H-pyrido[2,3-b][1,4]benzodiazepin-6-on;
5,11-Dihydro-8-fluor-11-[6-(1-piperidinyl)hexyl]-6H-pyrido[2,3-b][1,4]benzodiazepin-6-on;
5,10-Dihydro-5-[6-(1-piperidinyl)hexyl]-11H-dibenzo[b,e][1,4]diazepin-11-on;
5,10-Dihydro-5-[6-(4-methyl-1-piperazinyl)hexyl]-11H-dibenzo[b,e][1,4]diazepin-11-on;
5,10-Dihydro-5-[6-(hexahydro-1H-1-azepinyl)hexyl-11H-dibenzo[b,e][1,4]diazepin-11-on-hydrochlorid.
5,10-Dihydro-5-[6-(1-pyrrolidinyl)hexyl]-11H-dibenzo[b,e][1,4]diazepin-11-on-hydrochlorid;
5,10-Dihydro-5-[6-[4-[2-(1,3-dioxolan-2-yl)ethyl]-1-piperazinyl]hexyl]-11H-dibenzo[b,e][1,4]diazepin-11-on;
4,9-Dihydro-3-methyl-4-[6-(1-piperidinyl)-2-hexinyl]-10H-thieno[3,4-b][1,5]benzodiazepin-10-on;
4,9-Dihydro-4-[6-(1-piperidinyl)-2-hexinyl]-10H-thieno[3,4-b][1,5]benzodiazepin-10-on;
(E)-4,9-Dihydro-3-methyl-4-[6-(1-piperidinyl)-2-hexenyl]-10H-thieno[3,4-b][1,5] benzodiazepin-10-on;
(Z)-4,9-Dihydro-3-methyl-4-[6-(1-piperidinyl)-2-hexenyl]-10H-thieno[3,4-b][1,5] benzodiazepin-10-on;
4,9-Dihydro-4-[-6-(hexahydro-1H-1-azepinyl)-2-hexinyl]-3-methyl-10H-thieno[3,4-b][1,5]benzodiazepin-10-on;
4,9-Dihydro-4-[1,1-dimethyl-6-(1-piperidinyl)-2-hexinyl]-3-methyl-10H-thieno[3,4-b][1,5]benzodiazepin-10-on;
4,9-Dihydro-3-methyl-4-[6-(4-methyl-1-piperazinyl)hexyl]-10H-thieno[3,4-b][1,5] benzodiazepin-10-on;
4,9-Dihydro-3-methyl-4-[6-(1-pyrrolidinyl)hexyl]-10H-thieno[3,4-b][1,5]benzodiazepin-10-on;
3-Chlor-4,9-dihydro-4-[6-(1-piperidinyl)-2-hexinyl]-10H-thieno[3,4-b][1,5]benzodiazepin-10-on;
1,3-Dimethyl-4-[6-(1-piperidinyl)-2-hexinyl]-10H-thieno[3,4-b][1,5]benzodiazepin-10-on;
1,3-Dimethyl-4-[6-(hexahydro-1H-1-azepinyl)-2-hexinyl]-1,4,9,10-tetrahydropyrrolo[3,2-b][1,5]benzodiazepin-10-on;
(E)-1,3-Dimethyl-4-[6-(1-piperidinyl)-3-hexenyl]-1,4,9,10-tetrahydropyrrolo[3,2-b][1,5]benzodiazepin-10-on;
1,3-Dimethyl-4-[1,1-dimethyl-6-(1-piperidinyl)-2-hexinyl]-1,4,9,10-tetrahydropyrrolo[3,2-b][1,5]benzodiazepin-10-on;
1,3-Dimethyl-4-[6-(4-methyl-1-piperazinyl)hexyl]-1,4,9,10-tetrahydropyrrolo[3,2-b][1,5]benzodiazepin-10-on;
1,3-Dimethyl-4-[6-(1-pyrrolidinyl)hexyl]-1,4,9,10-tetrahydropyrrolo[3,2-b][1,5] benzodiazepin-10-on;
3-Chlor-1-methyl-4-[6-(1-piperidinyl)-2-hexinyl]-1,4,9,10-tetrahydropyrrolo[3,2-b][1,5]benzodiazepin-10-on;
1-Methyl-4-[6-(1-piperidinyl)-2-hexinyl]-1,4,9,10-tetrahydropyrrolo[3,2-b][1,5] benzodiazepin-10-on;
1,3-Dimethyl-4-[6-(1-piperidinyl)-2-hexinyl]-1,4,9,10-tetrahydropyrazolo[4,3-b][1,5]benzodiazepin-10-on;
1,3-Dimethyl-4-[6-hexahydro-1H-1-azepinyl)-2-hexinyl]-1,4,9,10-tetrahydropyrazolo[4,3-b][1,5]benzodiazepin-10-on;
(Z)-1,3-Dimethyl-4-[6-(1-piperidinyl)-2-hexenyl]-1,4,9,10-tetrahydropyrazolo[4,3-b][1,5]benzodiazepin-10-on;
1,3-Dimethyl-4-[1,1-dimethyl-6-(1-piperidinyl)hexyl]-1,4,9,10-tetrahydropyrazolo[4,3-b][1,5]benzodiazepin-10-on;
1,3-Dimethyl-4-[6-(4-methyl-1-piperazinyl)hexyl]-1,4,9,10-tetrahydropyrazolo[4,3-b][1,5]benzodiazepin-10-on;
1,3-Dimethyl-4-[6-(1-pyrrolidinyl)hexyl]-1,4,9,10-tetrahydropyrazolo[4,3-b][1,5]benzodiazepin-10-on;
1-Methyl-4-[6-(1-piperidinyl)-2-hexinyl]-1,4,9,10-tetrahydropyrazolo[4,3-b][1,5]benzodiazepin-10-on;

4-[6-[2-[(Diethylamino)methyl]-1-piperidinyl]hexyl]-1-methyl1,4,9,10-tetrahydropyrazolo[4,3-b][1,5]benzodiazepin-10-on.

Erfindungsgemäß erhält man die neuen basisch substituierten kondensierten Diazepinone der allgemeinen Formel I nach folgenden Verfahren:

a.) Basisch substituierte kondensierte Diazepinone der allgemeinen Formel I, in der $A^1$, $A^2$, $R^1$, $R^2$, $R^3$, $R^4$, $X^1$ und $X^2$ wie eingangs definiert sind und der zweiwertige Rest 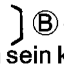 gleichfalls die angegebenen Bedeutungen hat mit der Einschränkung, daß $R^7$ kein Wasserstoffatom sein kann, erhält man durch Überführung von Tricyclen der allgemeinen Formel II,

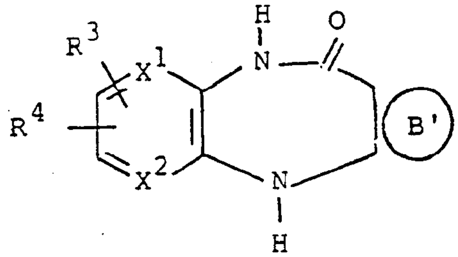

(II)

in der

die Reste $R^3$, $R^4$, $X^1$ und $X^2$ wie eingangs definiert sind und 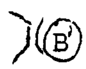 einen der zweiwertigen Reste S, U, V, W oder T'

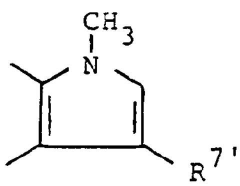

(T')

darstellt, wobei $R^{7'}$ ein Chloratom oder eine Methylgruppe ist,
in die entsprechenden Dilithiumsalze der allgemeinen Formel III,

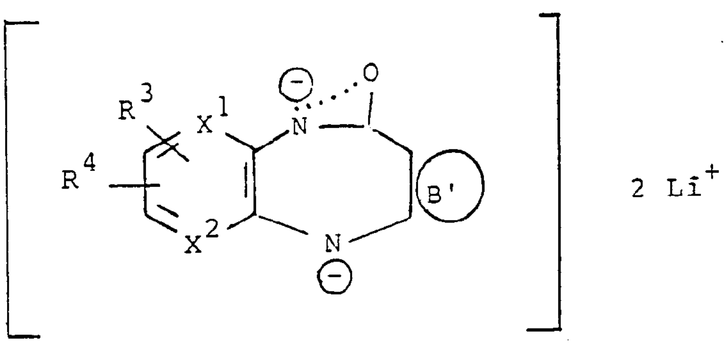

(III)

und anschließende Alkylierung mit einer Verbindung der allgemeinen Formel IV,

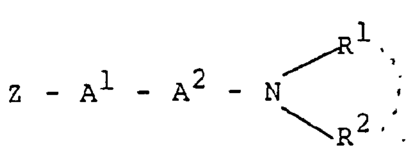

(IV)

in der $A^1$, $A^2$, $R^1$ und $R^2$ die eingangs angegebenen Bedeutungen haben und Z ein Chlor-, Brom- oder Iodatom, die Methansulfonyloxy-, die Ethansulfonyloxy- oder eine Arensulfonyloxygruppe, beispielsweise die Benzensulfonyloxy-, die p-Toluolsulfonyloxy oder die 4-Brombenzensulfonyloxygruppe, darstellt. Die Umsetzung wird üblicherweise als Eintopfverfahren durchgeführt, das heißt: Die Dilithiumsalze der allgemeinen Formel III werden nicht isoliert sondern in situ erzeugt und nach ihrer Entstehung im gleichen Reaktionsmedium sofort weiter umgesetzt.

Die Überführung der Tricyclen der allgemeinen Formel II in die Dilithiumsalze der allgemeinen Formel III gelingt durch Einwirkung von wenigstens 2 Mol eines Lithiumalkyls, insbesondere von n-Butyllithium, n-Butyllithium in Gegenwart von Tetramethylethylendiamin, t-Butyllithium, eines Lithiumdialkylamids, insbesondere von Lithiumdiisopropylamid, Lithiumdicyclohexylamid, Lithiumisopropylcyclohexylamid, oder von Lithiumarylen, z. B. von Phenyllithium. Die Erzeugung der Dilithiumsalze und die nachfolgende Alkylierung erfolgt in einem organischen Lösungsmittel und bei Temperaturen zwischen -80 und +70°C, vorzugsweise aber zwischen -10 und +30°C. Als organische Lösungsmittel dienen solche, die für Umsetzungen mit Lithiumalkylen, Lithiumdialkylamiden und Lithiumarylen gebräuchlich sind; besonders vorteilhaft ist die Verwendung von Ethern, wie Diethylether, Dioxan oder Tetrahydrofuran, von aliphatischen Kohlenwasserstoffen, wie Hexan, oder von Gemischen hiervon, gegebenenfalls auch in Gegenwart von Hexamethylphosphorsäuretriamid, 1,3-Dimethyl-2-imidazolidinon oder 1,3-Dimethyl-3,4,5,6-tetrahydro-2(1H)-pyrimidinon als Cosolventien. Kurze Zeit nach Beendigung der Zugabe des Lithiumalkyls bzw. Lithiumaryls gibt man die stöchiometrische Menge des jeweiligen Alkylierungsmittels der allgemeinen Formel IV hinzu und rührt zur Vervollständigung der Umsetzung einige Zeit bei Zimmertemperatur.

b.) Basisch substituierte kondensierte Diazepinone der allgemeinen Formel I, in der $A^1$, $R^1$, $R^2$, $R^3$, $R^4$, $X^1$ und $X^2$ wie eingangs definiert sind, $A^2$ eine geradkettige, drei bis sieben Kohlenstoffatome umfassende gesättigte Alkylenkette darstellt, die zusätzlich methylsubstituiert sein kann, und der zweiwertige Rest

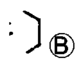 die oben angegebenen Bedeutungen hat mit der Einschränkung, daß $R^7$ kein Wasserstoffatom ist, erhält man durch Überführung von (Arylmethyl)-tricyclen der allgemeinen Formel V,

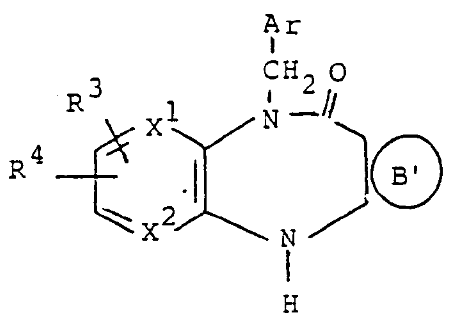

(V)

in der

die Reste 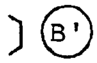, $R^3$, $R^4$, $X^1$ und $X^2$ wie oben definiert sind und
Ar einen gegebenenfalls durch jeweils ein bis zwei Methyl- und/oder Methoxygruppen substituierten phenylrest darstellt, in das entsprechende Metallsalz der allgemeinen Formel Va,

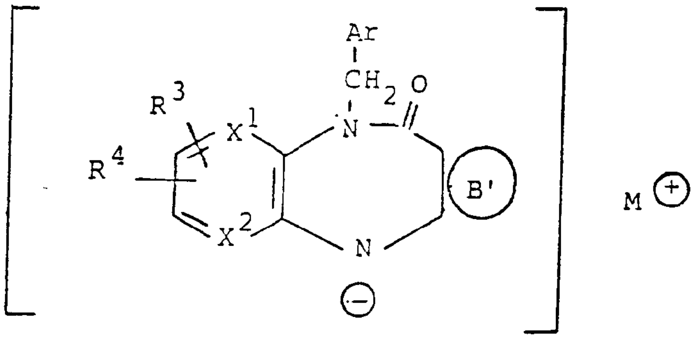

(Va)

in der

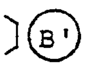

, $R^3$, $R^4$, $X^1$, $X^2$ und Ar wie vorstehend definiert sind und M ein Alkalimetall, wie Lithium, Natrium, Kalium, Rubidium oder Cäsium oder den Rest MgHal bedeutet, wobei Hal ein Chlor-, Brom- oder Iodatom ist, anschließende Alkylierung mit einer Verbindung der allgemeinen Formel IV,

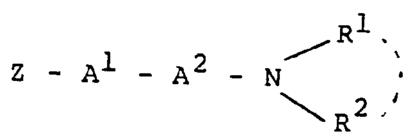

(IV)

in der
$A^1$, $R^1$, $R^2$ und Z wie oben definiert sind und $A^2$ eine geradkettige, drei bis sieben Kohlenstoffatome umfassende gesättigte Alkylenkette darstellt,
und abschließende acidolytische Entfernung der Arylmethyl-Gruppe.

Die Salze der allgemeinen Formel Va werden vorteilhafterweise unmittelbar vor der Umsetzung im Reaktionsgemisch erzeugt, beispielsweise durch Einwirkung von Lithiumalkylen, etwa von n-Butyllithium, n-Butyllithium in Gegenwart von Tetramethylethylendiamin, von t-Butyllithium, von Lithiumdialkylamiden, z. B. von Lithiumisopropylamid, Lithiumdicyclohexylamid, Lithiumdiisopropylcyclohexylamid, von Lithiumarylen, wie beispielsweise von Phenyllithium, von Alkalimetallhydroxiden, wie Lithium-, Natrium- oder Kaliumhydroxid, von Alkalimetallhydriden, wie beispielsweise von Natrium- oder Kaliumhydrid, oder von Alkalimetallamiden, wie beispielsweise Natrium- oder Kaliumamid, oder von Grignard-Reagenzien, wie etwa von Methylmagnesiumiodid, Ethylmagnesiumbromid oder Phenylmagnesiumbromid, auf einen entsprechenden (Arylmethyl)-tricyclus der allgemeinen Formel V. Die Metallierung wird üblicherweise in einem inerten organischen Lösungsmittel und bei Temperaturen zwischen -100°C und dem Siedepunkt des jeweiligen Reaktionsgemisches durchgeführt. Verwendet man zur Metallierung Lithiumalkyle, Lithiumaryle, Lithiumdialkylamide oder Grignard-Reagenzien, so bevorzugt man als Lösungsmittel Ether, wie Tetrahydrofuran, Diethylether oder Dioxan, evtl. im Gemisch mit aliphatischen oder aromatischen Kohlenwasserstoffen, wie Hexan oder Benzol und arbeitet bei Temperaturen zwischen -20 und +80°C; bei Metallierungen mit Alkalimetallhydriden und Alkalimetallamiden kommen als Lösungsmittel neben den genannten auch Xylol, Toluol, Acetonitril, Dimethylformamid und Dimethylsulfoxid in Betracht, bei Verwendung von Akalimetallhydroxiden auch Alkohole, wie Ethanol, Methanol sowie aliphatische Ketone, wie etwa Aceton, sowie Gemische dieser Solventien mit Wasser. Die anschließende Alkylierung mit einer Verbindung der allgemeinen Formel IV erfolgt ähnlich im Verfahren a) beschrieben, wobei zur Vervollständigung der Umsetzung auch längere Zeit auf Temperaturen bis zum Siedepunkt des Lösungsmittelgemisches erhitzt werden kann.

Die acidolytische Abspaltung der Arylmethyl-Gruppe aus den so erhaltenen Verbindungen der allgemeinen Formel VI,

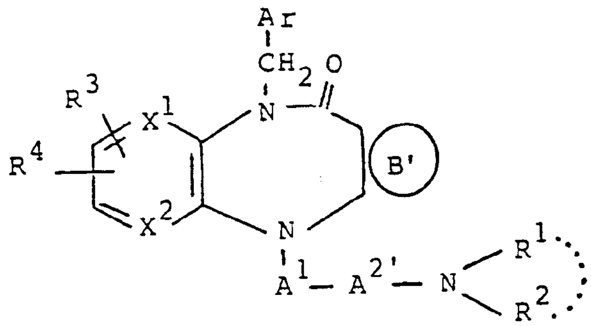

(VI)

in der

$A^1$, $R^1$, $R^2$, $R^3$, $R^4$, $X^1$ und $X^2$ wie eingangs definiert sind, $A^{2\prime}$ eine geradkettige, drei bis sieben Kohlenstoffatome umfassende gesättigte, gegebenenfalls methylsubstituierte Alkylenkette darstellt, der zweiwertige Rest 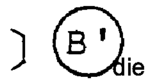die eingangs für 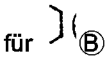 angegebenen Bedeutungen hat mit der Maßgabe, daß $R^7$ kein Wasserstoffatom ist, erfolgt durch Umsetzung mit starken Säuren oder Lewis-Säuren und bei Temperaturen zwischen -20 und +150°C. Als starke Säuren werden Schwefelsäure, Methansulfonsäure, konz. Phosphorsäure, besonders aber polyphosphorsäure eingesetzt, wobei sich im Falle der Verwendung von Phosphorsäure und Polyphosphorsäure ein Zusatz von Benzol, Toluol, Phenol, Anisol oder Veratrol zum Abfangen der entstandenen (Arylmethyl)-Kationen besonders bewährt. Verwendet man zur Entfernung der Arylmethyl-Gruppe Lewis-Säuren, wie beispielsweise Aluminiumchlorid oder Aluminiumbromid, so sind als Lösungsmittel aromatische Kohlenwasserstoffe, wie Benzol, Toluol, Anisol, sowie Gemische dieser Aromaten mit Dichlormethan geeignet.

c.) Basisch substituierte kondensierte Diazepinone der allgemeinen Formel I, in der $A^1$, $R^1$, $R^2$, $R^4$, $X^1$ und $X^2$ wie eingangs definiert sind, $A^2$ eine geradkettige, drei bis sieben Kohlenstoffatome umfassende gesättigte Alkylenkette darstellt, die zusätzlich methylsubstituiert sein kann, $R^3$ ein Wasserstoffatom oder eine Alkylgruppe mit 1 bis 4 C-Atomen ist und 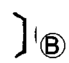 einen der zweiwertigen Reste S, T, U oder W darstellt mit der Einschränkung, daß $R^5$, $R^6$ und $R^7$ keine Chlor- bzw. Bromatome bedeuten, erhält man auch dadurch, daß man Verbindungen der allgemeinen Formel VIa,

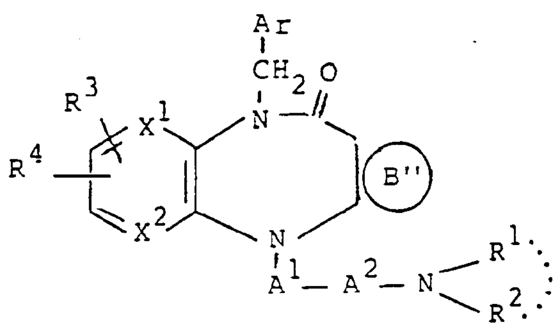

(VIa)

in der

die Reste $A^1$, $A^2$, $R^1$, $R^2$, $R^3$, $R^4$, $X^1$, $X^2$ und Ar die angegebenen Bedeutungen haben und 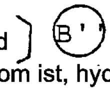 einen der zweiwertigen Reste S, T, U oder W darstellt mit der Maßgabe, daß $R^7$ kein Wasserstoffatom ist, hydrogenolysiert.

Die Hydrogenolyse wird in Gegenwart von Katalysatoren von Metallen aus der VIII. Nebengruppe des Periodensystems der Elemente, beispielsweise von Palladium auf Tierkohle, Palladiumhydroxid, Palladium auf Bariumsulfat, Raney-Nickel oder Raney-Cobalt, bei Wasserstoffdrucken von 1 bis 300 bar und Temperaturen von 0°C bis 130°C und in Gegenwart von Lösungsmitteln, beispielsweise Alkoholen, wie Methanol, Ethanol; Ethern wie Dioxan, Tetrahydrofuran; Estern, wie beispielsweise Essigsäureethylester; von Carbonsäuren , beispielsweise Essigsäure, Gemischen von Essigsäure und Acetanhydrid und gegebenenfalls in Gegenwart von

Mineralsäuren, beispielsweise Chlorwasserstoffsäure, Phosphorsäure oder Perchlorsäure, durchgeführt.

In den Ausgangsverbindungen der allgemeinen Formel VIa, die in völliger Analogie zu den unter b) angegebenen Verfahren hergestellt werden können, etwa enthaltene Chlor- oder Bromsubstituenten werden hierbei in der Regel durch Wasserstoff ersetzt, in $A^2$ etwa vorhandene Doppel- oder Dreifachbindungen aufhydriert.

d.) Basisch substituierte kondensierte Diazepinone der allgemeinen Formel I, in der $A^1$, $R^1$, $R^2$, $R^4$, $X^1$ und $X^2$ wie eingangs definiert sind, $A^2$ eine geradkettige, gegebenenfalls eine Doppelbindung enthaltende und drei bis sieben Kohlenstoffatome umfassende Alkylenkette darstellt, die zusätzlich methylsubstituiert sein kann, $R^3$ ein Wasserstoffatom oder eine Alkylgruppe mit 1 bis 4 C-Atomen ist und 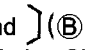 einen der zweiwertigen Reste S, T, U oder W darstellt mit der Einschränkung, daß $R^5$, $R^6$ und $R^7$ keine Chlor- oder Bromatome bedeuten,

erhält man durch katalytische Hydrierung einer Verbindung der allgemeinen Formel VII,

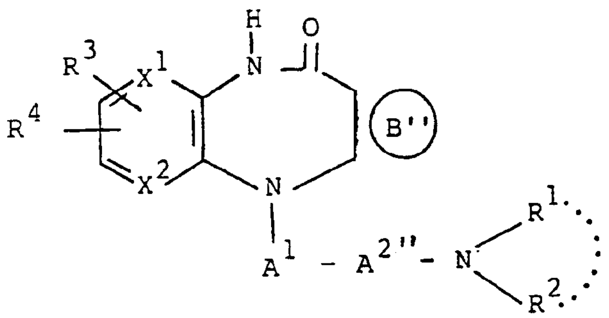

(VII)

in der

die Reste $A^1$, $R^1$, $R^2$, $R^3$, $R^4$, $X^1$, $X^2$ und 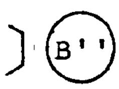 die angegebenen Bedeutungen haben und $A^{2''}$ eine geradkettige, eine Dreifachbindung enthaltende und drei bis sieben Kohlenstoffatome umfassende Alkylenkette ist, die zusätzlich methylsubstituiert sein kann.

Die katalytische Hydrierung wird in Gegenwart von Metallen aus der VIII. Nebengruppe des Periodensystems der Elemente, beispielsweise von Palladium auf Tierkohle, Palladium auf Bariumsulfat, Raney-Nickel oder Raney-Kobalt, und bei Wasserstoffdrucken von 0,1 bis 300 bar und Temperaturen von 0 bis 130°C, vorzugsweise bei Zimmertemperatur, und in Gegenwart von Lösungsmitteln, beispielsweise Alkoholen wie Methanol oder Ethanol, Ethern wie Dioxan, Diethylether oder Tetrahydrofuran, Carbonsäuren wie Essigsäure oder tertiären Aminen wie Triethylamin durchgeführt.

In den Ausgangsmaterialien der Formel VII etwa vorhandenes Halogen wird mit der Ausnahme von Fluor bei der katalytischen Hydrierung in der Regel abgespalten.

Zur Herstellung von Verbindungen der Formel I, in der $A^2$ eine eine Doppelbindung enthaltende Alkylengruppe darstellt, wird ausgehend von einer Verbindung der Formel VII vorzugsweise in der Weise verfahren, daß man die katalytische Hydrierung entweder nach der Aufnahme von 1 Mol Wasserstoff abbricht und/oder in Gegenwart eines desaktivierten Katalysators, z. B. von mit Blei oder Cadmium desaktiviertem Palladium auf Calciumcarbonat (Lindlar-Katalysator), von Palladium auf Bariumsulfat unter Zusatz von Chinolin oder von P2-Nickel in Anwesenheit von Ethylendiamin, durchgeführt.

Hierbei entstehen vorwiegend solche Verbindungen der Formel I, die durch Z-Geometrie hinsichtlich der olefinischen Doppelbindung charakterisiert sind.

Selbstverständlich kann eine so erhaltene Verbindung der Formel I, in der $A^2$ eine eine Doppelbindung enthaltende Alkylengruppe darstellt, anschließend - wie oben beschrieben - zu einer Verbindung der Formel I, in der $A^2$ eine gesättigte Alkylengruppe darstellt, weiterhydriert werden.

e.) Unter die allgemeine Formel I fallende basisch substituierte kondensierte Diazepinone der allgemeinen Formel Ia,

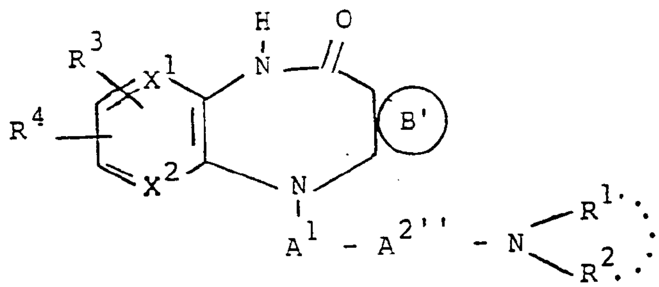

(Ia)

in der

die Reste $A^1$, $A^{2''}$, $R^1$, $R^2$, $R^3$, $R^4$, $X^1$, $X^2$ und 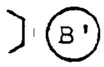 wie oben definiert sind, erhält man aus kondensierten Tricyclen der allgemeinen Formel VIII,

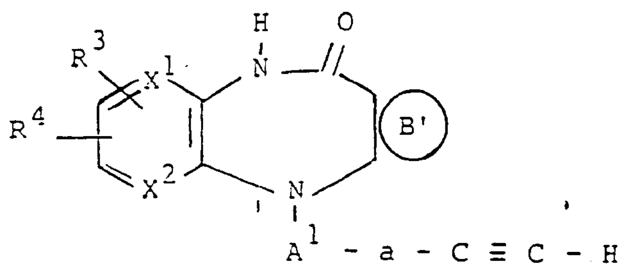

(VIII)

in der

$A^1$, 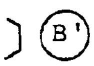, $R^3$, $R^4$, $X^1$ und $X^2$ die angegebenen Bedeutungen haben und a eine Einfachbindung oder eine geradkettige, gesättigte, gegebenenfalls durch eine Methylgruppe substituierte Alkylenkette mit höchstens 4 Kohlenstoffatomen darstellt,
durch Aminoalkylierung mit Verbindungen der allgemeinen Formel IX,

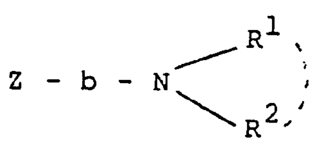

(IX)

in der
$R^1$, $R^2$ und Z wie oben angegeben definiert sind und
b eine geradkettige, gesättigte, gegebenenfalls durch eine Methylgruppe substituierte Alkylengruppe mit ein bis vier Kohlenstoffatomen in der Kette bedeutet.

Die Aminoalkylierung wird unter Umständen vorteilhaft nach vorheriger Metallierung und/oder in Gegenwart von katalytisch wirkenden Kupfer-Verbindungen druchgeführt.

Will man beispielsweise in ein 1-Alkin der allgemeinen Formel VIII den (Dialkylamino)methyl- bzw. 1-(Dialkylamino)-ethyl-Rest einführen, so erwärmt man das Ausgangsmaterial der allgemeinen Formel VIII in einem polaren Lösungsmittel mit Formaldehyd bzw. Paraformaldehyd oder Acetaldehyd bzw. Paraldehyd oder Metaldehyd und einem sekundären Amin der allgemeinen Formel X,

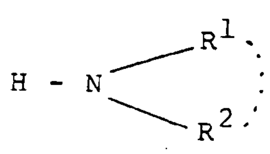

(X)

in der

$R^1$ und $R^2$ wie eingangs angegeben definiert sind. Bevorzugt wird die Umsetzung in Gegenwart von Kupfersalzen, beispielsweise von Kupfer (I)-chlorid, Kupfer(I)-bromid, Kupfer(I)-iodid, Kupfer(II)-acetat oder Kupfer(II)-sulfat. Als polare Lösungsmittel kommen beispielsweise Dimethylformamid, Dimethylacetamid, Tetrahydrofuran, 1,4-Dioxan, Dibutylether, tert.-Butanol oder Gemische mit Wasser in Betracht. Geeignete Reaktionstemperaturen liegen zwischen 20°C und dem Siedepunkt des Reaktionsgemisches. Die günstigsten Ausbeuten werden in schwach alkalischem Reaktionsmedium bei einem pH-Wert von ca. 9 erzielt. Wirksames Agens dieser Reaktion ist der in der Regel nicht isolierte α-Aminoalkohol der Formel IXa,

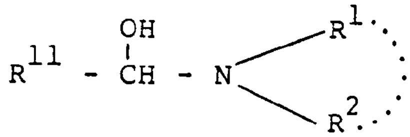

(IXa)

worin

$R^1$ und $R^2$ wie oben definiert sind und $R^{11}$ das Wasserstoffatom oder die Methylgruppe ist. Statt eines Gemisches aus Formaldehyd bzw. Acetaldehyd und einem sekundären Amin der allgemeinen Formel X kann man auch die literaturbekannten oder in Analogie zu vorbeschriebenen Verfahren leicht herstellbaren Aminale der allgemeinen Formel IXb,

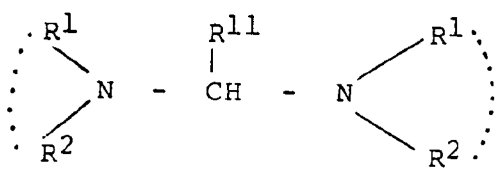

(IXb)

oder α-Alkoxyamine der Formel IXc einsetzen, worin

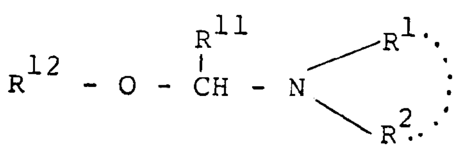

(IXc)

$R^1$, $R^2$ und $R^{11}$ die angegebenen Bedeutungen haben und $R^{12}$ einen Alkylrest mit 1 bis 10 C-Atomen darstellt. Bei der Reaktion mit α-Alkoxyaminen der Formel IXc wird die Ausgangsverbindung der allgemeinen Formel VIII bevorzugt als Grignard-Verbindung der nachfolgend aufgeführten allgemeinen Formel VIIIa, in der M=MgHal ist, eingesetzt.

Zur Alkylierung mit Verbindungen der allgemeinen Formel IX, in der b eine gegebenenfalls methylsubstituierte 1,2-Ethylen-, 1,3-Propylen- oder 1,4-Butylengruppe bedeutet, überführt man die Ausgangsmaterialien der allgemeinen Formel VIII zunächst in Metallsalze der allgemeinen Formel VIIIa,

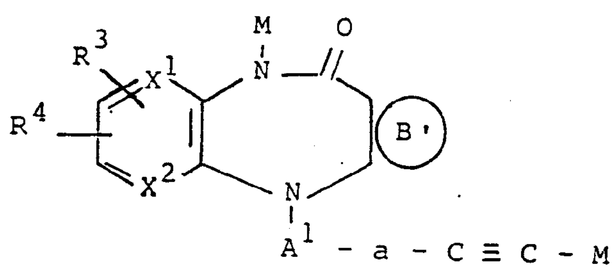

(VIIIa)

worin

a, $A^1$, 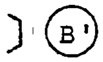 , $R^3$, $R^4$, $X^1$ und $X^2$ wie angegeben definiert sind und

M ein Alkalimetallatom, bevorzugt Lithium, oder den Rest MgHal bedeutet, wobei Hal ein Chlor-, Brom- oder Iodatom ist, und setzt sie ohne Isolierung mit den alkylierenden Agenzien der allgemeinen Formel IX um. Zur Lithiierung verwendet man exakt zwei Mol des Metallierungsreagens, beispielsweise Lithiumalkyle, wie n-Butyllithium, n-Butyllithium in Gegenwart von Tetramethylethylendiamin, tert. Butyllithium oder Methyllithium, Lithiumamide, wie Lithiumdiisopropylamid, Lithiumamid, Lithiumdicyclohexylamid oder Lithiumisopropylcyclohexylamid, Lithiumaryle, wie Phenyllithium, oder auch metallisches Lithium, etwa in Gegenwart von Phenanthren. Soll in der Formel VIIIa M den Rest MgHal bedeuten, so setzt man die 1-Alkine der allgemeinen Formel VIII mit wenigstens 2 Mol eines Grignard-Reagens um, beispielsweise mit Methylmagnesiumiodid, Ethylmagnesiumbromid oder Phenylmagnesiumbromid. Die Metallierung und nachfolgende Aminoalkylierung wird üblicherweise in einem organischen Lösungsmittel und bei Temperaturen zwischen -80°C und +150°C, vorzugsweise zwischen -40°C und +140°C durchgeführt. Als organische Lösungsmittel dienen solche, die für Umsetzungen mit Lithium- und Magnesium-organischen Verbindungen gebräuchlich sind; besonders vorteilhaft ist die Verwendung von Ethern, wie Diethylether, Tetrahydrofuran, Dioxan oder 1,2-Dimethoxyethan, von flüssigem Ammoniak, gegebenenfalls im Gemisch und unter Verwendung von polaren Cosolventien, wie Hexamethylphosphorsäuretriamid, Dimethylsulfoxid, 1,3-Dimethyl-2-imidazolidinon oder 1,3-Dimethyl-3,4,5,6-tetrahydro-2(1H)-pyrimidinon. Ein Zusatz von Katalysatoren, insbesondere von wasserfreien Kupfer(I)-salzen, beispielsweise von Kupfer(I)-chlorid, Kupfer(I)-iodid, Kupfer(I)iodid in Gegenwart von Lithium- oder Natriumiodid, von Kupfer(I)-cyanid, hat in der Regel einen beschleunigenden Einfluß auf den Verlauf der Reaktion.

Die erfindungsgemäßen basisch substituierten kondensierten Diazepinone der allgemeinen Formel I enthalten bis zu drei Chiralitätszentren in der Seitenkette und können daher gegebenenfalls in bis zu vier diastereomeren Formen, die ihrerseits in (+)- und (-)-Enantiomere gespalten werden können, auftreten. Diastereomere cis-/trans-Formen sind auch möglich, wenn $A^2$ in den beanspruchten Verbindungen der allgemeinen Formel I einen eine olefinische Doppelbindung enthaltenden Alkylenrest bedeutet. Nur ein Chiralitätszentrum enthaltende Verbindungen der allgemeinen Formel I liegen als Racemate vor, die in die jeweiligen (+)- und (-)-Antipoden aufgetrennt werden können.

Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z. B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Racemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Enantiomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres der unter die allgemeine Formel I fallenden Verbindungen wird auch dadurch erhalten, daß man die oben beschriebenen Synthesen mit nur einem Enantiomeren der allgemeinen Formeln IV, VI, VIa, VIII, VIIIa oder IX durchführt.

Die Tricyclen der allgemeinen Formel II sind aus der Patentliteratur bekannt oder können in enger Anlehnung an publizierte Verfahren aus gängigen Ausgangsmaterialien synthetisiert werden.

Die Ausgangsverbindungen der allgemeinem Formel IV sind literaturbekannt oder in enger Anlehnung an vorbeschriebene Verfahren zugänglich. Beispielsweise lassen sich die Aminoalkohole der allgemeinem Formel IVa,

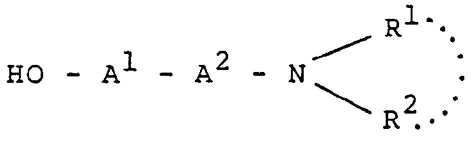

(IVa)

in der

$A^1$, $A^2$, $R^1$ und $R^2$ die eingangs angegebenen Bedeutungen haben, durch Behandlung mit konzentrierter Salzsäure bzw. Thionylchlorid, mit konzentrierter Bromwasserstoffsäure bzw. Thionylbromid, Iodwasserstoffsäure oder Methansulfonsäure-, Ethansulfonsäure- oder Arensulfonsäurederivaten, bevorzugt den entsprechenden Sulfonsäurechloriden oder -bromiden, in Verbindungen der allgemeinen Formel IV überführen, in der Z ein Chlor-, Brom- oder Iodatom, die Methansulfonyloxy-, die Ethansulfonyloxy- oder eine Arensulfonyloxygruppe bedeutet. Die Verbindungen der allgemeinen Formel IV kann man aber auch dadurch erhalten, daß man sekundäre Amine der allgemeinen Formel X mit einer bifunktionelle Verbindung der allgemeinen Formel IVb,

$$Z - A^1 - A^2 - Z' \qquad (IVb)$$

in der
$A^1$, $A^2$ und Z wie eingangs definiert sind
und Z′, das gleich oder verschieden von Z sein kann, die gleichen Bedeutungen annehmen darf wie Z, alkyliert.

Die Ausgangsverbindungen der allgemeinen Formel V synthetisiert man in Anlehnung an literaturbekannte Methoden durch Arylmethylierung von Tricyclen der allgemeinen Formel II in Gegenwart von Basen, z. B. von Natriumhydrid, Kaliumcarbonat, Natriumcarbonat, Natriumhydroxid oder Triethylamin (siehe auch: DAS 24 24 811 vom 22.05.1974/18.12.1975/02.10. 1980).

Ausgangsverbindungen der allgemeinen Formel VIa erhält man in völliger Analogie zu den Verbindungen der allgemeinen Formel VI.

Ausgangsverbindungen der allgemeinen Formel VII stellen eine Untergruppe der Verbindungen der allgemeinen Formel I dar und sind zum Beispiel nach den oben angegeben Verfahren a) und e) erhältlich.

Die Ausgangsverbindungen der allgemeinen Formel VIII kann man aus den Dilithiumsalzen der allgemeinen Formel III durch Alkylierung mit Halogeniden der allgemeinen Formel XI,

$$Hal - A^1 - a - C{\equiv}C - H \qquad (XI)$$

in der
$A^1$ und a wie oben definiert sind und Hal ein Chlor-, Brom- oder Iodatom bedeutet, leicht herstellen. Die Ausgangsverbindungen der allgemeinen Formel XI sind käuflich bzw. literaturbekannt (siehe beispielsweise: K.E. Schulte und K.Th. Reiss, Chem. Ber. 86, 777-781 [1953]) oder in Anlehnung an literaturbekannte Verfahren leicht herstellbar.

Auch die Ausgangsverbindungen der allgemeinen Formel IX sind käuflich oder können aus käuflichen Vorstufen nach bekannten Methoden leicht synthetisiert werden.

Die Ausgangsverbindungen der allgemeinen Formel X sind käuflich oder bekannt (siehe beispielsweise: DE-A-36 26 095).

Die Ausgangsverbindungen der allgemeinen Formeln IXb und IXc sind nach bekanntem Vorbild aus sekundären Aminen der allgemeinen Formel X und Formaldehyd oder Acetaldehyd, bzw. aus Aminen der allgemeinen Formel X, Formaldehyd oder Acetaldehyd, sowie Alkoholen der allgemeinen Formel XII,

$$R^{12} - OH \qquad (XII)$$

in der
$R^{12}$ wie angegeben definiert ist, zugänglich.

Die basisch substituierten kondensierten Diazepinone der allgemeinen Formel I und ihre Säureadditionssalze besitzen wertvolle Eigenschaften; insbesondere besitzen sie bei völliger Hydrolysestabilität, hoher Selektivität und guter Resorption nach oraler Gabe günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase. Auf Grund ihrer antiemetischen Eigenschaften eignet sich ein Teil der Verbindungen der allgemeinen Formel I auch zur Vorbeugung gegen Reise- und Seekrankheit und angesichts ihrer günstigen Effekte auf die cerebrale Durchblutung zur Anwendung in der Geriatrie und bei Migräne. Die Verbindungen zeigen darüber hinaus infolge ihrer hohen Lipophilie in der Regel gute ZNS-Gängigkeit und werden daher zur Therapie von Erkrankungen des Zentralnervensystems, insbesondere des Morbus Alzheimer und des Morbus Parkinson, eingesetzt; beim Morbus Alzheimer beeinflussen die Verbindungen die autoregulatorische Funktion präsynaptischer Muskarinrezeptoren auf die Freisetzung von Acetylcholin und führen dadurch zu einer Verstärkung des Impulsmusters der noch vorhandenen cholinergen Fasern; beim Morbus Parkinson besteht der Vorteil des Einsatzes von Verbindungen der allgemeinen Formel I an Stelle der bisher üblichen nichtselektiven Antimuskarinika in dem Fehlen nicht tolerabler peripherer und zentraler atropinartiger Nebenwirkungen.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von

besonderer Wichtigkeit. Die folgenden Versuche zeigen, daß die erfindungsgemäßen Verbindungen diesbezüglich überraschend günstige Relationen aufweisen.

Bindungsstudien an muscarinischen Rezeptoren:

Bestimmung des $IC_{50}$-Wertes in vitro

Als Organspender dienten männliche Sprague-Dawley-Ratten mit 180-220 g Körpergewicht. Nach Entnahme von Herz und Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30°C. Die Inkubationsdauer betrug 45 Minuten. Als Radioligand wurde 0,3 nmolar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nicht-markierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$-Selektivität an der Ratte und
2. Speichelsekretionshemmende Wirkung an der Ratte
3. Hemmung der Acetylcholinwirkung auf Blase, Bronchien und Herzfrequenz des Meerschweinchens

untersucht.

1. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichelsekretionshemmende Wirkung an der Ratte

Nach Lavy und Mulder (Arch. int. Pharmacodyn. 178, 437-445, (1969)) erhielten mit 1,2 g Urethan/kg narkotisierte männliche THOM-Ratten steigende Dosen der Substanz i.v.. Die Speichelsekretion wurde durch s.c.

Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Am narkotisierten Meerschweinchen wurden 5 Minuten nach Gabe der Prüfsubstanz 10 µg/kg Acetylcholin sowohl intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

C. Untersuchungen auf mögliche Einflüsse auf Imipramin-, Histamin-Hl- oder Serotonin-S2-Rezeptoren:

Methodik:

a.) Bindungsstudien am Imipraminrezeptor

Als Organspender dienten männliche Sprague-Dawley-Ratten mit 180-220 g Körpergewicht. Die Großhirnrinde wurde entnommen und in eiskaltem Tris-HCl-Puffer (pH 7,5; 50 mM Tris, 5 mM KCl) mit einem Ultra-Turrax homogenisiert. Dieses Homogenat wurde 3mal gewaschen und bei 50 000 x g 10 Minuten zentrifugiert. Das erhaltene Pellet wurde, bezogen auf das Ausgangsgewicht, im Verhältnis 1:125 verdünnt. Im folgenden Bindungsansatz wurde diese Membranpräparation mit 1 nM $^3$H-Imipramin und einer Konzentrationsreihe der Testsubstanzen bei 0°C 60 Minuten inkubiert.

Die Beendigung der Inkubation erfolgte durch rasche Vakuumfiltration. Nach Spülung der Filter mit Puffer wurde deren Radioaktivität gemessen. Der Anteil der unspezifischen Bindung wurde als Anteil der Radioaktivität in Anwesenheit von 100 µM Desipramin definiert. Die $IC_{50}$-Werte wurden graphisch bestimmt. Die Ergebnisse sind in der Tabelle IV ersichtlich.

b.) Bindungsstudien am Serotonin-S2-Rezeptor

Für diese Versuche wurde in analoger Weise zur oben genannten Bindungsstudie am Imipraminrezeptor Großhirnrindengewebe genommen. die Homogenisation erfolgte in Tris-HCl-Puffer (50 mM Tris pH 7,7; 5,7 mM Ascorbinsäure) mit einem Ultra-Turrax. Das Homogenat wurde nun bei 50 000 x g zentrifugiert und das Pellet bezogen auf das Gewebegewicht im Verhältnis 1:250 verdünnt. Der Inkubationspuffer im Bindungsansatz enthielt zusätzlich 0,1 mM Nialamid. Die Inkubation wurde bei Zimmertemperatur mit 0,3 nM 3H-Ketanserin und einer Konzentrationsreihe der Testsubstanzen 60 Minuten durchgeführt und darauf die Ansätze unter Vakuum filtriert. Die Filter wurden dann der Radioaktivitätsmessung zugeführt. Die unspezifische Bindung wurde durch Ansätze ermittelt, die zusätzlich 3 µM Ketanserin enthielten. Die Auswertung erfolgte wie bei den Bindungsstudien am Imipraminrezeptor. Die Ergebnisse sind in der Tabelle IV enthalten.

c.) Bindungsstudien am Histamin-Hl-Rezeptor

Bei diesen Untersuchungen wurden ganze Rattenhirne ohne Kleinhirn verwendet. Die Homogenisation des Gewebes erfolgte in einem Phosphatpuffer pH 7,5 nach Sörensen mit dem Ultra-Turrax. Das Homogenat wurde nun 2mal gewaschen und mit 50 000 x g zentrifugiert. Das Pellet wurde in einer Verdünnung bezogen auf das Ausgangsgewebe von 1:100 aufgenommen. Die Inkubation der Bindungsansätze erfolgte bei Zimmertempe ratur mit 2 nM 3H-Pyrilamin und einer Konzentrationsreihe der Testsubstanzen 60 Minuten lang. Die Ansätze wurden auch hier durch Vakuumfiltration beendet und die Radioaktivität der Filter gemessen. Die unspezifische Bindung wurde mit Ansätzen bestimmt, die 10 µM Triprolidin enthielten. Die Auswertung erfolgte wie bei den Bindungsstudien im Imipraminrezeptor beschrieben. Die Ergebnisse sind in der Tabelle IV enthalten.

Nach den vorstehenden Angaben wurden beispielsweise die folgenden Verbindungen untersucht:
A = 5,11-Dihydro-11-[6-(1-piperidinyl)hexyl]-6H-pyrido[2,3-b][1,4]benzodiazepin-6-on
B = 11-[4-[2-[(Diethylamino)methyl]-1-piperidinyl]butyl]-5,11-dihydro-6H-pyrido[2,3-b][1,4]benzodiazepin-6-on,
C = 11-[4-[2-[(Diethylamino)methyl]-1-piperidinyl]-2-butinyl]-5,11-dihydro-6H-pyrido[2,3- b][1,4]benzodiazepin-6-on,
und als Vergleichssubstanzen

D = 11-[[2-[(Diethylamino)methyl]-1-piperidinyl]acetyl]-5,11-dihydro-6H-pyrido[2,3-b][1,4]benzodiazepin-6-on (siehe US-Patent Nr. 4 550 107)
E = 5,11-Dihydro-11-[(4-methyl-1-piperazinyl)acetyl]-6H-pyrido[2,3-b][1,4]benzodiazepin-6-on(Pirenzepin, siehe US-Patent Nr. 3 660 380)
und
F = Atropin

Die folgenden Tabellen enthalten die dabei gefundenen Ergebnisse:

Tabelle I:

Rezeptor-Bindungs-Tests, in vitro:

Ergebnisse:

| Substanz | Rezeptor-Bindungs-Tests $IC_{50}$ [nM] Cortex (C) | Herz (H) | Submandibularis (SM) | Selektivitätsfaktoren: $\frac{IC_{50}(SM)}{IC_{50}(H)}$ | $\frac{IC_{50}(C)}{IC_{50}(H)}$ |
|---|---|---|---|---|---|
| A | 4 | 0,6 | 10 | 16,7 | 6,7 |
| B | 20 | 2 | 80 | 40 | 10 |
| C | 200 | 18 | 700 | 38,9 | 11,1 |
| D | 1200 | 140 | 5000 | 36 | 8,6 |
| E | 100 | 1500 | 200 | 0,13 | 0,07 |
| F | 2 | 4 | 4 | 1 | 0,5 |

nM = nanomolar.

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde und Submandibularis. Es muß dar auf hingewiesen werden, daß die Verbindungen A, B und C bei erhaltener Selektivität eine beträchtlich höhere Wirkstärke im Vergleich zur Verbindung D aufweisen.

Tabelle II:

$M_1$-/$M_2$-Selektivität und Speichelsekretionshemmung an der Ratte:

Ergebnisse:

| | -log $ED_{50}$ [$Mkg^{-1}$] | | |
|---|---|---|---|
| Substanz | Herz | Blutdruck | Salivationshemmung |
| A | 7,96 | 6,53 | 6,35 |
| B | 7,40 | 6,17 | 5,50 |
| C | 7,52 | 5,96 | 5,12 |
| D | 6,42 | 5,63 | 5,00 |
| E | 5,60 | 6,94 | 6,22 |
| F | 7,94 | 7,34 | 7,60 |

Tabelle III:

Hemmung der Acetylcholinwirkung auf Blase, Bronchien und Herzfrequenz des Meerschweinchens:

Ergebnisse:

| | $-\log ED_{50}$ [$Molkg^{-1}$] | | |
|---|---|---|---|
| Substanz | Herz | Bronchien | Blase |
| A | 7,18 | 7,07 | 89 |
| B | 6,33 | 6,19 | < 5,00 |
| C | 6,61 | 5,80 | < 5,64 |
| D | 5,84 | 5,58 | 4,73 |
| E | 5,85 | 6,57 | 5,36 |
| F | 7,70 | 7.96 | 7,03 |

Tabelle IV

Einfluß auf die Imipramin-, Histamin-H1- und Serotonin-S2-Rezeptoren.

Ergebnisse:

| Substanz | Imipramin | $IC_{50}$ (nM) S2 | H1 |
|---|---|---|---|
| A | 10 000 | 20 000 | 10 000 |
| B | 60 000 | 30 000 | 9 000 |
| C | 100 000 | 40 000 | 50 000 |
| Desipramin | 200 | | |
| Ketanserin | | 15 | |
| Pyrilamin | | | 7 |

Die Tabelle zeigt, daß die angegebenen Verbindungen in den Konzentrationen, die bereits eine Hemmung des muskarinischen Rezeptors bewirken, keinen Einfluß auf den Imipramin-, Histamin-HI- oder Serotonin-S2-Rezeptor haben. In therapeutischen Dosen sind daher die mit diesen Rezeptoren verbundenen Nebenwirkungen nicht zu erwarten.

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung D eine wesentlich gesteigerte Wirkungsstärke auf. Dabei bleibt die therapeutisch nutzbare Selektivität erhalten. Dies führt zu einer geringeren Substanzbelastung des Patienten, ohne das Risiko muskarinischer Nebenwirkungen zu erhöhen.

Ferner sind die Erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Zur pharmazeutischen Anwendung lassen sich die Verbindungen der allgemeinen Formel I in an sich bekannter Weise in die üblichen pharmazeutischen Zubereitungsformen, z. B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die nachfolgenden Beispiele sollen die Erfindung näher erläutern:

Für alle Verbindungen liegen befriedigende Elementaranalysen, IR-, UV-, $^1$H-NMR-, häufig auch Massenspektren vor.

Beispiel 1

5,11-Dihydro-11-[4-(1-pyrrolidinyl)-2-butinyl]-6H-pyrido[2,3-b][1,4]benzodiazepin-6-on

a.) 5,11-Dihydro-11-(2-propinyl)-6H-pyrido[2,3-b][1,4]benzodiazepin-6-on

Zu einer Suspension von 7,4 g (0,035 Mol) 5,11-Dihydro-6H-pyrido[2,3-b][1,4]benzodiazepin-6-on in 150 ml wasserfreiem Tetrahydrofuran tropfte man unter Rühren und bei einer Reaktionstemperatur von 0 bis +10°C 44,8 ml (ca. 0,07 Mol) einer 15-proz. Lösung von n-Butyllithium in n-Hexan. Nach Beendigung der Zugabe wurde das Gemisch 30 Minuten bei Zimmertemperatur gerührt, bevor man tropfenweise eine Lösung von 4,16 g (0,035 Mol) 3-Brom-1-propin in 25 ml wasserfreiem Tetrahydrofuran zugab. Man rührte noch 2 Stunden bei Raumtemperatur, trug die Mischung in 1 l gesättigte, wäßrige Kochsalz-Lösung ein und extrahierte erschöpfend mit Essigsäureethylester. Die vereinigten Essigesterextrakte wurden noch zweimal mit je 100 ml gesättigter Kochsalz-Lösung gewaschen, über Natriumsulfat unter Zusatz von 1 g Aktivkohle getrocknet und im Vakuum eingedampft. Der Rückstand wurde an Kieselgel unter Verwendung von Dichlormethan/Essigsäureethylester 95/5 (v/v) zum Eluieren chromatographisch gereinigt. Man erhielt 8,5 g (97 % der Theorie) an farblosen Kristallen vom Fp. 199°C (Z.).

b.) 5,11-Dihydro-11-[4-(1-pyrrolidinyl)-2-butinyl]-6H-pyrido[2,3-b][1,4]benzodiazepin-6-on

Die Mischung aus 8,5 g (0,034 Mol) 5,11-Dihydro-11-(2-propinyl)-6H-pyrido[2,3-b][1,4]benzodiazepin-6-on, 50 ml wasserfreiem Dioxan, 1,08 g (0,036 Mol) Paraformaldehyd, 2,77 g (0,039 Mol) Pyrrolidin und 0,2 g Kupfer(I)-chlorid wurde 1 Stunde unter Rühren und unter Rückfluß gekocht, anschließend nach dem Erkalten im Wasserstrahlvakuum eingedampft. Der Rückstand wurde zwischen Wasser und Essigsäureeethylester verteilt, die anfallende Essigester-Lösung mit 2 g Tierkohle versetzt, über Natriumsulfat getrocknet, filtriert und eingeengt. Der Rückstand wurde an Kieselgel säulenchromatographisch unter Verwendung von zunächst Essigsäureethylester, dann Dichlormethan/Cyclohexan/Methanol/konz. Ammoniak 68/15/15/2 (v/v/v/v) zum Eluieren chromatographisch gereinigt. Das nach dem Eindampfen der geeigneten Eluate erhaltene Rohprodukt wurde noch einmal aus Essigsäureethylester umkristallisiert. Ausbeute: 1,6 g (14 % der Theorie) an farblosen Kristallen vom Fp. 153°C; $R_F$ 0,5 (Merck, DC-Fertigplatten, Kieselgel 60 F-254; Fließmittel: Dichlormethan/Cyclohexan/Methanol/konz. Ammoniak 68/15/15/2 v/v/v/v).

Beispiel 2

11-[4-[2-[(Diethylamino)methyl]-1-piperidinyl]-2-butinyl]-5,11-dihydro-6H-pyrido[2,3-b][1,4]benzodiazepin-6-on

Hergestellt analog Beispiel 1b) aus 5,11-Dihydro-11-(2-propinyl)-6H-pyrido[2,3-b][1,4]benzodiazepin-6-on, Paraformaldehyd und 2-[(Diethylamino)methyl]piperidin in einer Ausbeute von 28 % der Theorie. Farblose Kristalle vom Fp. 132-134°C.

Beispiel 3

trans-5,11-Dihydro-11-[4-[(4-hydroxycyclohexyl)methylamino]-2-butinyl]-6H-pyrido[2,3-b][1,4]benzodiazepin-6-on

Hergestellt analog Beispiel 1b) aus 5,11-Dihydro-11-(2-propinyl)-6H-pyrido[2,3-b][1,4] benzodiazepin-6-on, paraformaldehyd und trans-(4-Hydroxycyclohexyl)methylamin in einer Ausbeute von 31 % der Theorie. Farblose Kristalle vom Fp. 183-184°C (nach dem Umkristallisieren aus Essigsäureethylester und Methanol).

Beispiel 4

11-[4-[(Cyclohexyl)methylamino]-2-butinyl]-5,11-dihydro-6H-pyrido[2,3-b][1,4]benzodiazepin-6-on

Hergestellt analog Beispiel 1b) aus 5,11-Dihydro-11-(2-propinyl) -6H-pyrido [2,3-b][1, 4] benzodiazepin-6-on, Paraformaldehyd und (Cyclohexyl)methylamin in einer Ausbeute von 17 % der Theorie. Farblose Kristalle vom Fp. 133-134°C (Diethylether)

Beispiel 5

5,11-Dihydro-11-[4[-1-piperidinyl)-2-butinyl]-6H-pyrido[2,3-b][1,4]benzodiazepin-6-on

Hergestellt analog Beispiel 1b) aus 5,11-Dihydro-11-(2-propinyl)-6H-pyrido [2,3-b][1,4] benzodiazepin-6-on, Paraformaldehyd und Piperidin in einer Ausbeute von 19 % der Theorie. Farblose Kristalle vom Fp. 165-168°C (Essigsäureethylester). Das Hemifumarat schmolz bei 198°C (Ethanol) unter Zersetzung.

Beispiel 6

5,11-Dihydro-11-[6-(1-piperidinyl)hexyl]-6H-pyrido[2,3-b][1,4]benzodiazepin-6-on

Zu der Suspension von 33,2 g (0,157 Mol) 5,11-Dihydro-6H-pyrido[2,3-b][1,4]benzodiazepin-6-on in 875 ml wasserfreiem Dioxan tropfte man bei Zimmertemperatur 173 ml (ca. 0,45 Mol) einer 2,6 molaren Lösung von n-Butyllithium in n-Hexan und rührte anschließend eine weitere Stunde bei der gleichen Temperatur. Dann wurde auf 70°C aufgeheizt und bei einer Reaktionstemperatur von maximal 75°C tropfenweise 83,0 g (0,034 Mol) 1-Brom-6-(1-piperidinyl)hexan zugegeben, abschließend zur Vervollständigung der Reaktion noch 4 Stunden bei 80°C gerührt. Nach dem Erkalten wurde das Lösungsmittel im Wasserstrahlvakumm abdestilliert, der Rückstand mit verdünnter wäßriger Salzsäure auf pH 7 eingestellt und die Mischung anschließend filtriert. Das Filtrat wurde mit konz. wäßriger Kalilauge alkalisch gemacht und mit Dichlormethan erschöpfend extrahiert. Die Dichlormethan-Auszüge wurden nach dem Trocknen über Natriumsulfat eingedampft, der verbliebene Rückstand mit 100 ml siedendem Diethylether digeriert und heiß filtriert. Der nach der Entfernung des Ethers verbliebene Rückstand wurde an Kieselgel (Macherey-Nagel, 0,2-0,5 mm) unter Verwendung von Dichlormethan/Methanol/Cyclohexan/Essigsäureethylester/konz. Ammoniak 59/7,5/7,5/25/1 (v/v/v/v/v) zum Eluieren chromatographisch gereinigt. Durch Eindampfen der geeigneten Fraktionen und nachfolgendes Umkristallisieren aus Acetonitril erhielt man in einer Ausbeute von 11,29 g (19 % der Theorie) ein farbloses, kristallines Produkt vom Fp. 131-132°C. Das Hydrochlorid schmolz bei 223°C (Ethanol).

Beispiel 7

5,11-Dihydro-11-[4-(4-morpholinyl)-2-butinyl]-6H-pyrido[2,3-b][1,4]benzodiazepin-6-on

Hergestellt analog Beispiel 1b) aus 5,11-Dihydro-11-(2-propinyl)-6H-pyrido[2,3-b][1,4]benzodiazepin-6-on, Paraformaldehyd und Morpholin in einer Ausbeute von 19 % der Theorie. Farblose Kristalle vom Fp. 168°C.

Beispiel 8

11-[4-[2-[(Diethylamino)methyl]-1-piperidinyl]butyl]-5,11-dihydro-6H-pyrido[2,3-b][1,4]benzodiazepin-6-on

4,3 g (0,01 Mol) 11-[4-[2-[(Diethylamino)methyl]-1-piperidinyl]2-butinyl]-5,11-dihydro-6H-pyrido[2,3- b][1,4] benzodiazepin-6-on wurden in 40 ml Ethanol gelöst und nach Zusatz von 0,5 g 10 proz. Palladium auf Tierkohle bei 3 bar Wasserstoffdruck und bei Zimmertemperatur bis zur Beendigung der Wasserstoffaufnahme hydriert. Man filtrierte, dampfte das Filtrat im Wasserstrahlvakuum ein und reinigte den verbliebenen öligen Rückstand säulenchromatographisch an Kieselgel (Macherey-Nagel 60, 0,063-0,2 mm) unter Verwendung von anfangs Essigsäureethylester, dann Essigsäureethylester/Methanol/konz. Ammoniak 90/10/2 zum Eluieren. Aus den entsprechenden Fraktionen erhielt man nach Abdampfen des Lösungsmittels und Umkristallisieren des Rückstands aus Diisopropylether farblose Kristalle vom Fp. 112°C, $R_F$ 0,75 (Merck, DC-Fertigplatten, Kieselgel 60F-254; Fließmittel: Dichlormethan/Cyclohexan/Methanol/konz. Ammoniak 68/15/15/2 v/v/v/v) Ausbeute: 2,2 g (50,5 % der Theorie).

Beispiel 9

11-[4-[2-[[(Cyclohexyl)methylamino]methyl]-1-piperidinyl]-2-butinyl]-5,11-dihydro-6H-pyrido[2,3-b][1,4]benzodiazepin-6-on

Hergestellt analog Beispiel 1b) aus 5,11-Dihydro-11-(2-propinyl)-6H-pyrido[2,3-b][1,4]benzodiazepin-6-on, Paraformaldehyd und 2-[[(Cyclohexyl)methylamino]methyl]piperidin in einer Ausbeute von 34 % der Theorie. Das Monohydrochlorid schmolz bei 205°C (Z.)

Beispiel 10

5,11-Dihydro-11-[4-(hexahydro-1H-1-azepinyl)-2-butinyl]-6H-pyrido[2,3-b][1,4]benzodiazepin-6-on

Hergestellt analog Beispiel 1b) aus 5,11-Dihydro-11-(2-propinyl)-6H-pyrido[2,3-b][1,4]benzodiazepin-6-on, Paraformaldehyd und Hexahydro-1H-azepin in einer Ausbeute von 35 % der Theorie. Farblose Kristalle vom Fp. 157°C (Essigsäureethylester).

Beispiel 11

5,11-Dihydro-11-[4-(hexahydro-1H-1-azepinyl)butyl]-6H-pyrido[2,3-b][1,4]benzodiazepin-6-on

Hergestellt analog Beispiel 8 aus 5,11-Dihydro-11-[4-(hexahydro-1H-1-azepinyl)-2-butinyl]-6H-pyrido[2,3-b][1,4]benzodiazepin-6- on in einer Ausbeute von 74 % der Theorie. Farblose Kristalle vom Fp. 99-101°C (Ether/Diisopropylether 1/1 v/v).

Beispiel 12

5,11-Dihydro-11-[6-(4-methyl-1-piperazinyl)hexyl]-6H-pyrido[2,3-b][1,4]benzodiazepin-6-on

Hergestellt analog Beispiel 6 aus 5,11-Dihydro-6H-pyrido[2,3-b][1,4]benzodiazepin-6-on, n-Butyllithium und 1-Brom-6-(4-methyl-1-piperazinyl)hexan in einer Ausbeute von 16 % der Theorie. Farblose Kristalle vom Fp. 149-150°C (aus Acetonitril unter Verwendung von Aktivkohle).

Beispiel 13

5,11-Dihydro-11-[4-(1-piperidinyl)butyl]-6H-pyrido[2,3-b][1,4]benzodiazepin-6-on

Hergestellt analog Beispiel 8 aus 5,11-Dihydro-11-[4-(1-piperidinyl)-2-butinyl]-6H-pyrido[2,3-b][1,4]benzodiazepin-6- on in einer Ausbeute von 35 % der Theorie. Farblose Kristalle vom Fp. 131-132°C (Essigsäureethylester).

Beispiel 14

5,10-Dihydro-5-[6-(1-piperidinyl)hexyl]-11H-dibenzo[b,e][1,4]-diazepin-11-on

Hergestellt analog Beispiel 6 aus 5,10-Dihydro-11H-dibenzo[b,e][1,4]diazepin-11-on, n-Butyllithium und 1-Brom-6-(1-piperidinyl)hexan in einer Ausbeute von 46 % der Theorie. Farblose Kristalle vom Fp. 124-125°C (aus Acetonitril unter Verwendung von Aktivkohle).

Beispiel 15

5,10-Dihydro-5-[6-(4-methyl-1-piperazinyl)hexyl]-11H-dibenzo[b,e][1,4]diazepin-11-on-dihydrochlorid

Hergestellt analog Beispiel 6 aus 5,10-Dihydro-11H-dibenzo[b,e][1,4]diazepin-11-on, n-Butyllithium und 1-Brom-6-(4-methyl-1-piperazinyl)hexan in einer Ausbeute von 18 % der Theorie. Das Dihydrochlorid schmolz bei 232-234°C (Acetonitril/Ethanol 1/1 v/v).

Bei der chromatographischen Reinigung wurde in einer Ausbeute von 4 % der Theorie ein farbloses kristallines Nebenprodukt vom Fp. 124-126°C (Diisopropylether/Acetonitril 9/1 v/v) isoliert, bei dem es sich auf Grund von IR-, UV-, $^1$H-NMR- und Massen-Spektren um 5,10-Dihydro-5-[6-[4-[2-(1,3-dioxolan-2-yl)ethyl]-1-piperazinyl]hexyl-11H-dibenzo[b,e][1,4]diazepin-11-on handelt.

Beispiel 16

5,10-Dihydro-5-[6-(hexahydro-1H-1-azepinyl)hexyl]-11H-dibenzo[b,e][1,4]diazepin-11-on-hydrochlorid

Hergestellt analog Beispiel 6 aus 5,10-Dihydro-11H-dibenzo[b,e][1,4]diazepin-11-on, n-Butyllithium und 1-Brom-6-(hexahydro-1H-1-azepinyl)hexan in einer Ausbeute von 15 % der Theorie. Das in Wasser schwer lösliche Monohydrochlorid schmolz bei 103-105°C (aus Acetonitril unter Verwendung von Aktivkohle).

Beispiel 17

5,10-Dihydro-5-[6-(1-pyrrolidinyl)hexyl]-11H-dibenzo[b,e][1,4]diazepin-11-on-hydrochlorid

Hergestellt analog Beispiel 6 aus 5,10-Dihydro-11H-dibenzo[b,e][1,4]diazepin-11-on, n-Butyllithium und 1-Brom-6-(1-pyrrolidinyl)hexan in einer Ausbeute von 25 % der Theorie. Das wasserlösliche Monohydrochlorid schmolz bei 185-188°C (Acetonitril).

Beispiel 18

5,11-Dihydro-11-[6-(1-piperidinyl)hexyl]-6H-pyrido[2,3-b][1,4]benzodiazepin-6-on

24,1 g (0,08 Mol) 5,11-Dihydro-5-(phenylmethyl)-6H-pyrido[2,3-b][1,4]benzodiazepin-6-on (Fp. 152-154°C) wurden in 180 ml wasserfreiem Dimethylformamid gelöst, bei Zimmertemperatur mit 2,88 g (0,096 Mol) einer 80 proz. Natriumhydrid-Dispersion in Mineralöl versetzt und 45 Minuten bei 60°C gerührt. Danach wurden 23,8 g (0,096 Mol) 1-Brom-6-(1-piperidinyl)- hexan zugetropft und die Mischung 30 Minuten auf 120°C erhitzt. Nach dem Eindampfen im Vakuum wurde der Rückstand zwischen Dichlormethan und 5 proz. wäßriger Salzsäure verteilt, die wäßrige Phase abgetrennt und natronalkalisch gestellt. Das abgeschiedene Öl wurde in Dichlormethan aufgenommen, das Lösungsmittel im Wasserstrahlvakuum abdestilliert, der ölige, hochviskose Rückstand mit 250 g 100 proz. Orthophosphorsäure und 7,5 g Phenol sorgfältig verrührt und 3 Stunden auf 120°C erhitzt. Die auf 70°C abgekühlte Mischung wurde in 2 kg gestoßenes Eis eingerührt, anschließend durch Zugabe von Ätznatron deutlich natronalkalisch gestellt und mit Dichlormethan erschöpfend extrahiert. Die Dichlormethanextrakte wurden über Natriumsulfat getrocknet und eingedampft, der verbliebene Rückstand mit 10 ml siedendem Ether digeriert und heiß filtriert. Der Ether wurde abgedampft, der Rückstand an Kieselgel (Macherey-Nagel, 0,2-0,5 mm) unter Verwendung von Dichlormethan/Methanol/Cyclohexan/Essigsäureethylester/konz. Ammoniak 59/7,5/7,5/25/1 (v/v/v/v/v) zum Eluieren chromatographisch gereinigt. Durch Eindampfen der geeigneten Fraktionen und nachfolgendes Umkristallisieren aus Acetonitril erhielt man in einer Ausbeute von 21,8 g (72 % der Theorie) farblose Kristalle vom Fp. 131-132°C, nach Mischschmelzpunkt, Dünnschichtchromatogramm, IR-, UV-, $^1$H-NMR-Spektren völlig identisch mit einem nach Beispiel 6 hergestellten Präparat.

Beispiel 19

5,11-Dihydro-11-[7-(1-piperidinyl)heptyl]-6H-pyrido [2,3-b][1,4] benzodiazepin-6-on

Hergestellt analog Beispiel 18 durch Umsetzung von 5,11-Dihydro-5-(phenylmethyl)-6H-pyrido[2,3-b][1,4]benzodiazepin-6-on mit 1-Brom-7-(1-piperidinyl)-heptan in Gegenwart von Natriumhydrid und Dimethylformamid sowie anschließende acidolytische Abspaltung der (Phenylmethyl)-Gruppe durch Erhitzen mit 80 proz. Polyphosphorsäure in Gegenwart von Phenol. Ausbeute: 17 % der Theorie. Farblose Kristalle vom Fp. 118-120°C (nach Umkristallisieren aus Diisopropylether und Cyclohexan).

Beispiel 20

4,9-Dihydro-3-methyl-4-[6-(4-methyl-1-piperidinyl)-4-hexinyl]-10H- thieno[3,4-b][1,5] benzodiazepin-10-on

a.) 4,9-Diydro-3-methyl-4-(2-propinyl)-10H-thieno[3,4-b][1,5] benzodiazepin-10-on

Hergestellt analog Beispiel 1a) aus 4,9-Dihydro-3-methyl-10H-thieno[3,4-b][1,5]benzodiazepin-10-on und 3-Brom-1-propin in Gegenwart von Butyllithium und Tetrahydrofuran in einer Ausbeute von 50 % der Theorie. Das kristalline Rohprodukt wurde ohne weitere chromatographische Reinigung für nachfolgende Umsetzungen verwendet.

b. 4,9-Dihydro-3-methyl-4-[6-(4-methyl-1-piperazinyl)-4-hexinyl]-10-thieno[3,4-b][1,5] benzodiazepin-10-on

Hergestellt analog Beispiel 1b) aus 4,9-Dihydro-3-methyl-4-(2-propinyl)-10H-thieno[3,4-b][1,5]benzodiazepin-10-on, Paraformaldehyd und N-Methylpiperazin in Gegenwart von Kupfer(I)-chlorid in einer Ausbeute von 17 % der Theorie. Das farblose Dihydrochlorid schmolz bei 178°C (Z.).

Beispiel 21

4,9-Dihydro-4-[6-(hexahydro-1H-1-azepinyl)-4-hexinyl]-3-methyl-10H- thieno[3,4-b][1,5]benzodiazepin-10-on

Hergestellt analog Beispiel 1b) aus 4,9-Dihydro-3-methyl-4-(2-propinyl)-10H-thieno[3,4-b][1,5]benzodiazepin-10-on, Paraformaldehyd und Hexamethylenimin in Gegenwart von Kupfer(I)-chlorid in einer Ausbeute von 3% der Theorie. Das farblose Monohydrochlorid schmolz bei 169-172°C.

Beispiel 22

4,9-Dihydro-3-methyl-4-[6-[4-(phenylmethyl)-1-piperazinyl]-4-hexinyl]-10H-thieno[3,4-b][1,5]benzodiazepin-10-on

Hergestellt analog Beispiel 1b) aus 4,9-Dihydro-3-methyl4-(2-propinyl)-10H-thieno[3,4-b][1,5]benzodiazepin-10-on, Paraformaldehyd und N-(Phenylmethyl)-piperazin in Gegenwart von Kuper(I)-chlorid in einer Ausbeute von 12 % der Theorie. $R_F$ 0,5 (Merck, DC-Fertigplatten, Kieselgel 60F-254; Fließmittel: Dichlormethan/Ethanol 9/1 v/v).

Beispiel 23

4,9-Dihydro-3-methyl-4-[6-[4-(2-phenylethyl-1-piperazinyl]-4-hexinyl]-10H-thieno[3,4-b][1,5]benzodiazepin-10-on

Hergestellt analog Beispiel 1b) aus 4,9-Dihydro-3-methyl-4-(2-propinyl)-10H-thieno[3,4-b][1,5]benzodiazepin-10-on, Paraformaldehyd und N-(2-phenylethyl)-piperazin in Gegenwart katalytischer Mengen an Kupfer(I)-chlorid in einer Ausbeute von 17 % der Theorie. Das farblose Monohydrochlorid schmolz bei 223-225°C unter Zersetzung; $R_F$ 0,8 (Merck, DC-Fertigplatten, Kieselgel 60 F-254; Fließmittel; Essigsäureethylester/Methanol/Cyclohexan/konz. Ammoniak 8/1/1/0,1 v/v/v/v). Im folgenden wird die Herstellung pharmazeutischen Anwendungsformen anhand einiger Beispiele beschrieben:

Beispiel I

Tabletten mit 5 mg 5,11-Dihydro-11-[6-(1-piperidinyl)hexyl]-6H-pyrido[2,3-b][1,4]benzodiazepin-6-on

Zusammensetzung:
1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.
Tablettengewicht: 220 mg
Stempel: 9 mm

Beispiel II

Dragées mit 1,5 mg 5,11-Dihydro-11-[6-(1-piperidinyl)hexyl]-6H-pyrido[2,3-b][1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 11-[4-[2-[(Diethylamino)methyl]-1-piperidinyl]butyl]-5,11-dihydro-6H-pyrido[2,3-b][1,4]benzodiazepin-6-on,

Zusammensetzung:
1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren :

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120°C.

Beispiel IV

Suppositorien mit 20 mg 5,11-Dihydro-11-[6-(1-piperidinyl)-hexyl-6H-pyrido[2,3-b][1,4]benzodiazepin-6-on

Zusammensetzung:
1 Zäpfchen enthält:

| | |
|---|---|
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht 1,7 g

Beispiel V

Tropfen mit 5,11-Dihydro-11-[6-(1-piperidinyl)hexyl]-6H-pyrido[2,3-b][1,4]benzodiazepin-6-on

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Patentansprüche

### Patentanprüche für folgende Vertragsstaaten : AT, BE, CH, DE, FR, GB, IT, LI, LU, NL, SE

1. Kondensierte Diazepinone der allgemeinen Formel I,

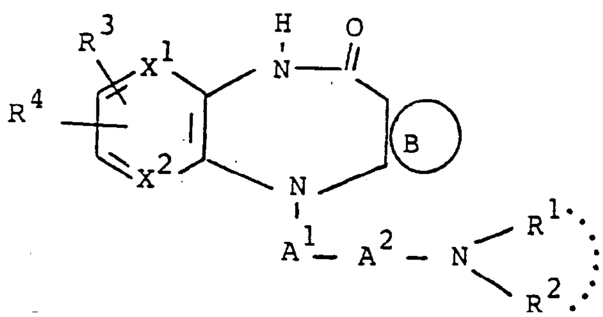

(I)

in der ⟩Ⓑ einen der zweiwertigen Reste

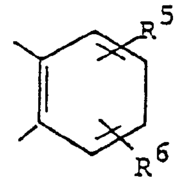 , 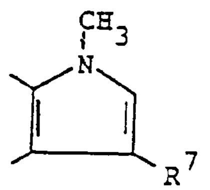 , 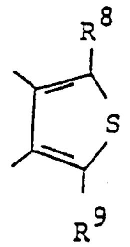 , 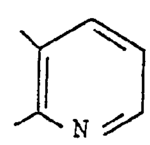 oder 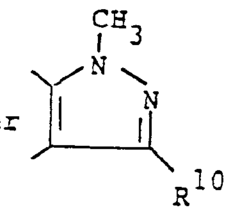

(S) (T) (U) (V) (W)

darstellt und
$X^1$, $X^2$, $A^1$, $A^2$ und $R^1$ bis $R^{10}$ die folgenden Bedeutungen besitzen:

$X^1$ und $X^2$ stellen eine =CH-Gruppe dar oder, sofern Ⓑ die Bedeutungen der oben genannten, zweiwertigen Reste (S), (U) oder (W) annimmt, können beide oder nur $X^1$ oder nur $X^2$ auch ein Stickstoffatom darstellen;
$A^1$ ist eine gegebenenfalls durch ein oder zwei Methylgruppen substituierte Methylengruppe;
$A^2$ eine geradkettige, gegebenenfalls eine Doppel- oder Dreifachbindung enthaltende und drei bis sieben Kohlenstoffatome umfassende Alkylenkette, die zusätzlich methylsubstituiert sein kann;
$R^1$ ist ein verzweigter oder unverzweigter Alkylrest mit 1 bis 4 Kohlenstoffatomen;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder eine Stickstoffunktion unterbrochen und/oder durch einen gegebenenfalls durch eine Phenyl-, Di-($C_1$-$C_3$)alkylamino- oder (Cycloalkyl)alkylaminogruppe substituierten Alkylrest mit 1 bis 6 Kohlenstoffatomen oder durch einen gegebenenfalls ein oder zwei Ether-Sauerstoffatome enthaltenden (Cycloalkyl)alkyl-Rest substituiert sein kann, wobei die zuvor genannten Cycloalkylgruppen 4 bis 7 Kohlenstoffatome und die an diese gebundenen Alkylengruppen 1 bis 3 Kohlenstoffatome enthalten können;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und

$R^{10}$ ein Wasserstoffatom oder eine Methylgruppe, wobei für den Fall, daß Ⓑ den zweiwertigen Rest T darstellt und $R^7$ ein Wasserstoffatom ist, $R^3$ kein Chloratom bedeuten und $A^2$ keine Doppel- oder Dreifachbindung enthalten darf, und ihre diastereomeren und enantiomeren Formen, ihre physiologisch verträglichen Salze mit anorganischen oder organischen Säuren.

2. Kondensierte Diazepinone gemäß Anspruch 1, wobei in der allgemeinen Formel I
$X^1$ eine =CH-Gruppe,

$X^2$ entweder ein Stickstoffatom und Ⓑ den zweiwertigen Rest (S) darstellt, mit der Maßgabe, daß $R^3$, $R^4$ und $R^5$ Wasserstoffatome sind und $R^6$ ein Wasserstoffatom, ein Chlor- oder Bromatom oder eine Methyl- oder Ethylgruppe in 8- oder 9-Stellung des Heterocyclus ist,

oder eine =CH-Gruppe darstellt, wenn Ⓑ die Bedeutung der zweiwertigen Reste U oder V annimmt, wobei $R^8$ ein Wasserstoffatom und $R^9$ eine Methylgruppe ist;
$A^1$ die Methylengruppe;
$A^2$ eine geradkettige, gegebenenfalls eine Doppel- oder Dreifachbindung enthaltende und drei bis sechs Kohlenstoffatome umfassende Alkylenkette;
$R^1$ einen verzweigten oder unverzweigten Alkylrest mit 1 bis 4 Kohlenstoffatomen;
$R^2$ einen verzweigten oder unverzweigten Alkylrest mit 1 bis 7 Kohlenstoffatomen, einen Cycloalkyl- oder (Cycloalkyl)methyl-Rest mit 3 bis 7 Kohlenstoffatomen im Ring, wobei der Cycloalkylring gegebenenfalls noch durch eine Hydroxygruppe substituiert sein kann, bedeuten
oder
$R^1$ und $R^2$ zusammen mit dem dazwischenliegenden Stickstoffatom einen 5- bis 7-gliedrigen gesättigten, monocyclischen, heterocyclischen Ring bilden, der gegebenenfalls durch ein Sauerstoffatom oder ein Stickstoffatom unterbrochen und/oder durch einen gegebenenfalls durch eine Phenyl-, Di-($C_1$-$C_3$)alkylamino- oder (Cycloalkyl)alkylaminogruppe substituierten Alkylrest mit 1 bis 3 Kohlenstoffatomen oder durch einen gegebenenfalls ein oder zwei Ether-Sauerstoffatome enthaltenden (Cycloalkyl)alkyl-Rest substituiert sein kann, wobei die zuvor genannten Cycloalkylgruppen 4 bis 7 Kohlenstoffatome und die an diese gebundenen Alkylengruppen 1 bis 3 Kohlenstoffatome enthalten können; und ihre diastereomeren und enantiomeren Formen, ihre physiologisch verträglichen Salze mit anorganischen oder organischen Säuren.

3. Kondensierte Diazepinone gemäß Anspruch 1, wobei in der allgemeinen Formel I
$X^1$ eine =CH-Gruppe,
$X^2$ ein Stickstoffatom und B den zweiwertigen Rest (S) darstellt, mit der Maßgabe, daß $R^3$, $R^4$ und $R^5$ Wasserstoffatome sind und $R^6$ ein Wasserstoff-, Chlor- oder Bromatom oder eine Methyl- oder Ethylgruppe in 8- oder 9-Stellung des Heterocyclus ist,
$A^1$ die Methylengruppe,
$A^2$ eine geradkettige, gegebenenfalls eine Doppel- oder Dreifachbindung enthaltende und drei bis sechs Kohlenstoffatome umfassende Alkylenkette und
$R^1$ und $R^2$ zusammen mit dem dazwischenliegenden Stickstoffatom den Dimethylamino-, Diethylamino-, Dipropylamino-, [Bis(methylethyl)]amino-; 1-Pyrrolidinyl-, 1-Piperidinyl-, Hexahydro-1H-1-azepinyl-, 4-Morpholinyl-, 2-[(Diethylamino)methyl]-1-piperidinyl-, trans-(4-Hydroxycyclohexyl)methylamino-, (Cyclohexyl)methylamino-, 2-[[(Cyclohexyl)methylamino]methyl]-1-piperidinyl-, 4-Methyl-1-piperazinyl-, 4-[2-(1,3-Dioxolan-2-yl)ethyl]-1-piperazinyl-, 4-(Phenylmethyl)-1-piperazinyl- oder 4-(2-Phenylethyl)-1-piperazinyl-Rest bedeuten und ihre diastereomeren und enantiomeren Formen, ihre physiologisch verträglichen Salze mit anorganischen oder organischen Säuren.

4. Verbindungen gemäß Anspruch 1:
5,11-Dihydro-11-[6-(1-piperidinyl)hexyl]-6H-pyrido[2,3-b][1,4]benzodiazepin-6-on
11-[4-[2-(Diethylamino)methyl]-1-piperidinyl]butyl]-5,11-dihydro-6H-pyrido[2,3-b][1,4]benzodiazepin-6-on
11-[4-[2-[(Diethylamino)methyl]-1-piperidinyl]-2-butinyl]-5,11-dihydro-6H-pyrido[2,3-b][1,4]benzodiazepin-6-on,
und ihre diastereomeren und enantiomeren Formen, ihre physiologisch verträglichen Salze mit anorganischen oder organischen Säuren.

5. Arzneimittel, enthaltend eine Verbindung nach mindestens einem der Ansprüche 1 bis 4 oder ein physiologisch verträgliches Säureadditionssalz neben gegebenenfalls einem oder mehreren inerten Trägerstoffen und/oder Verdünnungsmitteln.

6. Verwendung der Verbindungen nach mindestens einem der Ansprüche 1 bis 4 zur Herstellung eines Arzneimittels, das als vagaler Schrittmacher zur Behandlung von Bradycardien und Bradyarrhythmien geeignet ist.

7. Verwendung der Verbindungen nach mindestens einem der Ansprüche 1 bis 4 zur Herstellung eines Arzneimittels mit spasmolytischen Eigenschaften auf periphere Organe, mit antiemetischen Eigenschaften, mit die cerebrale Durchblutung fördernden Eigenschaften und zur Therapie von Erkrankungen des Zentralnervensystems, insbesondere des Morbus Alzheimer und des Morbus Parkinson.

8. Verfahren zur Herstellung kondensierter Diazepinone der allgemeinen Formel I gemäß Anspruch 1, dadurch gekennzeichnet, daß

a.) zur Herstellung von Verbindungen der allgemeinen Formel I, in der $R^7$ ein Chloratom oder eine Methylgruppe bedeutet und die übrigen Reste die oben angegebenen Bedeutungen besitzen, ein Tricyclus der allgemeinen Formel II,

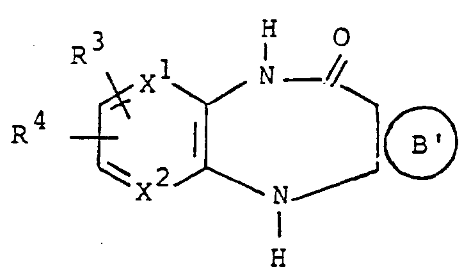

(

in der

die Reste $R^3$, $R^4$, $X^1$ und $X^2$ wie vorstehend erwähnt definiert sind und 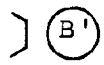 einen der zweiwertigen Reste S, U, V, W oder T′

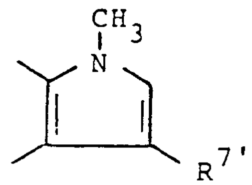

(T')

darstellt, worin $R^{7'}$ ein Chloratom oder eine Methylgruppe bedeutet, in sein Dilithiumsalz überführt und dieses anschließend mit einer Verbindung der allgemeinen Formel IV,

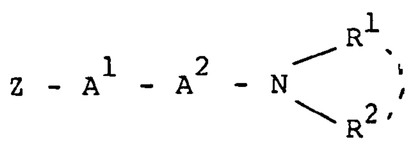

(IV)

in der
$A^1$, $A^2$, $R^1$ und $R^2$ die oben angegebenen Bedeutungen haben und Z ein Chlor-, Brom- oder Iodatom, die Methansulfonyloxy-, Ethansulfonyloxy- oder eine Arensulfonyloxygruppe darstellt, alkyliert wird, wobei die Erzeugung des Dilithiumsalzes und die Alkylierung, die auch im gleichen Reaktionsmedium erfolgen können, in einem organischen Lösungsmittel bei Temperaturen zwischen -80 und +70°C durchgeführt werden, oder

b.) zur Herstellung von Verbindungen der allgemeinen Formel I, in der $A^2$ eine geradkettige, drei bis sieben Kohlenstoffatome umfassende gesättigte Alkylenkette, die gegebenenfalls noch methylsubstituiert sein kann und $R^7$ ein Chloratom oder eine Methylgruppe darstellen, ein (Arylmethyl)tricyclus der allgemeinen Formel V,

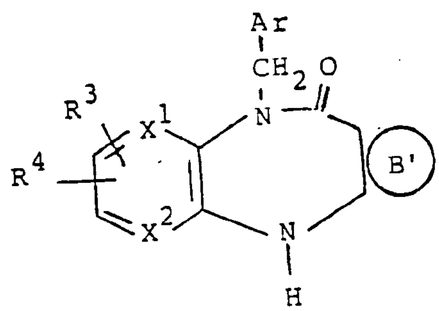

(V)

in der

die Reste 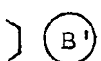, $R^3$, $R^4$, $X^1$ und $X^2$ wie oben definiert sind und Ar einen gegebenenfalls durch jeweils ein bis zwei Methyl- und/oder Methoxygruppen substituierten Phenylrest darstellt, in sein Metallsalz der allgemeinen Formel Va,

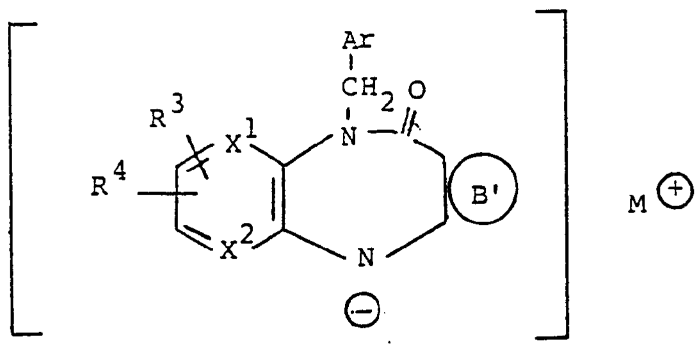

(Va)

überführt, worin M ein Alkalimetallatom ist oder den Rest MgHal darstellt, worin Hal Chlor, Brom oder Iod ist, und dieses anschließend mit einer Verbindung der allgemeinen Formel IV,

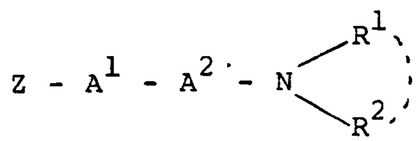

(IV)

in der
$A^1$, $R^1$, $R^2$ und Z die oben angegebenen Bedeutungen haben und $A^2$ eine geradkettige, drei bis sieben Kohlenstoffatome umfaßende gesättigte Alkylenkette, die gegebenenfalls noch methylsubstituiert sein kann, darstellt, alkyliert wird und aus dieser dabei entstehenden Verbindung abschließend die Arylmethylgruppe acidolytisch entfernt wird, wobei die Metallierung in einem inerten organischen Lösungsmittel bei Temperaturen zwischen -100°C und dem Siedepunkt des Reaktionsgemisches mit Lithiumarylen, Lithiumalkylen, Lithiumdialkylamiden oder Grignard-Reagenzien, Alkalimetallhydriden, Alkalimetallamiden, Alkalimetallhydroxiden erfolgt, die Alkylierung in einem organischen Lösungsmittel bei Temperaturen zwischen -80 und +70°C und die acidolytische Abspaltung der Arylmethylgruppe mit starken Säuren oder Lewis-Säuren bei Temperaturen zwischen -20 und +150°C durchgeführt wird oder

c.) zur Herstellung von Verbindungen der allgemeinen Formel I, in der $A^2$ eine geradkettige, drei bis sieben Kohlenstoffatome umfassende gesättigte Alkylenkette, die gegebenenfalls noch methylsubstituiert sein kann, $R^3$ ein Wasserstoffatom oder eine Alkylgruppe mit 1 bis 4 Kohlenstoffatomen und 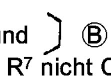 einen der zeiwertigen Reste S, T, U oder W darstellen mit der Einschränkung, daß $R^5$, $R^6$ und $R^7$ nicht Chlor oder Brom sein können, eine Verbindung der allgemeinen Formel VIa,

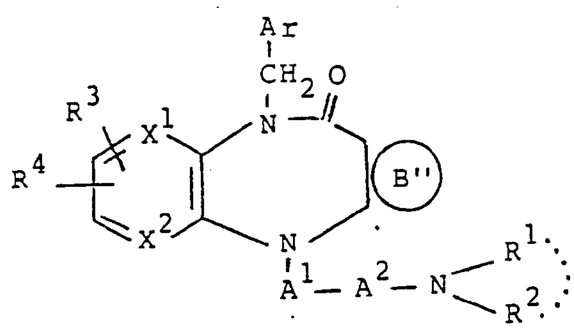

(VIa)

in der

die Reste $A^1$, $A^2$, $R^1$, $R^2$, $R^3$, $R^4$, $X^1$, $X^2$ und Ar die angegebenen Bedeutungen haben und 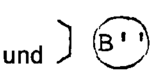 einen der zweiwertigen Reste S, T, U oder W mit der Maßgabe, daß $R^7$ kein Wasserstoffatom ist, bedeutet, in Gegenwart von Katalysatoren aus Metallen aus der VIII. Nebengruppe des Periodensystems der Elemente,

bei Wasserstoffdrucken von 1 bis 300 bar und Temperaturen von 0°C bis 130°C und in Gegenwart von Lösungsmitteln und, gegebenenfalls, von Mineralsäuren hydrogenolysiert wird, oder

d.) zur Herstellung von Verbindungen der allgemeinen Formel I, in der $A^2$ eine geradkettige, gegebenenfalls eine Doppelbindung enthaltende und drei bis sieben Kohlenstoffatome umfassende Alkylkette, die zusätzlich methylsubstituiert sein kann, $R^3$ ein Wasserstoffatom oder eine Alkylgruppe mit 1 bis 4 Kohlenstoffatomen und )Ⓑ einen der zweiwertigen Reste S, T, U oder W darstellen, mit der Einschränkung, daß $R^5$, $R^6$ und $R^7$ keine Chlor- oder Bromatome bedeuten, eine Verbindung der allgemeinem Formel VII,

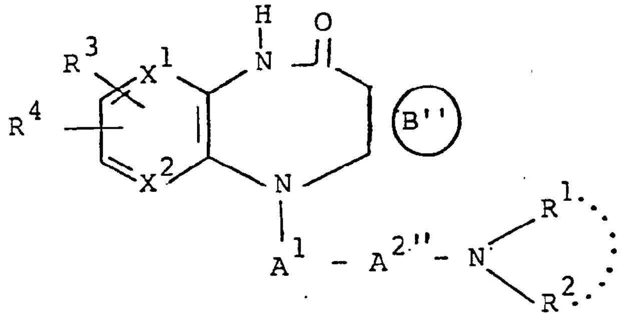

(VII)

in der

die Reste $A^1$, $R^1$ bis $R^4$, $X^1$, $X^2$ und 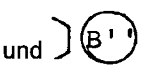 die oben angegebenen Bedeutungen haben und $A^{2''}$ eine geradkettige, eine Dreifachbindung enthaltende und drei bis sieben Kohlenstoffatome umfassende Alkylenkette ist, die auch methylsubstituiert sein kann,

in Gegenwart von Metallen der VIII. Nebengruppe des Periodensystems der Elemente bei Wasserstoffdrucken von 0,1 bis 300 bar und Temperaturen von 0 bis 130°C und in Gegenwart von Lösungsmitteln katalytisch hydriert wird, wobei zur Herstellung von Verbindungen der allgemeinen Formel I, in der $A^2$ eine eine Doppelbindung enthaltende Alkylengruppe darstellt, die katalytische Hydrierung entweder nach Aufnahme von 1 Mol Wasserstoff unterbrochen oder sie in Gegenwart eines desaktivierenden Katalysators durchgeführt wird, oder

e.) zur Herstellung von Verbindungen der allgemeinen Formel I, in der $A^2$ eine geradkettige, eine Dreifachbindung enthaltende und drei bis sieben Kohlenstoffatome umfassende Alkylenkette ist, die auch methylsubstituiert sein kann und )Ⓑ den oben definierten zweiwertigen Rest 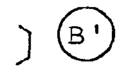 bedeutet, ein kondensierter Tricyclus der allgemeinen Formel VIII,

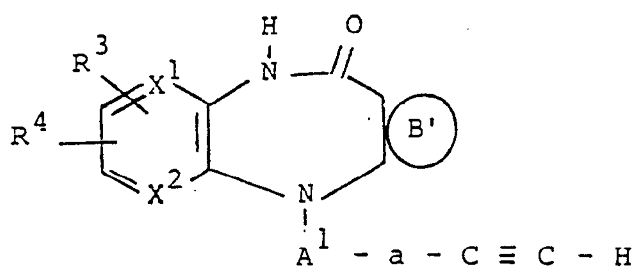

(VIII)

in der

$A^1$, 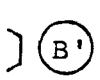, $R^3$, $R^4$, $X^1$ und $X^2$ die oben angegebenen Bedeutungen besitzen und "a" eine Einfachbindung oder eine geradkettige, gesättigte, gegebenenfalls durch eine Methylgruppe substituierte Alkylenkette mit 1 bis 4 Kohlenstoffatomen darstellt, mit einer Verbindung der allgemeinen Formel IX

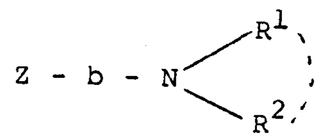

(IX)

in der

$R^1$, $R^2$ und Z wie oben angegeben definiert sind und "b" eine geradkettige, gesättigte, gegebenenfalls durch eine Methylgruppe substituierte Alkylengruppe mit ein bis vier Kohlenstoffatomen bedeutet, gegebenenfalls in Gegenwart von Kupfersalzen, in einem polaren Lösungsmittel bei Temperaturen zwischen 20°C und dem Siedepunkt des Reaktionsgemisches, gegebenenfalls in Gegenwart eines schwach alkalischen Mediums, aminoalkyliert wird

und gegebenenfalls anschließend eine so erhaltene Verbindung der allgemeinen Formel I in ihre Isomeren aufgetrennt wird und/oder eine so erhaltene Verbindung der allgemeinen Formel I in ihre Säureadditionssalze, vorzugsweise in ihre physiologisch verträglichen Säureadditionssalze, mit anorganischen oder organischen Säuren übergeführt wird.

9. Verfahren gemäß Anspruch 8e, dadurch gekennzeichnet, daß zur Einführung eines (Dialkylamino)methyl- bzw. 1-(Dialkylamino)ethylrestes in eine Verbindung der allgemeinen Formel VIII, diese Verbindung in einem polaren Lösungsmittel mit Formaldehyd bzw. Paraformaldehyd oder, zur Einführung des zweitgenannten Restes, mit Acetaldehyd bzw. Paraldehyd bzw. Metaldehyd und mit einem sekundären Amin der allgemeinen Formel X

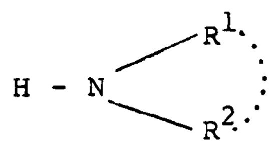

(X)

in der

$R^1$ und $R^2$ wie eingangs angegeben definiert sind, gegebenenfalls in Gegenwart von Kupfersalzen, bei Temperaturen zwischen 20°C und dem Siedepunkt des Reaktionsgemisches, gegebenenfalls in Gegenwart eines schwach alkalischen Reaktionsgemisches, umgesetzt wird.

10. Verfahren gemäß Anspruch 8e, dadurch gekennzeichnet, daß

a.) die Verbindung der allgemeinen Formel VIII mit einem Aminal der allgemeinen Formel IXb

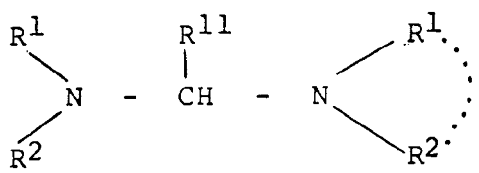

(IXb)

in der

$R^1$ und $R^2$ wie oben definiert sind und $R^{11}$ ein Wasserstoffatom oder eine Methylgruppe bedeutet, oder

b.) die Verbindung der allgemeinen Formel VIII oder die davon abgeleitete Grignard-Verbindung der allgemeinen Formel VIIIa,

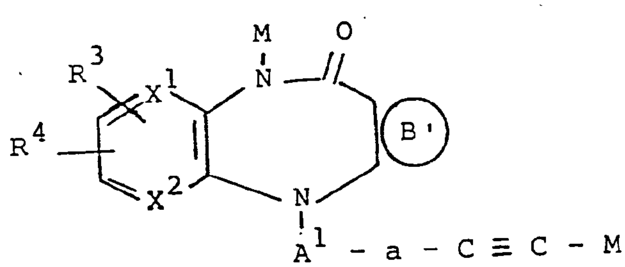

(VIIIa)

worin a, $A^1$, 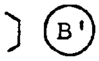 , $R^3$, $R^4$, $X^1$ und $X^2$ wie angegeben definiert sind und M die MgHalgruppe (Hal=Halogenatom) ist, mit einem Alkoxyamin der allgemeinen Formel IXc,

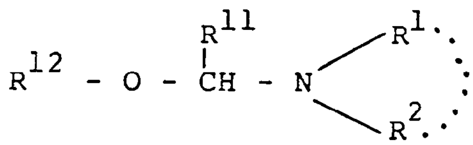

(IXc)

in der
$R^1$, $R^2$ und $R^{11}$ wie oben definiert sind und
$R^{12}$ einen Alkylrest mit 1 bis 10 Kohlenstoffatomen darstellt, in einem Lösungsmittel umgesetzt wird oder

c.) ein Metallsalz der obigen allgemeinen Formel VIIIa, in der M ein Alkalimetallatom oder den Rest MgHal (Hal=Halogen) bedeutet, mit einer Verbindung der allgemeinen Formel IX, in der b eine gegebenenfalls methylsubstituierte 1,2-Ethylen-, 1,3-Propylen- oder 1,4-Butylengruppe bedeutet, in einem organischen Lösungsmittel bei Temperaturen zwischen -80°C und +150°C, gegebenenfalls in Gegenwart eines Kupfersalzes, umgesetzt wird und, gegebenenfalls, das erhaltene Isomerengemisch anschließend aufgetrennt und/oder die erhaltenen Verbindungen in ihre Säureadditionssalze mit anorganischen oder organischen Säuren übergeführt werden.

**Patentansprüche für folgende Vertragsstaaten: ES, GR.**

1. Verfahren zur Herstellung neuer kondensierter Diazepinone der allgemeinen Formel I

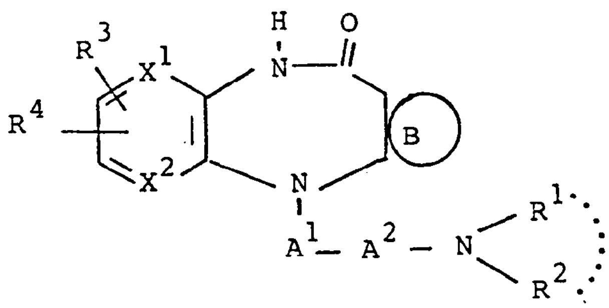

(I)

in der 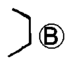 einen der zweiwertigen Reste

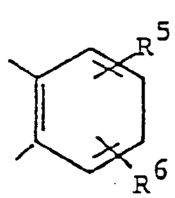 , 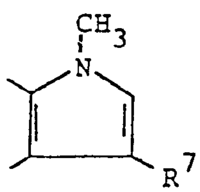 , 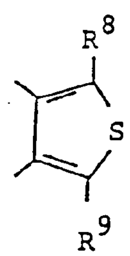 , 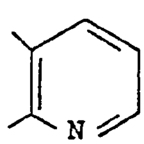 oder 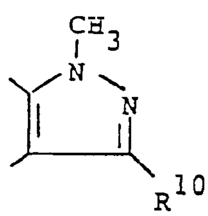

(S) (T) (U) (V) (W)

darstellt und
$X^1$, $X^2$, $A^1$, $A^2$ und $R^1$ bis $R^{10}$ die folgenden Bedeutungen besitzen:

$X^1$ und $X^2$ stellen eine =CH-Gruppe dar oder, sofern 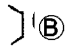 die Bedeutungen der oben genannten, zweiwertigen Reste (S), (U) oder (W) annimmt, können beide oder nur $X^1$ oder nur $X^2$ auch ein Stickstoffatom darstellen;
$A^1$ ist eine gegebenenfalls durch ein oder zwei Methylgruppen substituierte Methylengruppe;
$A^2$ eine geradkettige, gegebenenfalls eine Doppel- oder Dreifachbindung enthaltende und drei bis sieben Kohlenstoffatome umfassende Alkylenkette, die zusätzlich methylsubstituiert sein kann;
$R^1$ ist ein verzweigter oder unverzweigter Alkylrest mit 1 bis 4 Kohlenstoffatomen;
$R^2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann;
$R^1$ und $R^2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder eine Stickstoffunktion unterbrochen und/oder durch einen gegebenenfalls durch eine Phenyl-, Di-($C_1$-$C_3$)alkylamino- oder (Cycloalkyl)alkylaminogruppe substituierten Alkylrest mit 1 bis 6 Kohlenstoffatomen oder durch einen gegebenenfalls ein oder zwei Ether-Sauerstoffatome enthaltenden (Cycloalkyl)alkyl-Rest substituiert sein kann, wobei die zuvor genannten Cycloalkylgruppen 4 bis 7 Kohlenstoffatome und die an diese gebundenen Alkylengruppen 1 bis 3 Kohlenstoffatome enthalten können;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Wasserstoffatom, ein Fluor-, Chlor- oder Bromatom oder eine Alkylgruppe mit 1 bis 4 C-Atomen;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und

$R^{10}$ ein Wasserstoffatom oder eine Methylgruppe, wobei für den Fall, daß 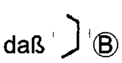 den zweiwertigen Rest (T) darstellt und $R^7$ ein Wasserstoffatom ist, $R^3$ kein Chloratom bedeuten und $A^2$ keine Doppel- oder Dreifachbindung enthalten darf, und ihrer diastereomeren und enantiomeren Formen, desweiteren ihrer Salze mit anorganischen oder organischen Säuren, dadurch gekennzeichnet, daß

a.) zur Herstellung von Verbindungen der allgemeinen Formel I, in der $R^7$ ein Chloratom oder eine Methylgruppe bedeutet und die übrigen Reste die oben angegebenen Bedeutungen besitzen, ein Tricyclus der allgemeinen Formel II,

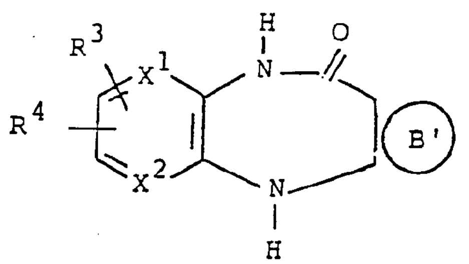

(II)

in der

die Reste $R^3$, $R^4$, $X^1$ und $X^2$ wie vorstehend erwähnt definiert sind und 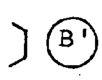 einen der zweiwertigen Reste S, U, V, W oder T'

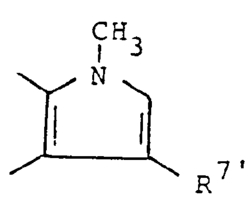

(T')

darstellt, worin $R^{7'}$ ein Chloratom oder eine Methylgruppe bedeutet, in sein Dilithiumsalz überführt und dieses anschließend mit einer Verbindung der allgemeinen Formel IV,

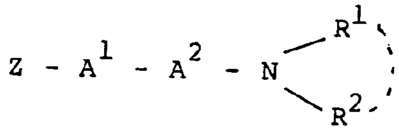

(IV)

in der

$A^1$, $A^2$, $R^1$ und $R^2$ die oben angegebenen Bedeutungen haben und Z ein Chlor-, Brom- oder Iodatom, die Methansulfonyloxy-, Ethansulfonyloxy- oder eine Arensulfonyloxygruppe darstellt, alkyliert wird, wobei die Erzeugung des Dilithiumsalzes und die Alkylierung, die auch im gleichen Reaktionsmedium erfolgen können, in einem organischen Lösungsmittel bei Temperaturen zwischen -80 und +70°C durchgeführt werden, oder

b.) zur Herstellung von Verbindungen der allgemeinen Formel I, in der $A^2$ eine geradkettige, drei bis sieben Kohlenstoffatome umfassende gesättigte Alkylenkette, die gegebenenfalls noch methylsubstituiert sein kann und $R^7$ ein Chloratom oder eine Methylgruppe darstellen, ein (Arylmethyl)tricyclus der allgemeinen Formel V,

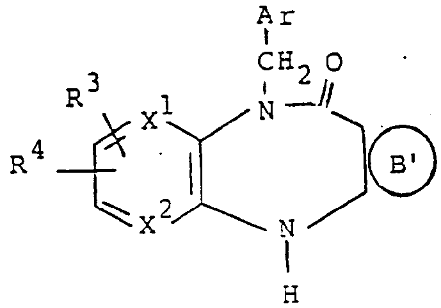

(V)

in der

die Reste 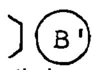 , $R^3$, $R^4$, $X^1$ und $X^2$ wie oben definiert sind und Ar einen gegebenenfalls durch jeweils ein bis zwei Methyl- und/oder Methoxygruppen substituierten Phenylrest darstellt, in sein Metallsalz der allgemeinen Formel Va,

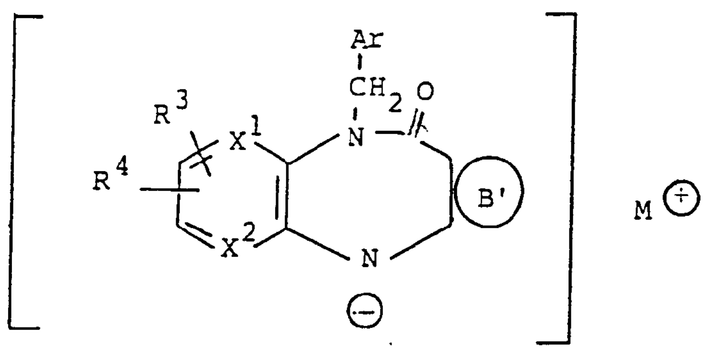

(Va)

überführt, worin M ein Alkalimetallatom ist oder den Rest MgHal darstellt, worin Hal Chlor, Brom oder Iod ist, und dieses anschließend mit einer Verbindung der allgemeinen Formel IV,

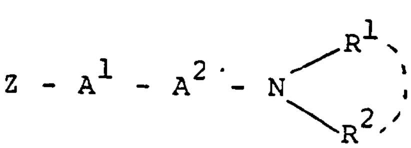

(IV)

in der

$A^1$, $R^1$, $R^2$ und Z die oben angegebenen Bedeutungen haben und $A^2$ eine geradkettige, drei bis sieben Kohlenstoffatome umfaßende gesättigte Alkylenkette, die gegebenenfalls noch methylsubstituiert sein kann, darstellt, alkyliert wird und aus dieser dabei entstehenden Verbindung abschließend die Arylmethylgruppe acidolytisch entfernt wird, wobei die Metallierung in einem inerten organischen Lösungsmittel bei Temperaturen zwischen -100°C und dem Siedepunkt des Reaktionsgemisches mit Lithiumarylen, Lithiumalkylen, Lithiumdialkylamiden oder Grignard-Reagenzien, Alkalimetallhydriden, Alkalimetallamiden, Alkalimetallhydroxiden erfolgt, die Alkylierung in einem organischen Lösungsmittel bei Temperaturen zwischen -80 und +70°C und die acidolytische Abspaltung der Arylmethylgruppe mit starken Säuren oder Lewis-Säuren bei Temperaturen zwischen -20 und +150°C durchgeführt wird oder

c.) zur Herstellung von Verbindungen der allgemeinen Formel I, in der $A^2$ eine geradkettige, drei bis sieben Kohlenstoffatome umfassende gesättigte Alkylenkette, die gegebenenfalls noch methylsubstituiert sein kann, $R^3$ ein Wasserstoffatom oder eine Alkylgruppe mit 1 bis 4 Kohlenstoffatomen und ⟩ Ⓑ einen der zeiwertigen Reste S, T, U oder W darstellen mit der Einschränkung, daß $R^5$, $R^6$ und $R^7$ nicht Chlor oder Brom sein können, eine Verbindung der allgemeinen Formel VIa,

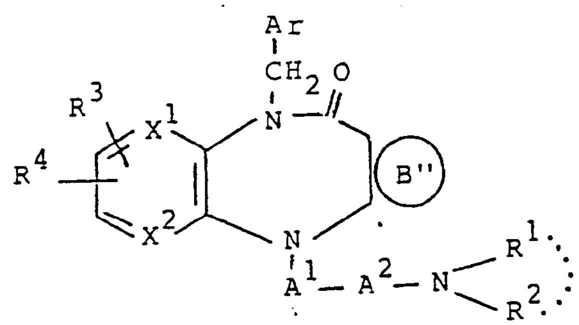

(VIa)

in der

die Reste $A^1$, $A^2$, $R^1$, $R^2$, $R^3$, $R^4$, $X^1$, $X^2$ und Ar die angegebenen Bedeutungen haben und ⟩ (B'') einen der zweiwertigen Reste S, T, U oder W mit der Maßgabe, daß $R^7$ kein Wasserstoffatom ist, bedeutet, in Gegenwart von Katalysatoren aus Metallen aus der VIII. Nebengruppe des Periodensystems der Elemente, bei Wasserstoffdrucken von 1 bis 300 bar und Temperaturen von 0°C bis 130°C und in Gegenwart von Lösungsmitteln und, gegebenenfalls, von Mineralsäuren hydrogenolysiert wird, oder

d.) zur Herstellung von Verbindungen der allgemeinen Formel I, in der $A^2$ eine geradkettige, gegebenenfalls eine Doppelbindung enthaltende und drei bis sieben Kohlenstoffatome umfassende Alkylkette, die zusätzlich methylsubstituiert sein kann, $R^3$ ein Wasserstoffatom oder eine Alkylgruppe mit 1 bis 4 Kohlenstoffatomen und ⟩ Ⓑ einen der zweiwertigen Reste S, T, U oder W darstellen, mit der Einschränkung, daß $R^5$, $R^6$ und $R^7$ keine Chlor- oder Bromatome bedeuten, eine Verbindung der allgemeinen Formel VII,

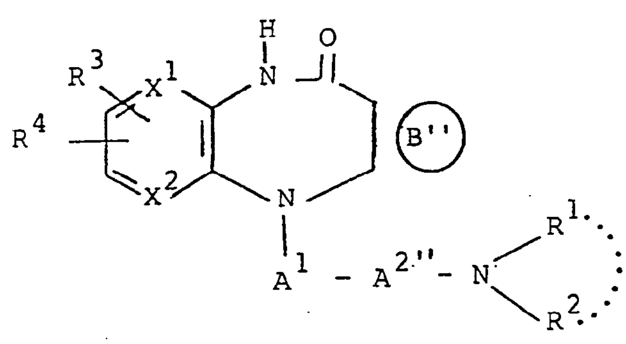

(VII)

in der

die Reste $A^1$, $R^1$ bis $R^4$, $X^1$, $X^2$ und 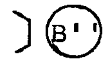 die oben angegebenen Bedeutungen haben und $A^{2''}$ eine geradkettige, eine Dreifachbindung enthaltende und drei bis sieben Kohlenstoffatome umfassende Alkylenkette ist, die auch methylsubstituiert sein kann,

in Gegenwart von Metallen der VIII. Nebengruppe des Periodensystems der Elemente bei Wasserstoffdrucken von 0,1 bis 300 bar und Temperaturen von 0 bis 130°C und in Gegenwart von Lösungsmitteln katalytisch hydriert wird, wobei zur Herstellung von Verbindungen der allgemeinen Formel I, in der $A^2$ eine eine Doppelbindung enthaltende Alkylengruppe darstellt, die katalytische Hydrierung entweder nach Aufnahme von 1 Mol Wasserstoff unterbrochen oder sie in Gegenwart eines desaktivierenden Katalysators durchgeführt wird, oder

e.) zur Herstellung von Verbindungen der allgemeinen Formel I, in der $A^2$ eine geradkettige, eine Dreifachbindung enthaltende und drei bis sieben Kohlenstoffatome umfassende Alkylenkette ist, die auch methylsubstituiert sein kann und 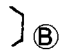 den oben definierten zweiwertigen Rest 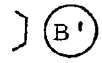 bedeutet, ein kondensierter Tricyclus der allgemeinen Formel VIII,

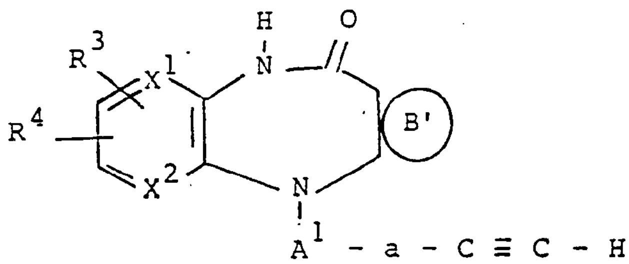

(VIII)

in der

$A^1$, 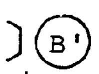, $R^3$, $R^4$, $X^1$ und $X^2$ die oben angegebenen Bedeutungen besitzen und "a" eine Einfachbindung oder eine geradkettige, gesättigte, gegebenenfalls durch eine Methylgruppe substituierte Alkylenkette mit 1 bis 4 Kohlenstoffatomen darstellt, mit einer Verbindung der allgemeinen Formel IX

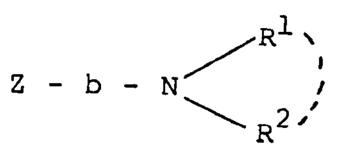

(IX)

in der

$R^1$, $R^2$ und Z wie oben angegeben definiert sind und "b" eine geradkettige, gesättigte, gegebenenfalls durch eine Methylgruppe substituierte Alkylengruppe mit ein bis vier Kohlenstoffatomen bedeutet, gegebenenfalls in Gegenwart von Kupfersalzen, in einem polaren Lösungsmittel bei Temperaturen zwischen 20°C und dem Siedepunkt des Reaktionsgemisches, gegebenenfalls in Gegenwart eines schwach alkalischen Mediums, aminoalkyliert wird

und gegebenenfalls anschließend eine so erhaltene Verbindung der allgemeinen Formel I in ihre Isomeren aufgetrennt wird und/oder eine so erhaltene Verbindung der allgemeinen Formel I in ihre Säureadditionssalze, vorzugsweise in ihre physiologisch verträglichen Säureadditionssalze, mit anorganischen oder organischen Säuren übergeführt wird.

2. Verfahren gemäß Anspruch 1e, dadurch gekennzeichnet, daß zur Einführung eines (Dialkylamino)methyl- bzw. 1-(Dialkylamino)ethylrestes in eine Verbindung der allgemeinen Formel VIII, diese Verbindung in einem polaren Lösungsmittel mit Formaldehyd bzw. Paraformaldehyd oder, zur Einführung des zweitgenannten Restes, mit Acetaldehyd bzw. Paraldehyd bzw. Metaldehyd und mit einem sekundären Amin der allgemeinen Formel X

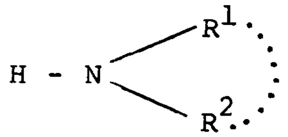

(X)

in der

$R^1$ und $R^2$ wie eingangs angegeben definiert sind, gegebenenfalls in Gegenwart von Kupfersalzen, bei Temperaturen zwischen 20°C und dem Siedepunkt des Reaktionsgemisches, gegebenenfalls in Gegenwart eines schwach alkalischen Reaktionsgemisches, umgesetzt wird.

3. Verfahren gemäß Anspruch 1e, dadurch gekennzeichnet, daß

a.) die Verbindung der allgemeinen Formel VIII mit einem Aminal der allgemeinen Formel IXb

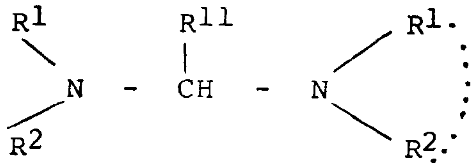

, (IXb)

in der

$R^1$ und $R^2$ wie oben definiert sind und $R^{11}$ ein Wasserstoffatom oder eine Methylgruppe bedeutet, oder

b.) die Verbindung der allgemeinen Formel VIII oder die davon abgeleitete Grignard-Verbindung der allgemeinen Formel VIIIa,

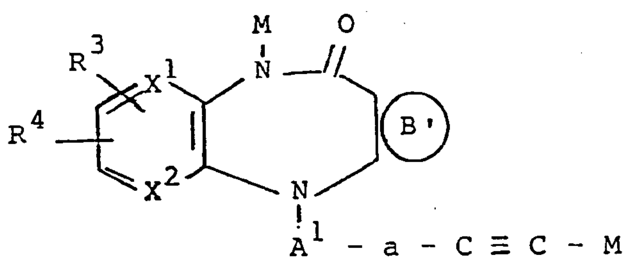

(VIIIa)

worin a, $A^1$, 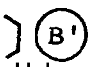 , $R^3$, $R^4$, $X^1$ und $X^2$ wie angegeben definiert sind

und M die MgHalgruppe (Hal=Halogenatom) ist, mit einem Alkoxyamin der allgemeinen Formel IXc,

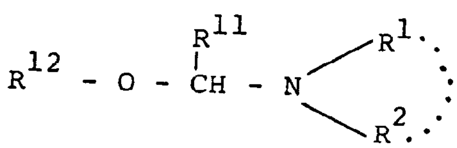

(IXc)

in der

$R^1$, $R^2$ und $R^{11}$ wie oben definiert sind und

$R^{12}$ einen Alkylrest mit 1 bis 10 Kohlenstoffatomen darstellt, in einem Lösungsmittel umgesetzt wird oder

c.) ein Metallsalz der obigen allgemeinen Formel VIIIa, in der M ein Alkalimetallatom oder den Rest MgHal (Hal=Halogen) bedeutet, mit einer Verbindung der allgemeinen Formel IX, in der b eine gegebenenfalls methylsubstituierte 1,2-Ethylen-, 1,3-Propylen- oder 1,4-Butylengruppe bedeutet, in einem organischen Lösungsmittel bei Temperaturen zwischen -80°C und +150°C, gegebenenfalls in Gegenwart eines Kupfersalzes, umgesetzt wird und, gegebenenfalls, das erhaltene Isomerengemisch anschließend aufgetrennt und/oder die erhaltenen Verbindungen in ihre Säureadditionssalze mit anorganischen oder organischen Säuren übergeführt werden.

## Claims

### Claims for the following Contracting States : AT, BE, CH, DE, FR, GB, IT, LI, LU, NL, SE

1. Condensed diazepinones of general formula I

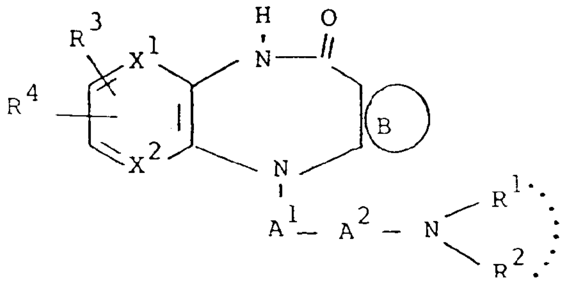

(I)

wherein ]Ⓑ represents one of the divalent groups

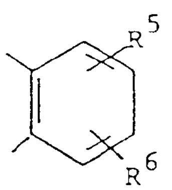 ,

(S)

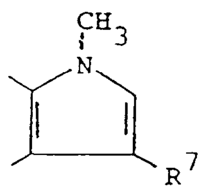 ,

(T)

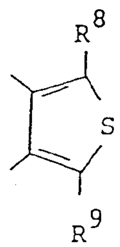 ,

(U)

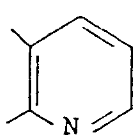

(V)

or

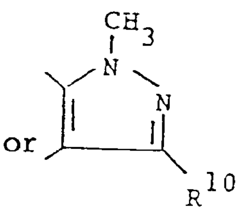

(W)

and
$X^1$, $X^2$, $A^1$, $A^2$ and $R^1$ to $R^{10}$ are defined as follows:
$X^1$ and $X^2$ represent a =CH group or, if ]Ⓑ assumes the definitions of the divalent groups (S), (U) or (W) mentioned above, both or only $X^1$ or only $X^2$ may also represent a nitrogen atom;
$A^1$ is a methylene group optionally substituted by one or two methyl groups;
$A^2$ is a straight-chained alkylene chain optionally containing a double or triple bond and comprising from 3 to 7 carbon atoms, which may additionally be methyl-substituted;
$R^1$ is a branched or unbranched alkyl group with 1 to 4 carbon atoms;
$R^2$ is a branched or unbranched alkyl group with 1 to 7 carbon atoms which may optionally also be substituted by a hydroxy group at its 2nd to 7th carbon atoms, or it represents a cycloalkyl or cycloalkylmethyl group with 3 to 7 carbon atoms in the ring, whilst the cycloalkyl ring may optionally also be substituted by a hydroxy group;
$R^1$ and $R^2$ together with the intervening nitrogen atom, may however also form a 4- to 7-membered saturated, monocyclic, heterocyclic ring which may optionally be interrupted by an oxygen atom or a nitrogen function and/or may be substituted by a $C_{1-6}$ alkyl group itself optionally substituted by a phenyl, di($C_{1-3}$alkyl)amino or (cycloalkyl)alkylamino group or by a (cycloalkyl)alkyl group optionally containing one or two etheric-oxygen atoms, whilst the above-mentioned cycloalkyl groups may contain 4 to 7 carbon atoms and the alkylene groups attached thereto may contain 1 to 3 carbon atoms;
$R^3$ is an alkyl group with 1 to 4 carbon atoms, a chlorine atom or a hydrogen atom;
$R^4$ is a hydrogen atom or a methyl group;
$R^5$ and $R^6$ each represents a hydrogen atom, a fluorine, chlorine or bromine atom or an alkyl group with 1 to 4 carbon atoms;
$R^7$ represents a hydrogen or chlorine atom or a methyl group;
$R^8$ represents a hydrogen atom or an alkyl group with 1 to 4 carbon atoms;
$R^9$ represents a hydrogen or halogen atom or an alkyl group with 1 to 4 carbon atoms and
$R^{10}$ represents a hydrogen atom or a methyl group,
wherein, if ]Ⓑ represents the divalent group T and $R^7$ is a hydrogen atom, $R^3$ cannot represent a chlorine atom and $A^2$ cannot contain any double or triple bonds,
and the diastereomeric and enantiomeric forms thereof and the physiologically acceptable salts thereof with

inorganic or organic acids.

2. Condensed diazepinones as claimed in claim 1, wherein in general formula I

$X^1$ is a =CH- group;

$X^2$ either represents a nitrogen atom and ]Ⓑ represents the divalent group (S) with the proviso that $R^3$, $R^4$ and $R^5$ are hydrogen atoms and $R^6$ is a hydrogen atom, a chlorine or bromine atom or a methyl or ethyl group in the 8 or 9-position of the heterocyclic group,

or represents a =CH- group, if ]Ⓑ assumes the meaning of the divalent groups U or V, whilst $R^8$ is a hydrogen atom and $R^9$ is a methyl group;

$A^1$ is the methylene group;

$A^2$ is a straight-chained alkylene chain optionally containing a double or triple bond and comprising 3 to 6 carbon atoms;

$R^1$ is a branched or unbranched alkyl group with 1 to 4 carbon atoms;

$R^2$ is a branched or unbranched alkyl group with 1 to 7 carbon atoms, a cycloalkyl or (cycloalkyl)methyl group with 3 to 7 carbon atoms in the ring, whilst the cycloalkyl ring may optionally also be substituted by a hydroxy group,

or

$R^1$ and $R^2$ together with the intervening nitrogen atom form a 5- to 7-membered saturated, monocyclic, heterocyclic ring which may optionally be interrupted by an oxygen atom or a nitrogen atom and/or substituted by a $C_{1-3}$alkyl group optionally substituted by a phenyl, di($C_{1-3}$alkyl)amino or (cycloalkyl)alkylamino group or by a (cycloalkyl)alkyl group optionally containing one or two etheric-oxygen atoms, whilst the above-mentioned cycloalkyl groups may contain 4 to 7 carbon atoms and the alkylene groups attached thereto may contain 1 to 3 carbon atoms; and the diastereomeric and enantiomeric forms thereof and the physiologically acceptable salts thereof with inorganic or organic acids.

3. Condensed diazepinones as claimed in claim 1, wherein in general formula I,

$X^1$ is =CH- group,

$X^2$ represents a nitrogen atom and ]Ⓑ represents the divalent group (S), with the proviso that $R^3$, $R^4$ and $R^5$ are hydrogen atoms and $R^6$ is a hydrogen, chlorine or bromine atom or a methyl or ethyl group in the 8- or 9-position of the heterocycle,

$A^1$ represents a methylene group,

$A^2$ represents a straight-chained alkylene chain optionally containing a double or triple bond and comprising three to six carbon atoms and

$R^1$ and $R^2$ together with the intervening nitrogen atom represent a dimethylamino, diethylamino, dipropylamino, [bis(methylethyl)]amino, 1-pyrrolidinyl, 1-piperidinyl, hexahydro-1H-1-azepinyl, 4-morpholinyl, 2-[(diethylamino)methyl]-1-piperidinyl, trans-(4-hydroxy-cyclohexyl)methylamino, (cyclohexyl)methylamino, 2-[[(cyclohexyl)methylamino]methyl]-1-piperidinyl, 4-methyl-1-piperazinyl, 4-[2-(1,3-dioxolan-2-yl)ethyl]-1-piperazinyl, 4-(phenylmethyl)-1-piperazinyl or 4-(2-phenylethyl)-1-piperazinyl group,

and the diastereomeric and enantiomeric forms thereof and the physiologically acceptable salts thereof with inorganic or organic acids.

4. Compounds according to claim 1:

5,11-dihydro-11-[6-(1-piperidinyl)hexyl]-6H-pyrido-[2,3-b][1,4]benzodiazepin-6-one

11-[4-[2-[(diethylamino)methyl]-1-piperidinyl]butyl]-5,11-dihydro-6H-pyrido[2,3-b][1,4]benzodiazepin-6-one

11-[4-[2-[(diethylamino)methyl]-1-piperidinyl]-but-2-ynyl]-5,11-dihydro-6H-pyrido[2,3-b][1,4]benzodiazepin-6-one,

and the diastereomeric and enantiomeric forms thereof and the physiologically acceptable salts thereof with inorganic or organic acids.

5. Pharmaceutical compositions containing a compound as claimed in at least one of claims 1 to 4 or a physiologically acceptable acid addition salt optionally together with one or more inert carriers and/or diluents.

6. Use of the compounds as claimed in at least one of claims 1 to 4 for preparing a pharmaceutical composition suitable for use as a vagal pacemaker for the treatment of bradycardia and bradyarrhythmia.

7. Use of the compounds as claimed in at least one of claims 1 to 4 for the preparation of a pharmaceutical composition with spasmolytic properties on peripheral organs, with antiemetic properties, with properties which promote cerebral blood flow and for the treatment of diseases of the central nervous system, particularly Alzheimer's disease and Parkinson's disease.

8. Process for preparing condensed diazepinones of general formula I according to claim 1, characterised in that

a.) in order to prepare compounds of general formula I wherein $R^7$ represents a chlorine atom or a methyl group and the other groups are defined as hereinbefore, a tricyclic compound of general formula II

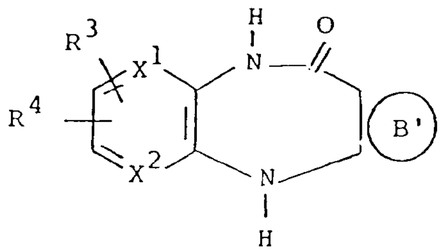

(II)

wherein

the groups $R^3$, $R^4$, $X^1$ and $X^2$ are as hereinbefore defined and ]B' represents one of the divalent groups S, U, V, W or T'

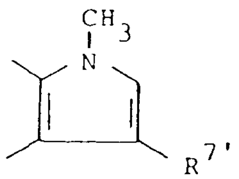

(T')

wherein $R^{7'}$ represents a chlorine atom or a methyl group, is converted into the di-lithium salt thereof and this is subsequently alkylated with a compound of general IV,

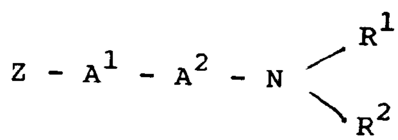

(IV)

wherein
$A^1$, $A^2$, $R^1$ and $R^2$ are as hereinbefore defined and Z represents a chlorine, bromine or iodine atom or a methanesulphonyloxy, ethanesulphonyloxy or arenesulphonyloxy group, whilst the production of the di-lithium salt and the alkylation, which may also take place in the same reaction medium, are carried out in an organic solvent at temperatures of between -80 and +70°C, or

b.) in order to prepare compounds of general formula I wherein $A^2$ represents a straight-chained saturated alkylene chain containing from 3 to 7 carbon atoms which may optionally also be methyl-substituted and $R^7$ represents a chlorine atom or a methyl group, an (arylmethyl)tricyclic compound of general formula V

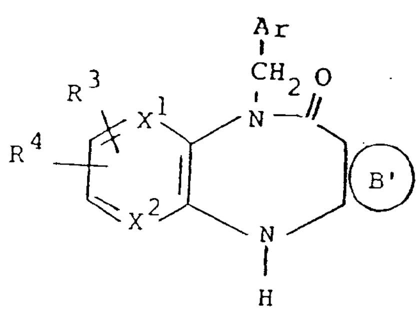

(V)

wherein the groups ]B', $R^3$, $R^4$, $X^1$ and $X^2$ are as hereinbefore defined and Ar represents a phenyl group optionally substituted by one to two methyl and/or methoxy groups, is converted into its metal salt of general formula Va

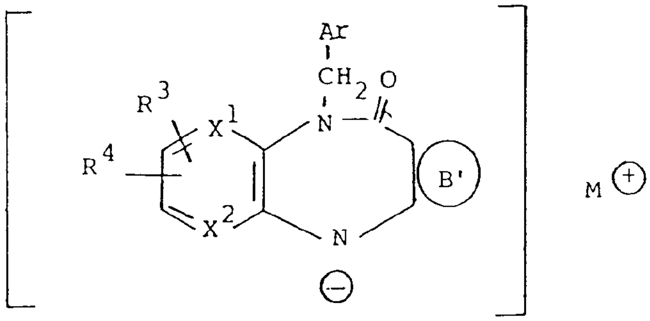

(Va)

wherein M is an alkali metal atom or the group MgHal, wherein Hal is chlorine, bromine or iodine, and this is subsequently alkylated with a compound of general formula IV

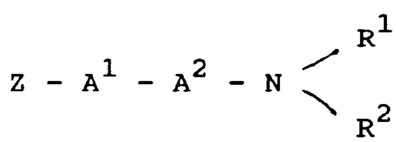

(IV)

wherein

$A^1$, $R^1$, $R^2$ and Z are as hereinbefore defined and $A^2$ represents a straight-chained saturated alkylene chain comprising three to seven carbon atoms and optionally also methyl-substituted, and from the compound thus formed the arylmethyl group is subsequently removed acidolytically, the metallisation being carried out in an inert organic solvent at temperatures of between -100°C and the boiling point of the reaction mixture with lithium aryls, lithium alkyls, lithium dialkylamides or Grignard reagents, alkali metal hydrides, alkali metal amides or alkali metal hydroxides, the alkylation is carried out in an organic solvent at temperatures of between -80 and +70°C and the acidolytic splitting off of the arylmethyl group with strong acids or Lewis acids is carried out at temperatures of between -20 and +150°C or

c.) in order to prepare compounds of general formula I wherein $A^2$ represents a straight-chained saturated alkylene chain which comprises three to seven carbon atoms and optionally also is methyl-substituted, $R^3$ represents a hydrogen atom or an alkyl group with 1 to 4 carbon atoms and ]Ⓑ represents one of the divalent groups S, T, U, or W with the proviso that $R^5$, $R^6$ and $R^7$ cannot be chlorine or bromine, a compound of general formula VIa

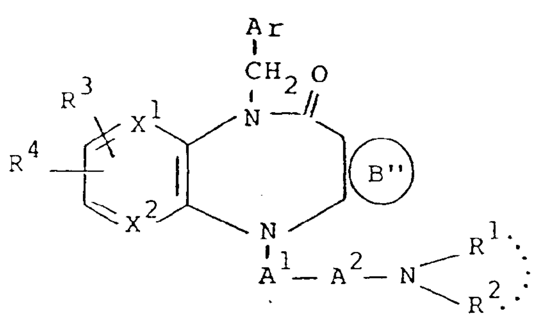

(VIa)

(wherein

the groups $A^1$, $A^2$, $R^1$, $R^2$, $R^3$, $R^4$, $X^1$, $X^2$ and Ar are as hereinbefore defined and ](B'') represents one of the divalent groups S, T, U or W with the proviso that $R^7$ is not a hydrogen atom) is hydrogenolysed in the presence of catalysts comprising metals of the VIIIth subgroup of the Periodic Table of Elements, under hydrogen pressures of from 1 to 300 bar and at temperatures of from 0°C to 130°C and in the presence of solvents and, optionally, inorganic acids, or

d.) in order to prepare compounds of general formula I wherein $A^2$ represents a straight-chained alkyl chain optionally containing a double bond and comprising three to seven carbon atoms and optionally additionally being methyl-substituted, $R^3$ represents a hydrogen atom or an alkyl group with 1 to 4 carbon atoms and ]Ⓑ represents one of the divalent groups S, T, U or W, with the proviso that $R^5$, $R^6$ and $R^7$ do not represent

chlorine or bromine atoms, a compound of general formula VII

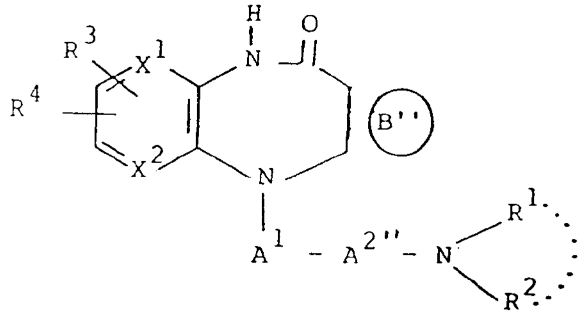

(VII)

(wherein

the group $A^1$, $R^1$ to $R^4$, $X^1$, $X^2$ and ](B'') are as hereinbefore defined and $A^{2''}$ represents a straight-chained alkylene chain which contains a triple bond and comprises three to seven carbon atoms, and which may also be methyl-substituted) is catalytically hydrogenated in the presence of metals of the VIIIth subgroup of the Periodic Table of Elements under hydrogen pressures of from 0.1 to 300 bar and at temperatures of from 0 to 130°C and in the presence of solvents, whilst in order to prepare compounds of general formula I wherein $A^2$ represents an alkylene group containing a double bond, the catalytic hydrogenation is either broken off after the uptake of 1 mol of hydrogen or is carried out in the presence of a deactivating catalyst, or

e.) in order to prepare compounds of general formula I wherein $A^2$ is a straight-chained alkylene chain containing a triple bond and comprising three to seven carbon atoms and possibly also being methyl- substituted and ]Ⓑ represents the divalent group ](B') defined above, a condensed tricyclic compound of general formula VIII,

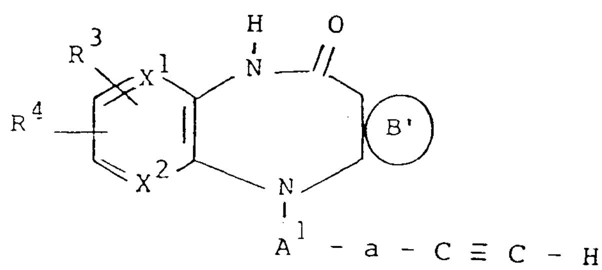

(VIII)

(wherein

$A^1$, ](B'), $R^3$, $R^4$, $X^1$ and $X^2$ are as hereinbefore defined and "a" represents a single bond or a straight-chained, saturated, optionally methyl-substituted alkylene chain with 1 to 4 carbon atoms) is aminoalkylated with a compound of general formula

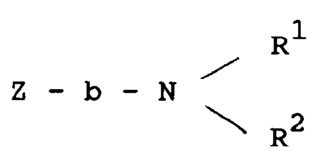

(IX)

wherein

$R^1$, $R^2$ and Z are as hereinbefore defined and "b" represents a straight-chained, saturated, optionally methyl-substituted alkylene group with 1 to 4 carbon atoms) optionally in the presence of copper salts, in a polar solvent at temperatures of between 20°C and the boiling point of the reaction mixture, optionally in the presence of a slightly alkaline medium,

and subsequently if desired a compound of general formula I thus obtained is separated into the isomers thereof and/or a compound of general formula I thus obtained is converted into the acid addition salts thereof, preferably into the physiologically acceptable acid addition salts thereof with inorganic or organic acids.

9. Process as claimed in claim 8e, characterised in that in order to introduce a (dialkylamino)methyl- or 1-(dialkylamino)ethyl group into a compound of general formula VIII, this compound is reacted in a polar solvent with formaldehyde or paraformaldehyde or, in order to introduce the second of these groups, with acetaldehyde or paraldehyde or metaldehyde and with a secondary amine of general formula X

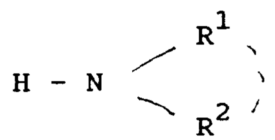

(X)

(wherein
$R^1$ and $R^2$ are as hereinbefore defined) optionally in the presence of copper salts, at temperatures of between 20°C and the boiling point of the reaction mixture, optionally in the presence of a slightly alkaline reaction mixture.

10. Process as claimed in claim 8e, characterised in that

a.) the compound of general formula VIII is reacted with an diamine of general formula IXb

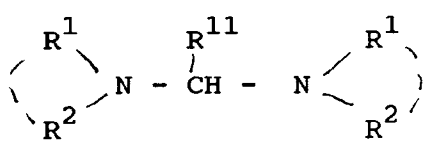

(IXb)

wherein
$R^1$ and $R^2$ are as hereinbefore defined and $R^{11}$ represents a hydrogen atom or a methyl group, or

b.) the compound of general formula VIII or the Grignard compound of general formula VIIIa derived therefrom

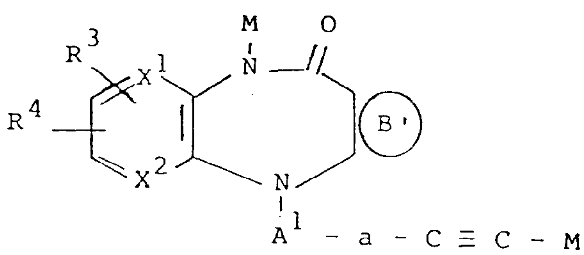

(VIIIa)

(wherein a, $A^1$, ]B', $R^3$, $R^4$, $X^1$ and $X^2$ are as hereinbefore defined and M is the MgHal group (Hal=halogen atom)) is reacted in a solvent with an alkoxyamine of general formula IXc

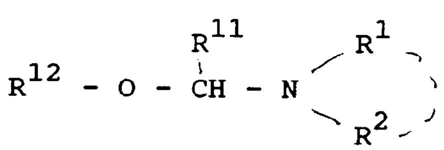

(IXc)

wherein
$R^1$, $R^2$ and $R^{11}$ are as hereinbefore defined and $R^{12}$ represents an alkyl group with 1 to 10 carbon atoms, or

c.) a metal salt of general formula VIIIa above, wherein M represents an alkali metal atom or the group MgHal (Hal=halogen), is reacted with a compound of general formula IX wherein b represents an optionally methyl-substituted 1,2-ethylene, 1,3-propylene or 1,4-butylene group, in an organic solvent at temperatures of between -80°C and +150°C, optionally in the presence of a copper salt, and, optionally, the isomer mixture obtained is subsequently separated and/or the compounds obtained are converted into the acid

addition salts thereof with inorganic or organic acids.

**Claims for the following Contracting States : ES, GR**

1. Process for preparing new condensed diazepinones of general formula I

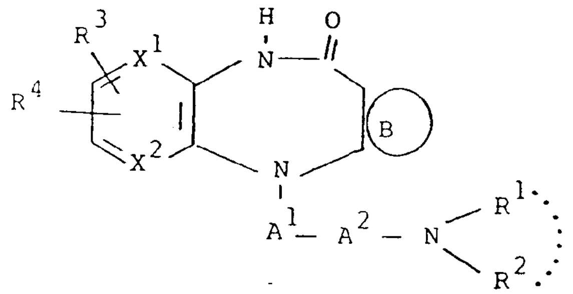

(I)

wherein ]Ⓑ represents one of the divalent groups

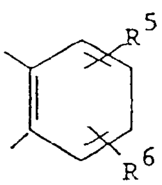 , 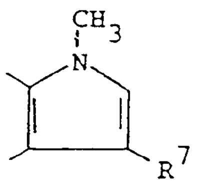 , 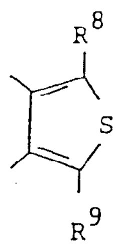 , 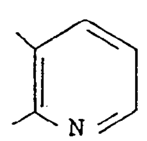 or 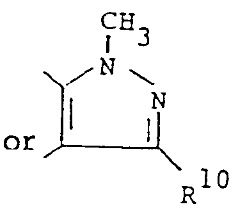

(S) (T) (U) (V) (W)

and

$X^1$, $X^2$, $A^1$, $A^2$ and $R^1$ to $R^{10}$ are defined as follows:

$X^1$ and $X^2$ represent a =CH group or, if ]Ⓑ assumes the definitions of the divalent groups (S), (U) or (W) mentioned above, both or only $X^1$ or only $X^2$ may also represent a nitrogen atom;

$A^1$ is a methylene group optionally substituted by one or two methyl groups;

$A^2$ is a straight-chained alkylene chain optionally containing a double or triple bond and comprising from 3 to 7 carbon atoms, which may additionally be methyl-substituted;

$R^1$ is a branched or unbranched alkyl group with 1 to 4 carbon atoms;

$R^2$ is a branched or unbranched alkyl group with 1 to 7 carbon atoms which may optionally also be substituted by a hydroxy group at its 2nd to 7th carbon atoms, or it represents a cycloalkyl or cycloalkylmethyl group with 3 to 7 carbon atoms in the ring, whilst the cycloalkyl ring may optionally also be substituted by a hydroxy group;

$R^1$ and $R^2$ together with the intervening nitrogen atom, may however also form a 4- to 7-membered saturated, monocyclic, heterocyclic ring which may optionally be interrupted by an oxygen atom or a nitrogen function and/or may be substituted by a $C_{1-6}$ alkyl group itself optionally substituted by a phenyl, di($C_{1-3}$alkyl)amino or (cycloalkyl)alkylamino group or by a (cycloalkyl)alkyl group optionally containing one or two etheric-oxygen atoms, whilst the above-mentioned cycloalkyl groups may contain 4 to 7 carbon atoms and the alkylene groups attached thereto may contain 1 to 3 carbon atoms;

$R^3$ is an alkyl group with 1 to 4 carbon atoms, a chlorine atom or a hydrogen atom;

$R^4$ is a hydrogen atom or a methyl group;

$R^5$ and $R^6$ each represents a hydrogen atom, a fluorine, chlorine or bromine atom or an alkyl group with 1 to 4 carbon atoms;

$R^7$ represents a hydrogen or chlorine atom or a methyl group;

$R^8$ represents a hydrogen atom or an alkyl group with 1 to 4 carbon atoms;

$R^9$ represents a hydrogen or halogen atom or an alkyl group with 1 to 4 carbon atoms and

$R^{10}$ represents a hydrogen atom or a methyl group,

wherein, if ]Ⓑ represents the divalent group T and $R^7$ is a hydrogen atom, $R^3$ cannot represent a chlorine atom and $A^2$ cannot contain any double or triple bonds,

and the diastereomeric and enantiomeric forms thereof as well as the salts thereof with organic or inorganic

acids, characterised in that

a.) in order to prepare compounds of general formula I wherein $R^7$ represents a chlorine atom or a methyl group and the other groups are defined as hereinbefore, a tricyclic compound of general formula II

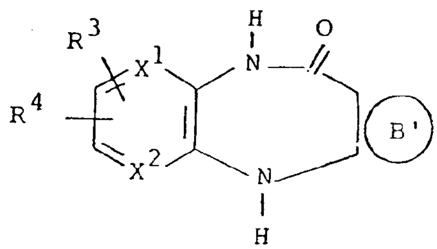

(II)

wherein

the groups $R^3$, $R^4$, $X^1$ and $X^2$ are as hereinbefore defined and 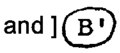 represents one of the divalent groups S, U, V, W or T'

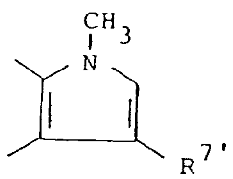

(T')

wherein $R^7$ represents a chlorine atom or a methyl group, is converted into the di-lithium salt thereof and this is subsequently alkylated with a compound of general IV,

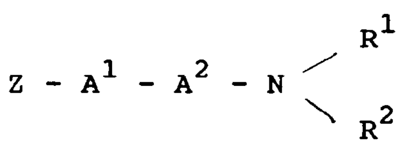

(IV)

wherein

$A^1$, $A^2$, $R^1$ and $R^2$ are as hereinbefore defined and Z represents a chlorine, bromine or iodine atom or a methanesulphonyloxy, ethanesulphonyloxy or arenesulphonyloxy group, whilst the production of the di-lithium salt and the alkylation, which may also take place in the same reaction medium, are carried out in an organic solvent at temperatures of between -80 and +70°C, or

b.) in order to prepare compounds of general formula I wherein $A^2$ represents a straight-chained saturated alkylene chain containing from 3 to 7 carbon atoms which may optionally also be methyl-substituted and $R^7$ represents a chlorine atom or a methyl group, an (arylmethyl)tricyclic compound of general formula V

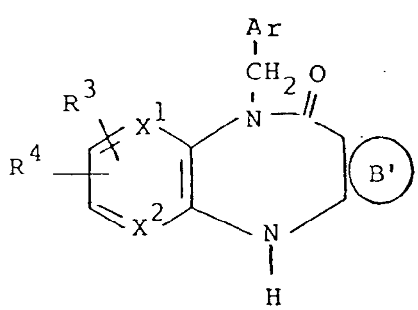

(V)

wherein the groups 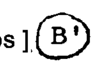, $R^3$, $R^4$, $X^1$ and $X^2$ are as hereinbefore defined and Ar represents a phenyl group

optionally substituted by one to two methyl and/or methoxy groups, is converted into its metal salt of general formula Va

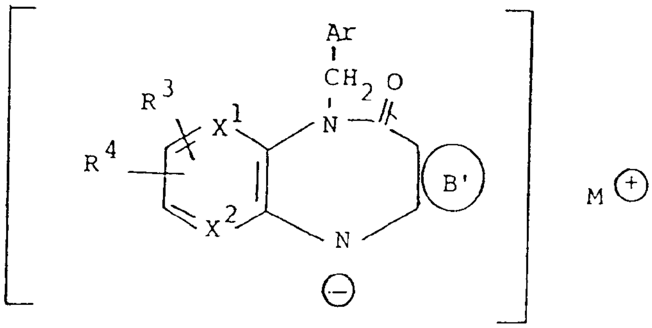

(Va)

wherein M is an alkali metal atom or the group MgHal, wherein Hal is chlorine, bromine or iodine, and this is subsequently alkylated with a compound of general formula IV

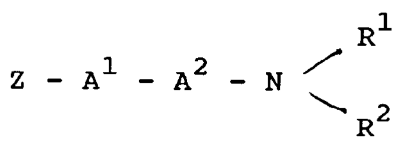

(IV)

wherein

$A^1$, $R^1$, $R^2$ and Z are as hereinbefore defined and $A^2$ represents a straight-chained saturated alkylene chain comprising three to seven carbon atoms and optionally also methyl-substituted, and from the compound thus formed the arylmethyl group is subsequently removed acidolytically, the metallisation being carried out in an inert organic solvent at temperatures of between -100°C and the boiling point of the reaction mixture with lithium aryls, lithium alkyls, lithium dialkylamides or Grignard reagents, alkali metal hydrides, alkali metal amides or alkali metal hydroxides, the alkylation is carried out in an organic solvent at temperatures of between -80 and +70°C and the acidolytic splitting off of the arylmethyl group with strong acids or Lewis acids is carried out at temperatures of between -20 and +150°C or

c.) in order to prepare compounds of general formula I wherein $A^2$ represents a straight-chained saturated alkylene chain which comprises three to seven carbon atoms and optionally also is methyl-substituted, $R^3$ represents a hydrogen atom or an alkyl group with 1 to 4 carbon atoms and ]Ⓑ represents one of the divalent groups S, T, U or W with the proviso that $R^5$, $R^6$ and $R^7$ cannot be chlorine or bromine, a compound of general formula VIa

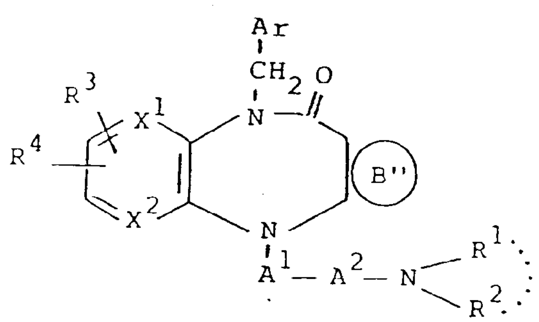

(VIa)

(wherein

the groups $A^1$, $A^2$, $R^1$, $R^2$, $R^3$, $R^4$, $X^1$, $X^2$ and Ar are as hereinbefore defined and ](B'') represents one of the divalent groups S, T, U or W with the proviso that $R^7$ is not a hydrogen atom) is hydrogenolysed in the presence of catalysts comprising metals of the VIIIth subgroup of the Periodic Table of Elements, under hydrogen pressures of from 1 to 300 bar and at temperatures of from 0°C to 130°C and in the presence of solvents and, optionally, inorganic acids, or

d.) in order to prepare compounds of general formula I wherein $A^2$ represents a straight-chained alkyl chain optionally containing a double bond and comprising three to seven carbon atoms and optionally additionally being methyl-substituted, $R^3$ represents a hydrogen atom or an alkyl group with 1 to 4 carbon atoms and ]Ⓑ represents one of the divalent groups S, T, U or W, with the proviso that $R^5$, $R^6$ and $R^7$ do not represent chlorine or bromine atoms,

a compound of general formula VII

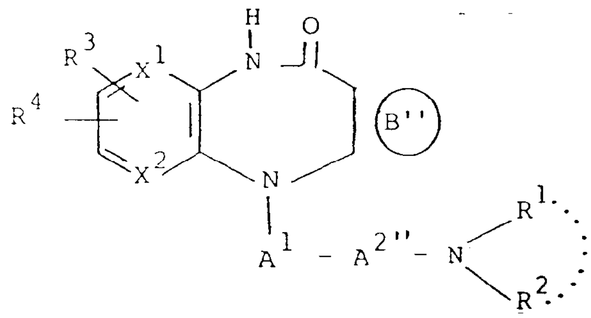

(VII)

the groups $A^1$, $R^1$ to $R^4$, $X^1$, $X^2$ and ](B'') are as hereinbefore defined and $A^{2''}$ represents a straight-chained alkylene chain which contains a triple bond and comprises three to seven carbon atoms, and which may also be methyl-substituted) is catalytically hydrogenated in the presence of metals of the VIIIth subgroup of the Periodic Table of Elements under hydrogen pressures of from 0.1 to 300 bar and at temperatures of from 0 to 130°C and in the presence of solvents, whilst in order to prepare compounds of general formula I wherein $A^2$ represents an alkylene group containing a double bond, the catalytic hydrogenation is either broken off after the uptake of 1 mol of hydrogen or is carried out in the presence of a deactivating catalyst, or

e.) in order to prepare compounds of general formula I wherein $A^2$ is a straight-chained alkylene chain containing a triple bond and comprising three to seven carbon atoms and possibly also being methyl- substituted and ]Ⓑ represents the divalent group ](B') defined above, a condensed tricyclic compound of general formula VIII,

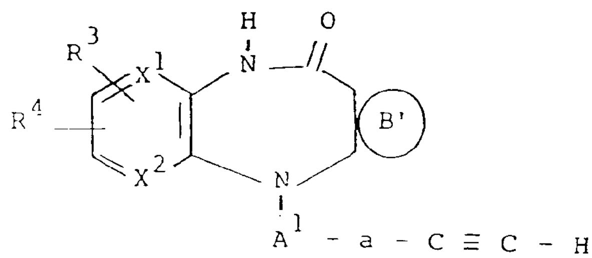

(VIII)

(wherein

$A^1$, ](B'), $R^3$, $R^4$, $X^1$ and $X^2$ are as herein- before defined and "a" represents a single bond or a straight-chained, saturated, optionally methyl-substituted alkylene chain with 1 to 4 carbon atoms) is aminoalkylated with a compound of general formula

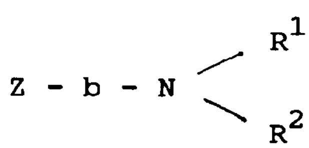

(IX)

wherein

$R^1$, $R^2$ and Z are as hereinbefore defined and "b" represents a straight-chained, saturated, optionally methyl-substituted alkylene group with 1 to 4 carbon atoms) optionally in the presence of copper salts, in a polar solvent at temperatures of between 20°C and the boiling point of the reaction mixture, optionally in the presence of a slightly alkaline medium,

and subsequently if desired a compound of general formula I thus obtained is separated into the isomers thereof and/or a compound of general formula I thus obtained is converted into the acid addition salts thereof, preferably into the physiologically acceptable acid addition salts thereof with inorganic or organic acids.

2. Process as claimed in claim 1e, characterised in that in order to introduce a (dialkylamino)methyl- or 1-(dialkylamino)ethyl group into a compound of general formula VIII, this compound is reacted in a polar solvent with formaldehyde or paraformaldehyde or, in order to introduce the second of these groups, with acetaldehyde or paraldehyde or metaldehyde and with a secondary amine of general formula X

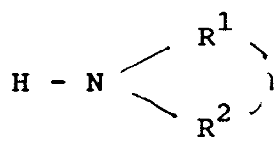

(X)

(wherein
$R^1$ and $R^2$ are as hereinbefore defined) optionally in the presence of copper salts, at temperatures of between 20°C and the boiling point of the reaction mixture, optionally in the presence of a slightly alkaline reaction mixture.

3. Process as claimed in claim 1e, characterised in that

a.) the compound of general formula VIII is reacted with an diamine of general formula IXb

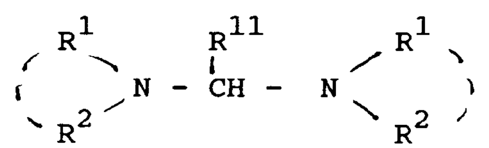

(IXb)

wherein
$R^1$ and $R^2$ are as hereinbefore defined and $R^{11}$ represents a hydrogen atom or a methyl group, or

b.) the compound of general formula VIII or the Grignard compound of general formula VIIIa derived therefrom

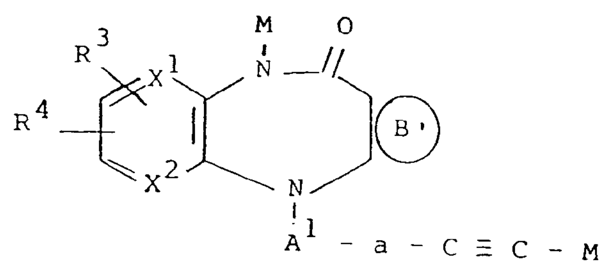

(VIIIa)

(wherein a, $A^1$, ]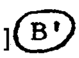, $R^3$, $R^4$, $X^1$ and $X^2$ are as hereinbefore defined and M is the MgHal group (Hal=halogen atom)) is reacted in a solvent with an alkoxyamine of general formula IXc

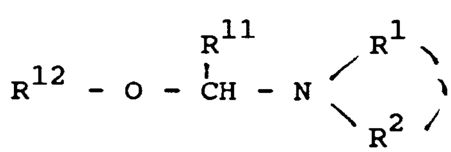

(IXc)

wherein
$R^1$, $R^2$ and $R^{11}$ are as hereinbefore defined and $R^{12}$ represents an alkyl group with 1 to 10 carbon atoms, or

c.) a metal salt of general formula VIIIa above, wherein M represents an alkali metal atom or the group MgHal (Hal=halogen), is reacted with a compound of general formula IX wherein b represents an optionally

methyl-substituted 1,2-ethylene, 1,3-propylene or 1,4-butylene group, in an organic solvent at temperatures of between -80°C and +150°C, optionally in the presence of a copper salt, and, optionally, the isomer mixture obtained is subsequently separated and/or the compounds obtained are converted into the acid addition salts thereof with inorganic or organic acids.

**Revendications**

**Revendications pour les Etats contractants suivants : AT, BE, CH, DE, FR, GB, IT, LI, LU, NL, SE**

1. Diazépinones condensées de formule générale I

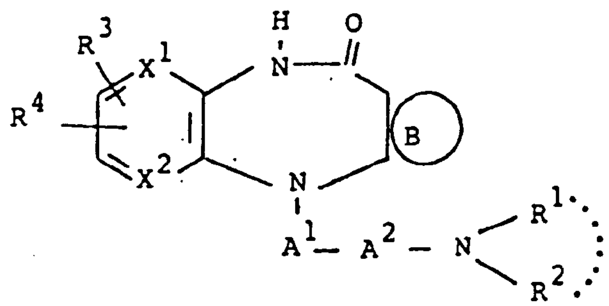

(I)

dans laquelle 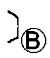 représente l'un des restes divalents

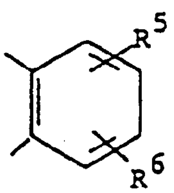 , 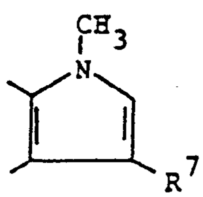 , 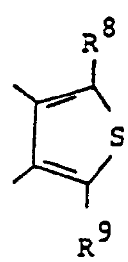 , 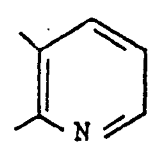 oder 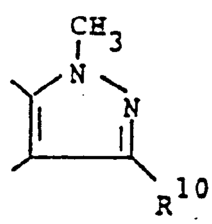

(S) (T) (U) (V) (W)

et

$X^1$, $X^2$, $A^1$, $A^2$ et $R^1$ à $R^{10}$ possèdent les significations suivantes:

$X^1$ et $X^2$ représentent un groupe =CH- ou, dans la mesure où 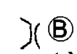 a les significations des restes divalents (S), (U) ou (W) cités ci-dessus, peuvent l'un et l'autre ou seulement $X^1$ ou seulement $X^2$ représenter aussi un atome d'azote;
$A^1$ est un groupe méthylène substitué éventuellement par un ou deux groupes méthyle;
$A^2$ représente une chaîne alkylène linéaire, contenant éventuellement une double ou triple liaison et comprenant trois à sept atomes de carbone, qui peut en outre être méthylsubstituée;
$R^1$ représente un reste alkyle ramifié ou non ramifié de 1 à 4 atomes de carbone;
$R^2$ représente un reste alkyle ramifié ou non ramifié de 1 à 7 atomes de carbone qui peut en outre éventuellement être substitué sur son deuxième à septième atome de carbone par un groupe hydroxyle, un reste cycloalkyle ou un reste cycloalkylméthyle ayant 3 à 7 atomes de carbone dans le cycle, le cycle cycloalkyle pouvant éventuellement être substitué encore par un groupe hydroxyle;
$R^1$ et $R^2$ peuvent cependant également former avec l'atome d'azote situé entre eux un cycle monocyclique, hétérocyclique saturé, de 4 à 7 chaînons, qui peut éventuellement être interrompu par un atome d'oxygène ou par une fonction azote et/ou qui peut être substitué par un reste alkyle de 1 à 6 atomes de carbone éventuellement substitué par un groupe phényle, dialkyl($C_1$-$C_3$)amino ou (cycloalkyl)alkylamino ou par un reste (cycloalkyl)alkyle contenant éventuellement un ou deux atomes d'oxygène d'éther, les groupes cycloalkyle

cités ci-dessus pouvant contenir 4 à 7 atomes de carbone et les groupes alkylène liés à ceux-ci pouvant contenir 1 à 3 atomes de carbone;
$R^3$ est un groupe alkyle de 1 à 4 atomes de carbone, un atome de chlore ou un atome d'hydrogène;
$R^4$ est un atome d'hydrogène ou un groupe méthyle;
$R^5$ et $R^6$ représentent chacun un atome d'hydrogène, un atome de fluor, de chlore ou de brome ou un groupe alkyle de 1 à 4 atomes de carbone;
$R^7$ représente un atome d'hydrogène ou de chlore ou un groupe méthyle;
$R^8$ représente un atome d'hydrogène ou un reste alkyle de 1 à 4 atomes de carbone;
$R^9$ représente un atome d'hydrogène ou d'halogène ou un groupe alkyle de 1 à 4 atomes de carbone et
$R^{10}$ représente un atome d'hydrogène ou un groupe méthyle, et lorsque )( Ⓑ représente le reste divalent T et $R^7$ est un atome d'hydrogène, $R^3$ ne doit pas représenter un atome de chlore et $A^2$ ne doit contenir aucune double ou triple liaison, et leurs formes diastéréoisomères et énantiomères, leurs sels compatibles du point de vue physiologique avec des acides inorganiques ou organiques.

2. Diazépinones condensées selon la revendication 1, dans lesquelles dans la formule générale I
$X^1$ représente un groupe =CH-,
$X^2$ représente soit un atome d'azote et )( Ⓑ représente le reste divalent (S), avec la condition que $R^3$, $R^4$ et $R^5$ soient des atomes d'hydrogène et que $R^6$ soit un atome d'hydrogène, un atome de chlore ou de brome ou un groupe méthyle ou éthyle en position 8 ou 9 de l'hétérocycle, soit un groupe =CH-, lorsque )( Ⓑ a la signification des restes divalents U ou V, $R^8$ étant un atome d'hydrogène et $R^9$ un groupe méthyle;
$A^1$ représente le groupe méthylène;
$A^2$ représente une chaîne alkylène linéaire, contenant éventuellement une double ou triple liaison et comprenant trois à six atomes de carbone;
$R^1$ représente un reste alkyle ramifié ou non ramifié contenant 1 à 4 atomes de carbone;
$R^2$ représente un reste alkyle ramifié ou non ramifié de 1 à 7 atomes de carbone, un reste cycloalkyle ou (cycloalkyl)méthyle de 3 à 7 atomes de carbone dans le cycle, le cycle cycloalkyle pouvant éventuellement être substitué encore par un groupe hydroxyle,
ou bien
$R^1$ et $R^2$ forment avec l'atome d'azote situé entre eux un cycle hétérocyclique, monocyclique, saturé, de 5 à 7 chaînons qui peut éventuellement être interrompu par un atome d'oxygène ou un atome d'azote et/ou être substitué par un reste alkyle de 1 à 3 atomes de carbone éventuellement substitué par un groupe phényle, dialkyl($C_1$-$C_3$)amino ou (cycloalkyl)alkylamino, ou par un reste (cycloalkyl)alkyle contenant éventuellement un ou deux atomes d'oxygène d'éther, les groupes cycloalkyle cités précédemment pouvant contenir 4 à 7 atomes de carbone et les groupes alkylène liés à ceux-ci pouvant contenir 1 à 3 atomes de carbone; et leurs formes diastéréoisomères et énantiomères, leurs sels compatibles du point de vue physiologique avec des acides inorganiques ou organiques.

3. Diazépinones condensées selon la revendication 1, dans lesquelles dans la formule générale I
$X^1$ représente un groupe =CH-,
$X^2$ représente un atome d'azote et B représente le reste divalent (S), avec la condition que $R^3$, $R^4$ et $R^5$ soient des atomes d'hydrogène et que $R^6$ soit un atome d'hydrogène, un atome de chlore ou de brome ou un groupe méthyle ou éthyle en position 8 ou 9 de l'hétérocycle,
$A^1$ représente le groupe méthylène,
$A^2$ représente une chaîne alkylène linéaire, contenant éventuellement une double ou triple liaison et comprenant trois à six atomes de carbone et
$R^1$ et $R^2$ représentent avec l'atome d'azote situé entre eux le reste diméthylamino, diéthylamino, dipropylamino, [bis(méthyléthyl)]amino, 1-pyrrolidinyle, 1-pipéridinyle, hexahydro-1H-1-azépinyle, 4-morpholinyle, 2-[(diéthylamino)-méthyl]-1-pipéridinyle, trans-(4-hydroxycyclohexyl)méthyl-amino, (cyclohexyl)méthylamino, 2-[[(cyclohexyl)méthyl-amino]méthyl]-1-pipéridinyle, 4-méthyl-1-pipérazinyle, 4-[2-(1,3-dioxolane-2-yl)éthyl]-1-pipérazinyle, 4-(phénylméthyl)-1-pipérazinyle ou 4-(2-phényléthyl)-1-pipérazinyle et leurs formes diastéréoisomères et énantiomères, leurs sels compatibles du point de vue physiologique avec des acides inorganiques ou organiques.

4. Composés selon la revendication 1:
5,11-dihydro-11-[6-(1-pipéridinyl)hexyl]-6H-pyrido[2,3-b]-[1,4]benzodiazépine-6-one
11-[4-[2-[(diéthylamino)méthyl]-1-pipéridinyl]butyl]-5,11-dihydro-6H-pyrido[2,3-b][1,4]benzodiazépine-6-one
11-[4-[2-[(diéthylamino)méthyl]-1-pipéridinyl]-2-butinyl]-5,11-dihydro-6H-pyrido[2,3-b][1,4]benzodiazépine-6-one
et leurs formes diastéréoisomères et énantiomères, leurs sels compatibles du point de vue physiologique avec

des acides inorganiques et organiques.

5. Médicament contenant un composé selon au moins l'une des revendications 1 à 4 ou un sel d'addition d'acide compatible du point de vue physiologique et éventuellement un ou plusieurs véhicules et/ou diluants inertes.

6. Utilisation des composés selon au moins l'une des revendications 1 à 4 pour la préparation d'un médicament qui convient comme stimulateur vagal pour le traitement des bradycardies et des bradyarythmies.

7. Utilisation des composés selon au moins l'une des revendications 1 à 4 pour la préparation d'un médicament aux propriétés spasmolytiques sur les organes périphériques, aux propriétés antiémétiques, aux propriétés favorisant l'irrigation sanguine cérébrale et pour la thérapie des maladies du système nerveux central, en particulier de la maladie d'Alzheimer et de la maladie de Parkinson.

8. Procédé de préparation de diazépinones condensées de formule générale I selon la revendication 1, caractérisé en ce que

a.) pour la préparation de composés de formule générale I dans laquelle $R^7$ représente un atome de chlore ou un groupe méthyle et les autres restes ont les significations données ci-dessus, un système tricyclique de formule générale II

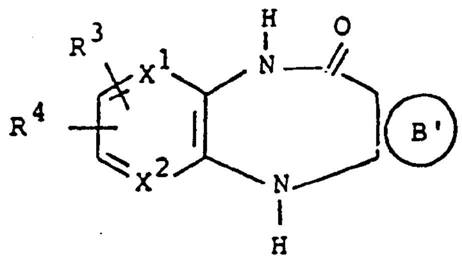

(II)

dans laquelle

les restes $R^3$, $R^4$, $X^1$ et $X^2$ sont définis de la manière indiquée ci-dessus et 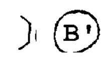 représente l'un des restes divalents S, U, V, W ou T'

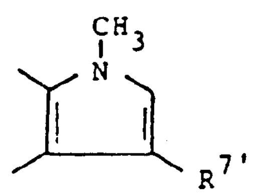

(T')

$R^{7'}$ représentant un atome de chlore ou un groupe méthyle, est converti en son sel dilithien et celui-ci est ensuite alkylé avec un composé de formule générale IV

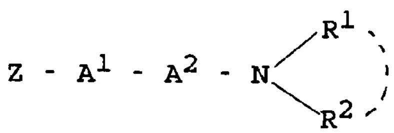

(IV)

dans laquelle

$A^1$, $A^2$, $R^1$ et $R^2$ ont les significations indiquées ci-dessus et Z représente un atome de chlore, de brome ou d'iode, le groupe méthanesulfonyloxy, éthanesulfonyloxy ou un groupe arènesulfonyloxy, la préparation du sel dilithien et l'alkylation, qui peuvent se dérouler aussi dans le même milieu réactionnel, étant accomplies dans un solvant organique à des températures comprises entre -80 et +70°C, ou

b.) pour la préparation de composés de formule générale I dans laquelle $A^2$ représente une chaîne alkylène linéaire, saturée, comportant trois à sept atomes de carbone qui peut en outre éventuellement être méthyl-substituée et $R^7$ représente un atome de chlore ou un groupe méthyle, un système (arylméthyl)tricyclique de formule générale V

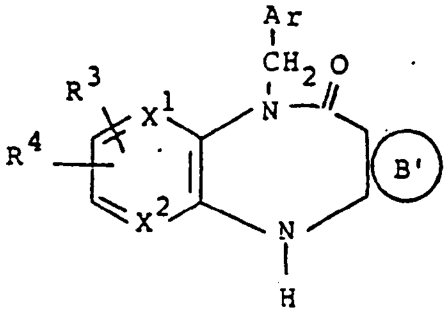

(V)

dans laquelle

les restes 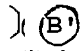, $R^3$, $R^4$, $X^1$ et $X^2$ sont définis comme ci-dessus et Ar représente un reste phényle éventuellement substitué par un ou deux groupes méthyle et/ou méthoxy, est converti en son sel métallique de formule générale Va

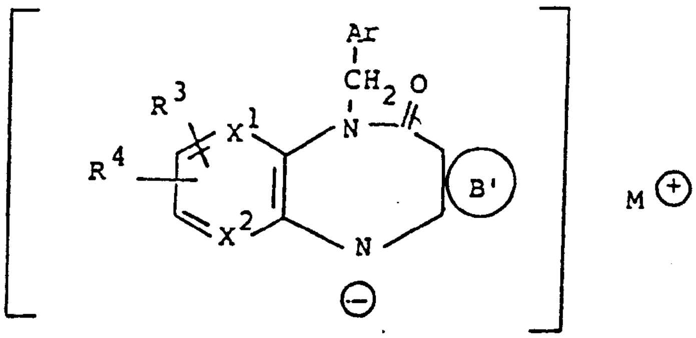

(Va)

dans laquelle M est un atome de métal alcalin ou représente le reste MgHal dans lequel Hal est le chlore, le brome ou l'iode, et celui-ci est ensuite alkylé avec un composé de formule générale IV

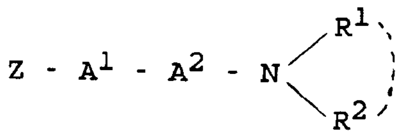

(IV)

dans laquelle

$A^1$, $R^1$, $R^2$ et Z ont les significations indiquées ci-dessus et $A^2$ représente une chaîne alkylène linéaire saturée comprenant trois à sept atomes de carbone, qui peut en outre éventuellement être méthylsubstituée, et le groupe arylméthyle est finalement séparé par voie acidolytique de ce composé ainsi formé, la métallation étant opérée dans un solvant organique inerte à des températures comprises entre -100°C et le point d'ébullition du mélange réactionnel avec des aryllithium, des alkyllithium, des dialkylamidures de lithium ou des réactifs de Grignard, des hydrures alcalins, des amidures alcalins, des hydroxydes alcalins, l'alkylation étant opérée dans un solvant organique à des températures comprises entre -80°C et +70°C et le clivage acidolytique du groupe arylméthyle étant opéré avec des acides forts ou des acides de Lewis à des températures comprises entre -20°C et +150°C

ou bien

c.) pour la préparation de composés de formule générale I dans laquelle $A^2$ représente une chaîne alkylène linéaire saturée comprenant trois à sept atomes de carbone, qui peut en outre être éventuellement méthylsubstituée, $R^3$ représente un atome d'hydrogène ou un groupe alkyle de 1 à 4 atomes de carbone et 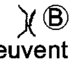 représente l'un des restes divalents S, T, U ou W avec la limitation selon laquelle $R^5$, $R^6$ et $R^7$ ne peuvent pas représenter le chlore ou le brome, un composé de formule générale VIa

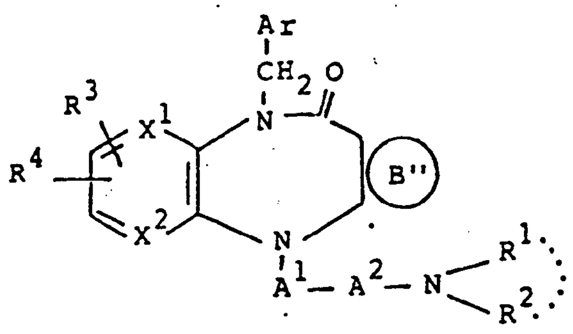

(VIa)

dans laquelle

Les restes $A^1$, $A^2$, $R^1$, $R^2$, $R^3$, $R^4$, $X^2$ et Ar ont les significations indiquée et )( (B'') représente l'un des restes divalents S, T, U ou W avec la condition que $R^7$ ne soit pas un atome d'hydrogène, est hydrogénolysé à des pressions d'hydrogène de 1 à 300 bar et à des températures de 0°C à 130°C en présence de catalyseurs de métaux du VIII$^{ème}$ sous-groupe du système périodique des éléments et en présence de solvants et, éventuellement, d'acides minéraux, ou bien

d.) pour la préparation de composés de formule générale I dans laquelle $A^2$ représente une chaîne alkylène linéaire, contenant éventuellement une double liaison et comprenant trois à sept atomes de carbone, qui peut en outre être méthylsubstituée, $R^3$ représente un atome d'hydrogène ou un groupe alkyle de 1 à 4 atomes de carbone et )(B) représente l'un des restes divalents S, T, U ou W avec la limitation selon laquelle $R^5$, $R^6$ et $R^7$ ne représentent pas un atome de chlore ou de brome, un composé de formule générale VII

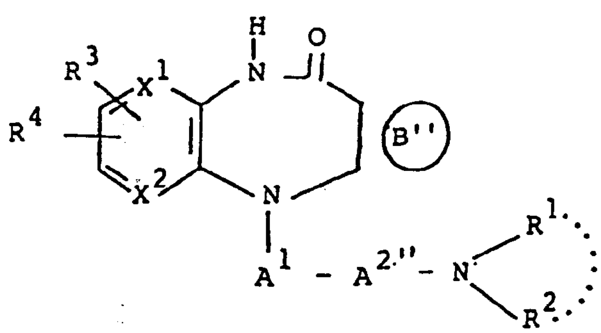

(VII)

dans laquelle

les restes $A^1$, $R^1$ à $R^4$, $X^1$, $X^2$ et )( (B'') ont les significations indiquées ci-dessus et $A^{2''}$ est une chaîne alkylène linéaire contenant une liaison triple et comportant trois à sept atomes de carbone, qui peut en outre être méthylsubstituée, est hydrogéné catalytiquement à des pressions d'hydrogène de 0,1 à 300 bar et à des températures de 0 à 130°C en présence de métaux du VIII$^{ème}$ sous-groupe du système périodique des éléments et en présence de solvants, l'hydrogénation catalytique étant soit interrompue après absorption de 1 mole d'hydrogène soit opérée en présence d'un catalyseur désactivant pour la préparation de composés de formule générale I dans laquelle $A^2$ représente un groupe alkylène contenant une double liaison, ou bien

e.) pour la préparation de composés de formule générale I dans laquelle $A^2$ est une chaîne alkylène linéaire contenant une triple liaison et comportant trois à sept atomes de carbone qui peut en outre être éventuellement méthylsubstituée et )(B) représente le reste divalent )((B') défini ci-dessus, un système tricyclique condensé de formule générale VIII

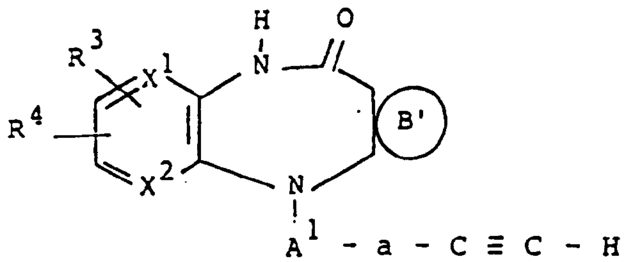

(VIII)

dans laquelle

$A^1$, 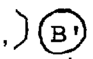, $R^3$, $R^4$, $X^1$ et $X^2$ possèdent les significations indiquées ci-dessus et "a" représente une liaison simple ou une chaîne alkylène linéaire, saturée, de 1 à 4 atomes de carbone, éventuellement substituée par un groupe méthyle, est aminoalkylé avec un composé de formule générale IX

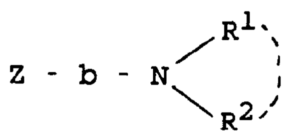

(IX)

dans laquelle
$R^1$, $R^2$ et Z sont définis comme ci-dessus et "b" représente un groupe alkylène linéaire, saturé, de un à quatre atomes de carbone, éventuellement substitué par un groupe méthyle, éventuellement en présence de sels de cuivre, dans un solvant polaire, à des températures comprises entre 20°C et le point d'ébullition du mélange réactionnel, éventuellement en présence d'un milieu faiblement alcalin,
puis éventuellement un composé de formule générale I ainsi obtenu est résolu en ses isomères et/ou un composé de formule générale I ainsi obtenu est converti en ses sels d'addition d'acide, de préférence en ses sels d'addition d'acide compatibles du point de vue physiologique, avec des acides inorganiques ou organiques.

9. Procédé selon la revendication 8e, caractérisé en ce que, pour introduire un reste (dialkylamino)méthyle ou 1-(dialkylamino)éthyle dans un composé de formule générale VIII, ce composé est mis à réagir dans un solvant polaire avec le formaldéhyde ou le paraformaldéhyde ou bien, pour introduire le reste cité en second lieu, avec l'acétaldéhyde, le paraldéhyde ou le métaldéhyde et avec une amine secondaire de formule générale X

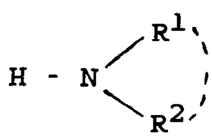

(X)

dans laquelle
$R^1$ et $R^2$ sont définis comme indiqué ci-dessus, éventuellement en présence de sels de cuivre, à des températures comprises entre 20°C et le point d'ébullition du mélange réactionnel, éventuellement en présence d'un mélange réactionnel faiblement alcalin.

10. Procédé selon la revendication 8e, caractérisé en ce que

a.) le composé de formule générale VIII est mis à réagir dans un solvant avec un aminal de formule générale IXb

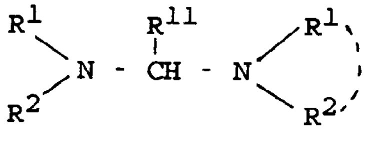

(IXb)

dans laquelle
$R^1$ et $R^2$ sont définis comme ci-dessus et $R^{11}$ représente un atome d'hydrogène ou un groupe méthyle, ou bien

b.) le composé de formule générale VIII ou le composé de Grignard qui en dérive de formule générale VTIIa

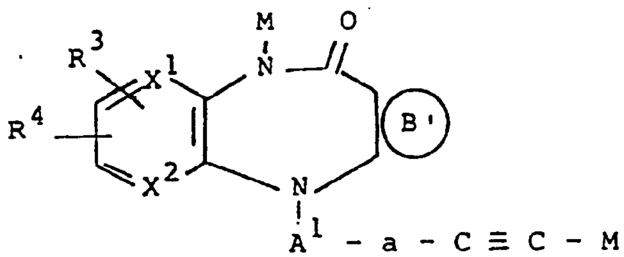

(VIIIa)

dans laquelle a, $A^1$, 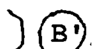, $R^3$, $R^4$, $X^1$ et $X^2$ sont définis de la manière indiquée et M est le groupe mgHal (Hal = atome d'halogène), est mis à réagir dans un solvant avec une alcoxyamine de formule générale Ixc

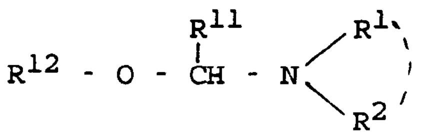

(IXc)

dans laquelle
$R^1$, $R^2$ et $R^{11}$ sont définis comme ci-dessus et
$R^{12}$ représente un reste alkyle de 1 à 10 atomes de carbone, ou bien

c. ) un sel métallique de formule générale VIIIa ci-dessus, dans laquelle M représente un atome de métal alcalin ou le reste MgHal (Hal = halogène) est mis à réagir dans un solvant organique, à des températures comprises entre -80°C et +150°C avec un composé de formule générale IX dans laquelle b représente un groupe 1,2-éthylène, 1,3-propylène ou 1,4-butylène éventuellement méthylsubstitué, éventuellement en présence d'un sel de cuivre, et le mélange d'isomères obtenu est ensuite éventuellement résolu et/ou les composés obtenus sont éventuellement convertis en leurs sels d'addition d'acide avec des acides inorganiques ou organiques.

**Revendications pour les Etats contractants suivants : ES, GR**

1. Procédé de préparation de nouvelles diazépinones condensées de formule générale I

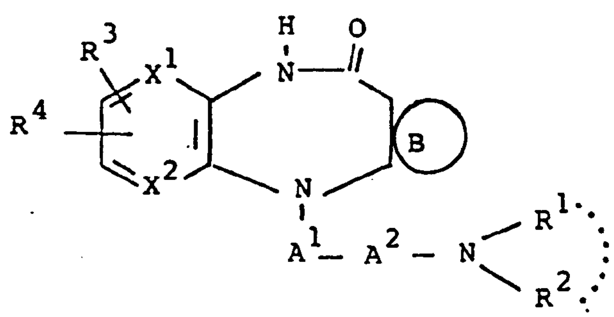

(I)

dans laquelle 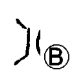 représente l'un des restes divalents

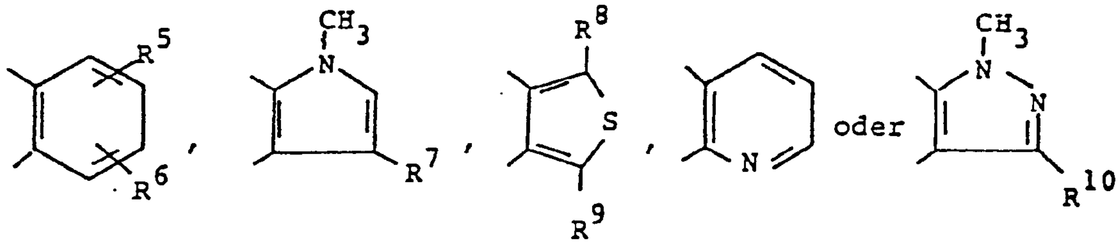

, , , oder

(S) (T) (U) (V) (W)

et

$X^1$, $X^2$, $A^1$, $A^2$ et $R^1$ à $R^{10}$ possèdent les significations suivantes:

$X^1$ et $X^2$ représentent un groupe =CH- ou, dans la mesure où Ⓑ a les significations des restes divalents (S), (U) ou (W) cités ci-dessus, peuvent l'un et l'autre ou seulement $X^1$ ou seulement $X^2$ représenter aussi un atome d'azote;
$A^1$ est un groupe méthylène substitué éventuellement par un ou deux groupes méthyle;
$A^2$ représente une chaîne alkylène linéaire, contenant éventuellement une double ou triple liaison et comprenant trois à sept atomes de carbone, qui peut en outre être méthylsubstituée;
$R^1$ représente un reste alkyle ramifié ou non ramifié de 1 à 4 atomes de carbone;
$R^2$ représente un reste alkyle ramifié ou non ramifié de 1 à 7 atomes de carbone qui peut en outre éventuellement être substitué sur son deuxième à septième atome de carbone par un groupe hydroxyle, un reste cycloalkyle ou un reste cycloalkylméthyle ayant 3 à 7 atomes de carbone dans le cycle, le cycle cycloalkyle pouvant éventuellement être substitué encore par un groupe hydroxyle;
$R^1$ et $R^2$ peuvent cependant également former avec l'atome d'azote situé entre eux un cycle monocyclique, hétérocyclique saturé, de 4 à 7 chaînons, qui peut éventuellement être interrompu par un atome d'oxygène ou par une fonction azote et/ou qui peut être substitué par un reste alkyle de 1 à 6 atomes de carbone éventuellement substitué par un groupe phényle, dialkyl($C_1$-$C_3$)amino ou (cycloalkyl)alkylamino ou par un reste (cycloalkyl)alkyle contenant éventuellement un ou deux atomes d'oxygène d'éther, les groupes cycloalkyle cités ci-dessus pouvant contenir 4 à 7 atomes de carbone et les groupes alkylène liés à ceux-ci pouvant contenir 1 à 3 atomes de carbone;
$R^3$ est un groupe alkyle de 1 à 4 atomes de carbone, un atome de chlore ou un atome d'hydrogène;
$R^4$ est un atome d'hydrogène ou un groupe méthyle;
$R^5$ et $R^6$ représentent chacun un atome d'hydrogène, un atome de fluor, de chlore ou de brome ou un groupe alkyle de 1 à 4 atomes de carbone;
$R^7$ représente un atome d'hydrogène ou de chlore ou un groupe méthyle;
$R^8$ représente un atome d'hydrogène ou un reste alkyle de 1 à 4 atomes de carbone;
$R^9$ représente un atome d'hydrogène ou d'halogène ou un groupe alkyle de 1 à 4 atomes de carbone et

$R^{10}$ représente un atome d'hydrogène ou un groupe méthyle, et lorsque Ⓑ représente le reste divalent T et $R^7$ est un atome d'hydrogène, $R^3$ ne doit pas représenter un atome de chlore et $A^2$ ne doit contenir aucune double ou triple liaison, de leurs formes diastéréoisomères et énantiomères, et de leurs sels compatibles du point de vue physiologique avec des acides inorganiques ou organiques, caractérisé en ce que

a.) pour la préparation de composés de formule générale I dans laquelle $R^7$ représente un atome de chlore ou un groupe méthyle et les autres restes ont les significations données ci-dessus, un système tricyclique de formule générale II

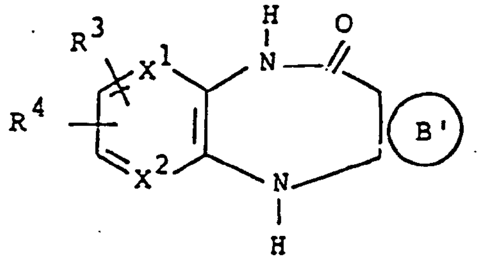

(II)

dans laquelle

les restes $R^3$, $R^4$, $X^1$ et $X^2$ sont définis de la manière indiquée ci-dessus et 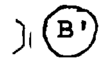 représente l'un des restes divalents S, U, V, W ou T′

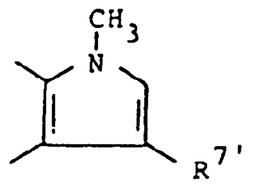

(T')

$R^{7'}$ représentant un atome de chlore ou un groupe méthyle, est converti en son sel dilithien et celui-ci est ensuite alkylé avec un composé de formule générale IV

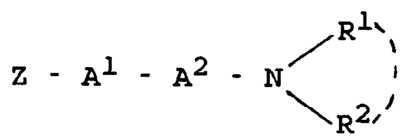

(IV)

dans laquelle
$A^1$, $A^2$, $R^1$ et $R^2$ ont les significations indiquées ci-dessus et Z représente un atome de chlore, de brome ou d'iode, le groupe méthanesulfonyloxy, éthanesulfonyloxy ou un groupe arènesulfonyloxy, la préparation du sel dilithien et l'alkylation, qui peuvent se dérouler aussi dans le même milieu réactionnel, étant accomplies dans un solvant organique à des températures comprises entre -80 et +70°C, ou

b.) pour la préparation de composés de formule générale I dans laquelle $A^2$ représente une chaîne alkylène linéaire, saturée, comportant trois à sept atomes de carbone qui peut en outre éventuellement être méthyl-substituée et $R^7$ représente un atome de chlore ou un groupe méthyle, un système (arylméthyl)tricyclique de formule générale V

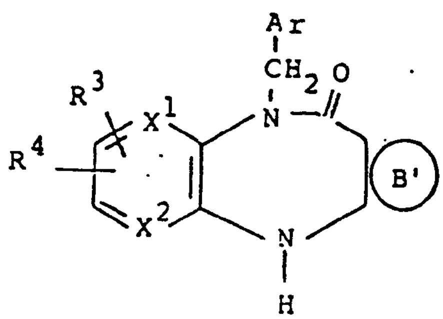

(V)

dans laquelle

les restes 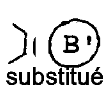, $R^3$, $R^4$, $X^1$ et $X^2$ sont définis comme ci-dessus et Ar représente un reste phényle éventuellement substitué par un ou deux groupes méthyle et/ou méthoxy, est converti en son sel métallique de formule générale Va

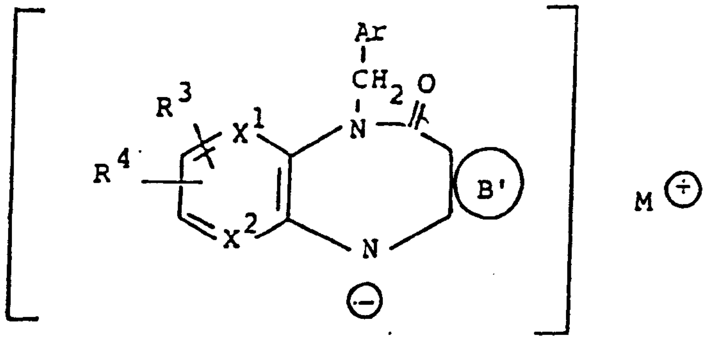

(Va)

dans laquelle M est un atome de métal alcalin ou représente le reste MgHal dans lequel Hal est le chlore, le brome ou l'iode, et celui-ci est ensuite alkylé avec un composé de formule générale IV

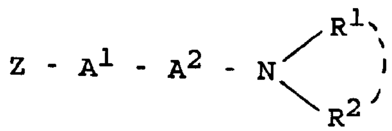

(IV)

dans laquelle
$A^1$, $R^1$, $R^2$ et Z ont les significations indiquées ci-dessus et $A^2$ représente une chaîne alkylène linéaire saturée comprenant trois à sept atomes de carbone, qui peut en outre éventuellement être méthylsubstituée, et le groupe arylméthyle est finalement séparé par voie acidolytique de ce composé ainsi formé, la métallation étant opérée dans un solvant organique inerte à des températures comprises entre -100°C et le point d'ébullition du mélange réactionnel avec des aryllithium, des alkyllithium, des dialkylamidures de lithium ou des réactifs de Grignard, des hydrures alcalins, des amidures alcalins, des hydroxydes alcalins, l'alkylation étant opérée dans un solvant organique à des températures comprises entre -80°C et +70°C et le clivage acidolytique du groupe arylméthyle étant opéré avec des acides forts ou des acides de Lewis à des températures comprises entre -20°C et +150°C
ou bien

c.) pour la préparation de composés de formule générale I dans laquelle $A^2$ représente une chaîne alkylène linéaire saturée comprenant trois à sept atomes de carbone, qui peut en outre être éventuellement méthylsubstituée, $R^3$ représente un atome d'hydrogène ou un groupe alkyle de 1 à 4 atomes de carbone et 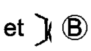 représente l'un des restes divalents S, T, U ou W avec la limitation selon laquelle $R^5$, $R^6$ et $R^7$ ne peuvent pas représenter le chlore ou le brome, un composé de formule générale VIa

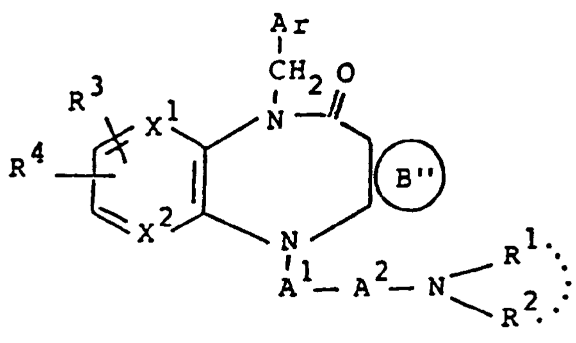

(VIa)

dans laquelle

Les restes $A^1$, $A^2$, $R^1$, $R^2$, $R^3$, $R^4$, $X^1$, $X^2$ et Ar ont les significations indiquée et 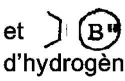 représente l'un des restes divalents S, T, U ou W avec la condition que $R^7$ ne soit pas un atome d'hydrogène,
est hydrogénolysé à des pressions d'hydrogène de 1 à 300 bar et à des températures de 0°C à 130°C en présence de catalyseurs de métaux du VIII$^{ème}$ sous-groupe du système périodique des éléments et en présence de solvants et, éventuellement, d'acides minéraux, ou bien

d.) pour la préparation de composés de formule générale I dans laquelle $A^2$ représente une chaîne alkylène linéaire, contenant éventuellement une double liaison et comprenant trois à sept atomes de carbone, qui peut en outre être méthylsubstituée, $R^3$ représente un atome d'hydrogène ou un groupe alkyle de 1 à 4 atomes de carbone et )(Ⓑ représente l'un des restes divalents S, T, U ou W, avec la limitation selon laquelle $R^5$, $R^6$ et $R^7$ ne représentent pas un atome de chlore ou de brome, un composé de formule générale VII

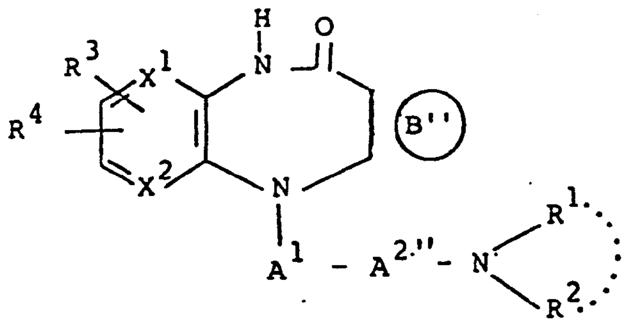

(VII) .

dans laquelle

les restes $A^1$, $R^1$ à $R^4$, $X^1$, $X^2$ et )( (B'') ont les significations indiquées ci-dessus et $A^{2''}$ est une chaîne alkylène linéaire contenant une liaison triple et comportant trois à sept atomes de carbone, qui peut en outre être méthylsubstituée, est hydrogéné catalytiquement à des pressions d'hydrogène de 0,1 à 300 bar et à des températures de 0 à 130°C en présence de métaux du VIII$^{ème}$ sous-groupe du système périodique des éléments et en présence de solvants, l'hydrogénation catalytique étant soit interrompue après absorption de 1 mole d'hydrogène soit opérée en présence d'un catalyseur désactivant pour la préparation de composés de formule générale I dans laquelle $A^2$ représente un groupe alkylène contenant une double liaison, ou bien

e.) pour la préparation de composés de formule générale I dans laquelle $A^2$ est une chaîne alkylène linéaire contenant une triple liaison et comportant trois à sept atomes de carbone qui peut en outre être éventuellement méthylsubstituée et )Ⓑ représente le reste divalent )( (B') défini ci-dessus, un systèe tricyclique condensé de formule générale VIII

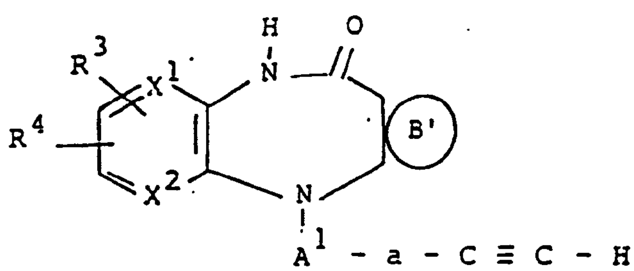

(VIII)

dans laquelle

$A^1$, ) (B'),, $R^3$, $R^4$, $X^1$ et $X^2$ possèdent les significations indiquées ci-dessus et "a" représente une liaison simple ou une chaîne alkylène linéaire, saturée, de 1 à 4 atomes de carbone, éventuellement substituée par un groupe méthyle, est aminoalkylé avec un composé de formnule générale IX

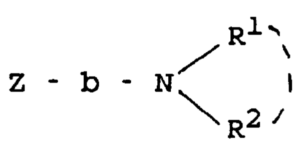

(IX)

dans laquelle
$R^1$, $R^2$ et Z sont définis comme ci-dessus et "b" représente un groupe alkylène linéaire, saturé, de un à quatre atomes de carbone, éventuellement substitué par un groupe méthyle, éventuellement en présence de sels de cuivre, dans un solvant polaire, à des températures comprises entre 20°C et le point d'ébullition du mélange réactionnel, éventuellement en présence d'un milieu faiblement alcalin,
puis éventuellement un composé de formule générale I ainsi obtenu est résolu en ses isomères et/ou un composé de formule générale I ainsi obtenu est converti en ses sels d'addition d'acide, de préférence en ses sels d'addition d'acide compatibles du point de vue physiologique, avec des acides inorganiques ou organiques.

2. Procédé selon la revendication le, caractérisé en ce que, pour introduire un reste (dialkylamino)méthyle ou 1-(dialkylamino)éthyle dans un composé de formule générale VIII, ce composé est mis à réagir dans un solvant polaire avec le formaldéhyde ou le paraformaldéhyde ou bien, pour introduire le reste cité en second lieu, avec l'acétaldéhyde, le paraldéhyde ou le métaldéhyde et avec une amine secondaire de formule générale X

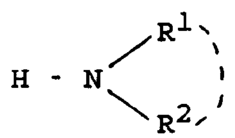

(X)

dans laquelle
$R^1$ et $R^2$ sont définis comme indiqué ci-dessus, éventuellement en présence de sels de cuivre, à des températures comprises entre 20°C et le point d'ébullition du mélange réactionnel, éventuellement en présence d'un mélange réactionnel faiblement alcalin.

3. Procédé selon la revendication 1e, caractérisé en ce que

a.) le composé de formule générale VIII est mis à réagir dans un solvant avec un aminal de formule générale Ixb

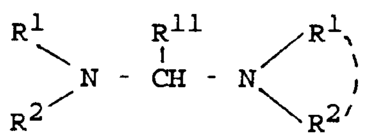

(IXb)

dans laquelle
$R^1$ et $R^2$ sont définis comme ci-dessus et $R^{11}$ représente un atome d'hydrogène ou un groupe méthyle, ou bien

b.) le composé de formule générale VIII ou le composé de Grignard qui en dérive de formule générale VIIIa

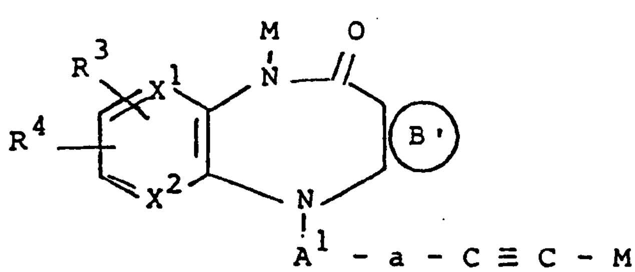

(VIIIa)

dans laquelle a, $A^1$, 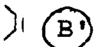, $R^3$, $R^4$, $X^1$ et $X^2$ sont définis de la manière indiquée
et M est le groupe MgHal (Hal = atome d'halogène), est mis à réagir dans un solvant avec une alcoxyamine de formule générale IXc

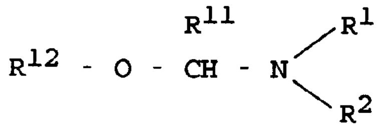

(IXc)

dans laquelle
$R^1$, $R^2$ et $R^{11}$ sont définis comme ci-dessus et
$R^{12}$ représente un reste alkyle de 1 à 10 atomes de carbone, ou bien

c.) un sel métallique de formule générale VIIIa ci-dessus, dans laquelle M représente un atome de métal alcalin ou le reste MgHal (Hal = halogène) est mis à réagir dans un solvant organique, à des températures comprises entre -80°C et +150°C avec un composé de formule générale IX dans laquelle b représente un groupe 1,2-éthylène, 1,3-propylène ou 1,4-butylène éventuellement méthylsubstitué, éventuellement en présence d'un sel de cuivre, et le mélange d'isomères obtenu est ensuite éventuellement résolu et/ou les composés obtenus sont éventuellement convertis en leurs sels d'addition d'acide avec des acides inorganiques ou organiques.